# EUROPEAN PATENT SPECIFICATION

(11) **EP 3 383 875 B1**
(45) Date of publication and mention of the grant of the patent: **09.02.2022**
(21) Application number: 16834200.4
(22) Date of filing: 30.11.2016
(51) Int. Cl.: C07D 471/04, A61K 31/506, A61P 35/00

(54) **3-PYRIMIDINYL PYRROLO [2,3-B] PYRIDINE AS ANTICANCER AGENTS AND THE PROCESS FOR THE PREPARATION THEREOF**
3-PYRIMIDINYL-PYRROLO[2,3-BETA]PYRIDIN ALS NEUE ANTIKREBSMITTEL UND VERFAHREN ZUR HERSTELLUNG DAVON
PYRIDINES 3-PYRIMIDINYL PYRROLO [2,3-B] UTILISÉES COMME AGENTS ANTICANCÉREUX ET LEUR PROCÉDÉ DE PRÉPARATION

(30) Priority: 30.11.2015 IN 3893DE2015
(43) Date of publication of application: 10.10.2018
(73) Proprietor: Council Of Scientific & Industrial Research, New Delhi 110 001 (IN)
(72) Inventor: SINGH, Umed, Jammu-Tawi 180001 (IN); MAHAJAN, Girish, Jammu-Tawi 180001 (IN); CHASHOO, Gousia, Jammu-Tawi 180001 (IN); THATIKONDA, Thanusha, Jammu-Tawi 180001 (IN); MAHAJAN, Priya, Jammu-Tawi 180001 (IN); ARURI, Hari Prasad, Jammu-Tawi 180001 (IN); GUDUP, Satish Sonbarao, Jammu-Tawi 180001 (IN); NARGOTRA, Amit, Jammu-Tawi 180001 (IN); MONDHE, Dilip Manikrao, Jammu-Tawi 180001 (IN); VISHWAKARMA, Ram Asrey, Jammu-Tawi 180001 (IN); SINGH, Parvinder Pal, Jammu-Tawi 180001 (IN)
(74) Representative: Harris, Jennifer Lucy
(86) International application number: PCT/IN2016/050426
(87) International publication number: WO 2017/094026

(56) References cited:
- WO-A1-2006/015123
- WO-A2-2010/003133
- WO-A2-2011/149950
- US-A1- 2010 184 790

## Description

### FIELD OF THE INVENTION:

The present invention relates to novel 3-pyrimidinyl pyrrolo [2,3-*b*] pyridine. Particularly, the present invention relates to the preparation of compounds and their anti-cancer activities. More particularly, the present invention relates to new compounds useful as modulators of cyclin-dependent kinase.

### BACKGROUND AND PRIOR ART OF THE INVENTION:

Our understanding of biology opens new opportunities to evade many dreadful diseases. One of the key components of the cycle-regulating machinery namely cyclin-dependent kinases (CDKs) along with their activating partners termed cyclins which play a critical role in the functioning of the cells (as they regulate cell-division cycle, apoptosis, transcription, and differentiation, as well as controlling functions in the nervous system) have been found deregulated in many diseases such as cancers, polycystic kidney and neurodegenerative diseases. Because of this, several CDK inhibitors have been reported for promising antitumor and neuro-protective activities (US20100184790A1). In the area of cancer, two CDK inhibitors *viz.*, flavopiridol (M. Drees et al., Clin Cancer Res 1997, 3, 273-279) and palbociclib or PD 0332991 (Peter L. Toogood et al., J. Med. Chem. 2005, 48, 2388-2406) are already got approval for cancer namely acute myeloid leukemia and breast cancer respectively, several others are in clinical trials viz., roscovitine (CYC202) (Whittaker SR et al., Cancer Res 2004, 64, 262-72), BMS-387032 (SNS-032) (Andrew Conroy et al., Cancer Chemother Pharmacol 2009, 64, 723-732). Considering the high attrition rate in clinical development, still there is a need to further enriched the clinical pipelines.

In this direction, azaoindole skeleton have been discovered for CDK inhibitory activities and several azaindole based compounds have been designed, synthesized and patented. For examples, patent applications, such as US20100184790A1, US20110201599A1, and WO2008129152A1, disclose numerous azaindole based compounds namely meriolins with substituted at 4-position of azainodole core. WO 2008/079346 A1 discloses numerous azaindole based compounds substituted at 5-position of azainodole core. WO2010003133 A2 describes numerous azaindole, which also include 3-pyrimidinylpyrrolo[2,3-*b*]pyridines as well as 5-phenyl-3-pyrimidinyl pyrrolo[2,3-*b*]pyridines as protein kinase inhibitors, however, no study regarding the activity of these compounds is disclosed in this document. In the similar way, US 2009/0143352 A1 discloses 3-heteraoaryl pyrrolo[2,3-*b*]pyridines which also include 3-pyrimidinyl pyrrolo[2,3-*b*]pyridine as kinase inhibitors.

### OBJECTIVE OF THE INVENTION:

The main object of the present invention is to provide a novel 3-pyrimidinyl pyrrolo[2,3-*b*]pyridine containing heterocyclic compounds as CDK inhibitor.

Still another object of the present invention is to provide a process for the preparation of 3-pyrimidinyl pyrrolo[2,3-*b*]pyridine containing heterocyclic compounds.

Yet another object of the present invention is to provide CDK based anti-cancer agent.

### SUMMARY OF THE INVENTION:

Accordingly, the present invention provides a compound of general Formula 1 is 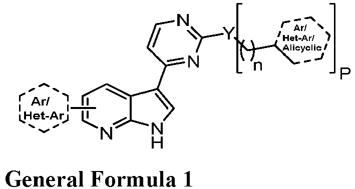 wherein,
'Y' is independently selected from any of NH, S, O, SO, SO₂;
'n' is independently selected from any of 0, 1, 2;
Pis 1;
**[Ar]** is independently selected from any of substituted or unsubstituted phenyl and substituted or unsubstituted aryl, attached through any available ring position, wherein substitution on phenyl or aryl, when present, is mono substitution by a substituent selected from any of F, Cl, Br, I, CN, NR₁R₂, CF₃, CHCF₂, CH₂F, OCF₃, OCH₂CF₃, OR₃, NO₂, NO, CHR₄R₅, alkyl chain from C1 to C14, COOR₆, CHO, COR₇, COCF₃, COCH₂CF₃, SR₈, SOR₉, SO₂R₁₀, SONR₁₁R₁₂ and SO₂NR₁₃R₁₄;
**[Hetero-Ar]** is independently selected from any of substituted or unsubstituted heterocycles, selected from pyridyl, triazolyl, triazinyl, pyrimidinyl, pyridazinyl, oxazolyl, furanyl, benzothiophenyl benzofuranyl, thiophenyl, pyrrolyl, imidazoyl, thiazoyl, quinolinyl, isoquinolinyl, benzooxazolyl, benzothiazolyl, indolyl, benzotriazolyl, and benzoimidazolyl, attached through any available ring position, wherein substitution on a heterocycle, when present, is mono substitution by a substituent selected from any of F, Cl, Br, I, CN, NR₄R₅, CF₃, CHCF₂, CH₂F, OCF₃, OCH₂CF₃, OR₆, NO₂, NO, CHR₄R₅, alkyl chain from C1 to C14, COOR₆, CHO, COR₇, COCF₃, COCH₂CF₃, SR₈, SOR₉, SO₂R₁₀, SONR₁₁R₁₂, and SO₂NR₁₃R₁₄;
R₁, R₂, R₃, R₄, R₅, R₆, R₇, R₈, R₉, R₁₀, R₁₁, R₁₂, R₁₃, and R₁₄, are independently mono substituents selected from any of H, linear C1-C10alkyl, branched C3-C10 alkyl, and unsubstituted phenyl; **[Alicyclic]** is independently selected from unsubstituted cycloalkanes selected from any of cyclopropane, cyclobutane, cyclopentane, cyclohexane, cycloheptane, and cyclooctane, attached through any available ring position.

In another embodiment of the present invention, said compounds of Formula 1 can be used in treating cancer.

In still another embodiment of the present invention, said compounds of Formula 1 inhibits CDK2 and CDK9.

In one embodiment of the present disclosure, said compounds of general **Formula 1** inhibits an *in vitro* cancer cell line activity.

The present invention also relates to a process for preparing compounds of Formula 1. In one embodiment of the present disclosure, wherein the process comprises;
reacting intermediate compound of Formula 13, 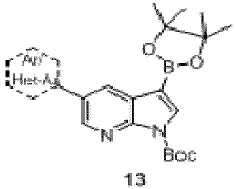 wherein,
**[Ar]** is independently selected from any of substituted or unsubstituted phenyl and substituted or unsubstituted aryl, attached through any available ring position, wherein substitution on phenyl or aryl, when present, is mono substitution by a subtituent selected from any of F, Cl, Br, I, CN, NR₁R₂, CF₃, CHCF₂, CH₂F, OCF₃, OCH₂CF₃, OR₃, NO₂, NO, CHR₄R₅, alkyl chain from C1 to C14, COOR₆, CHO, COR₇, COCF₃, COCH₂CF₃, SR₈, SOR₉, SO₂R₁₀, SONR₁₁R₁₂, and SO₂NR₁₃R₁₄;
**[Hetero-Ar]** is independently selected from substituted or unsubstituted heterocycles selected from pyridyl, triazolyl, triazinyl, pyrimidinyl, pyridazinyl, oxazolyl, furanyl, benzothiophenyl benzofuranyl, thiophenyl, pyrrolyl, imidazoyl, thiazoyl, quinolinyl, isoquinolinyl, benzooxazolyl, benzothiazolyl, indolyl, benzotriazolyl, and benzoimidazolyl, attached through any available ring position, wherein substitution on a heterocycle, when present, is mono substitution by a substituent selected from any of F, Cl, Br, I, CN, NR₄R₅, CF₃, CHCF₂, CH₂F, OCF₃, OCH₂CF₃, OR₆, NO₂, NO, CHR₄R₅, alkyl chain from C1 to C14, COOR₆, CHO, COR₇, COCF₃, COCH₂CF₃, SR₈, SOR₉, SO₂R₁₀, SONR₁₁R₁₂, and SO₂NR₁₃R₁₄; R₁, R₂, R₃, R₄, R₅, R₆, R₇, R₈, R₉, R₁₀, R₁₁, R₁₂, R₁₃, and R₁₄, are independently mono substituents selected from any of H, linear C1-C10alkyl chain, branched C3-C10 alkyl, and unsubstituted phenyl,
   - with a substituted chloropyrimidine intermediate compound in presence of a catalyst in a solvent followed by addition of the base at a temperature in the range of 80 °C to 100 °C for a period in the range of 24 to 49 hrs to obtain product compound of general Formula 1.

In preferred embodiment of the present invention, wherein the substituted chloropyrimidne intermediate compound may be selected from the group consisting of 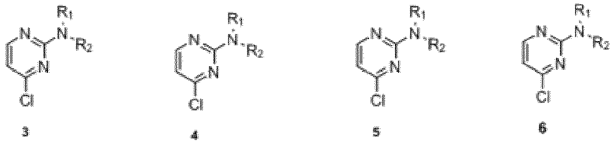 wherein,
R1=H; R2=cyclopropyl, cyclopentyl, cycloheptyl or 2-phenylethyl

In yet another embodiment of the present invention, wherein the catalyst used is tetrakis(triphenylphosphino) palladium(0).

In still another embodiment of the present invention, wherein the solvent is selected from the group consisting of dry dioxane and methanol.

In another embodiment of the present invention, wherein the base is selected from the group consisting cesium carbonate and triethyl amine.

### BRIEF DESCRIPTION OF THE DRAWINGS:

**Fig 1** shows the synthetic approach (scheme 1) for the synthesis of compounds 1A (1-20).
**Fig 2** shows the synthetic approach (scheme2) for the synthesis of compounds 1A (21-40).
**Fig 3** shows the synthetic approach (scheme3) for the synthesis of compounds 1A (41-60).
**Fig 4** shows the synthetic approach (scheme4) for the synthesis of compounds 1B (1-20).
**Fig 5** shows the synthetic approach (scheme5) for the synthesis of compounds 1C (1-20), which are provided for reference.

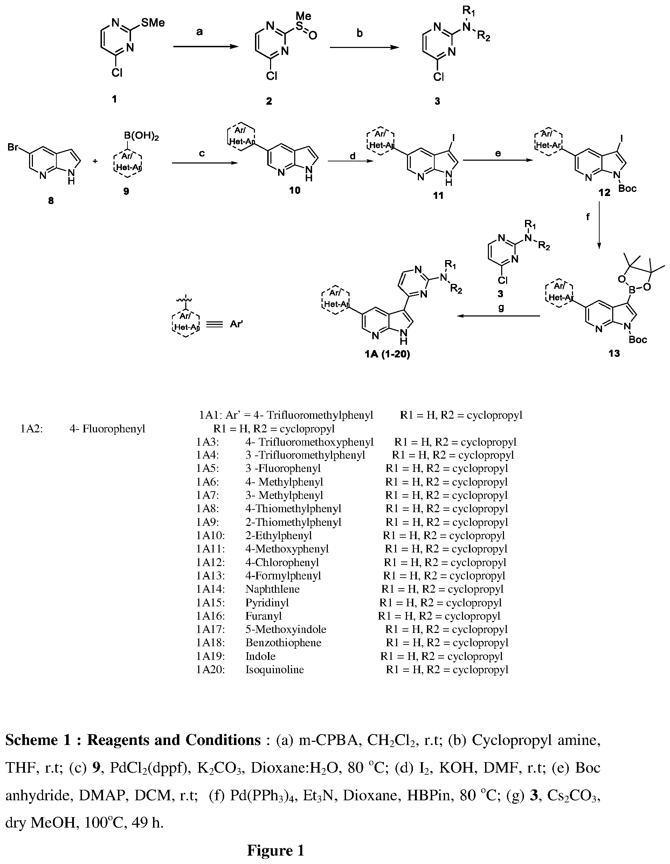

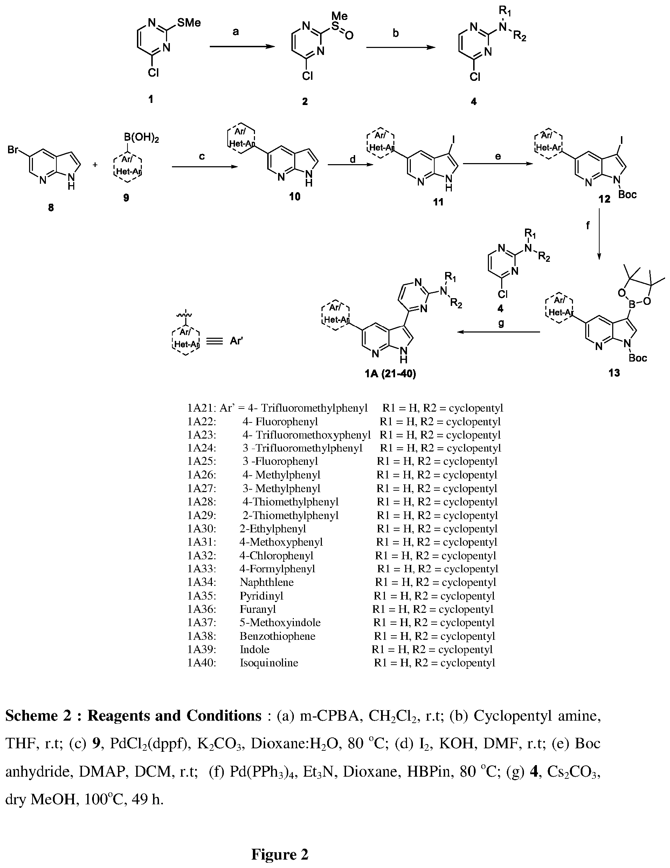

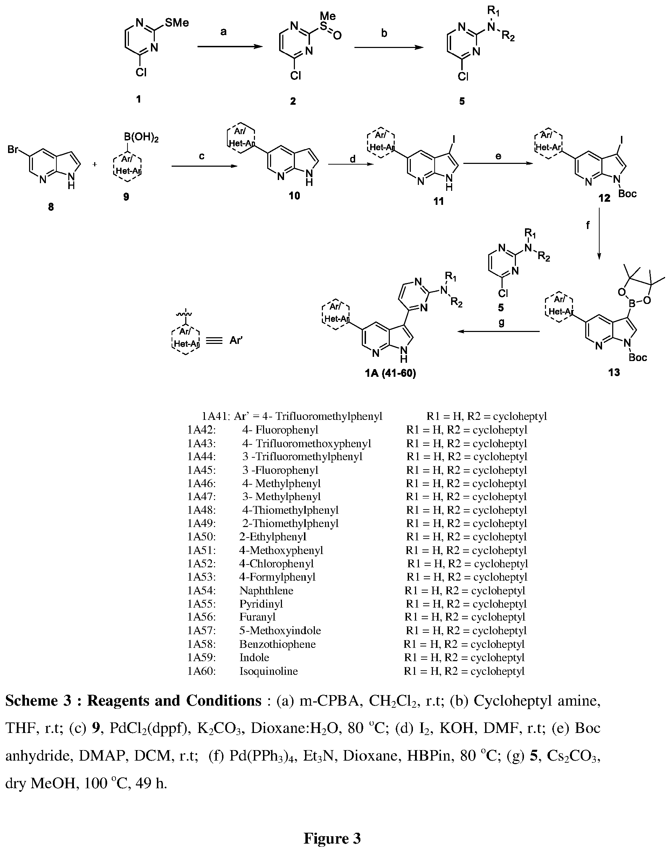

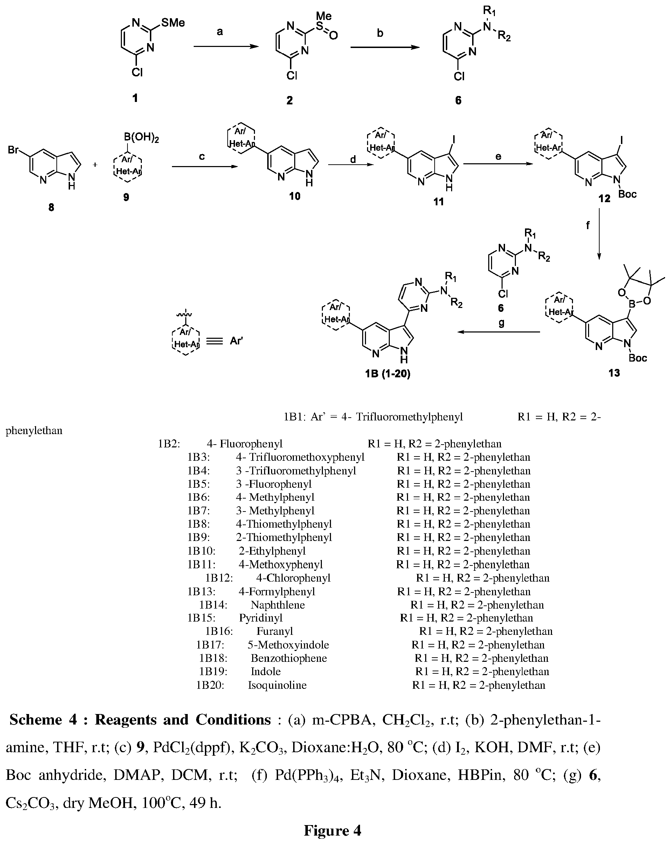

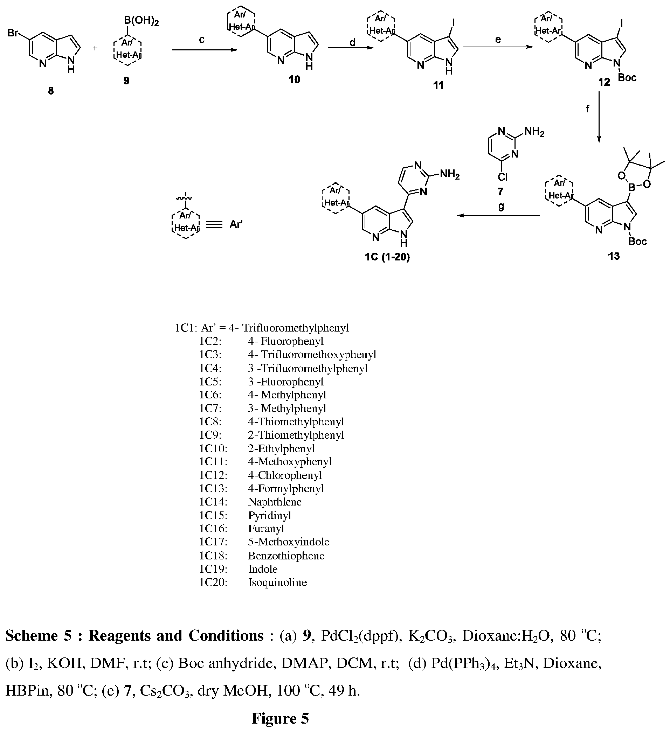

**Table 1** shows the structures of representative compounds IA (1-20) belong to Formula **IA** and synthesized as per scheme 1 provided in (Fig 1).
**Table 2** shows the structures of representative compounds IA (21-40) belong to Formula **IA** and synthesized as per scheme 2 provided in (Fig 2).
**Table 3** shows the structures of representative compounds (41-60) belong to Formula **IA** and synthesized as per scheme 3 provided in (Fig 3).
**Table 4** shows the structures of representative compounds IB (1-20) belong to Formula **IB** and synthesized as per scheme 4 provided in (Fig 4).
**Table 5** is provided for reference and shows the structures of representative compounds IC (1-20) belonging to Formula **IC** and synthesized as per scheme 5 provided in (Fig 5).
**Table 6** shows the inhibition results of compounds general Formula 1 by enzyme based assay CDK.
**Table 7** shows the inhibition results of compounds general Formula 1 by enzyme based assay CDK.
**Table 8** shows the inhibition results of compounds of general Formula 1 by MTT assay on cancer cell line
**Table 9** is provided for reference and shows the inhibition results of compounds 1C (1-20) by MTT assay on cancer cell line

### DETAILED DESCRIPTION OF THE INVENTION:

The present invention relates to novel 3-pyrimidinyl pyrrolo[2,3-*b*]pyridine, their method of preparation, and their use as drugs for treating cancer. These derivatives are effective CDK inhibitor. In a first aspect, the present invention pertains to a compound having a general Formula **1** 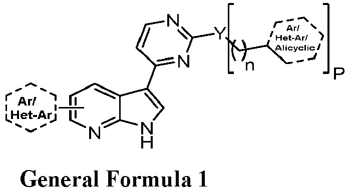 wherein,
'Y' is independently selected from any of NH, S, O, SO, SO₂;
'n' is independently selected from any of 0, 1, 2;
Pis 1;
[Ar] is independently selected from any of substituted or unsubstituted phenyl and substituted or unsubstituted aryl, attached through any available ring position, wherein substitution on phenyl or aryl, when present, is mono substitution by a substituent selected from any of F, Cl, Br, I, CN, NR₁R₂, CF₃, CHCF₂, CH₂F, OCF₃, OCH₂CF₃, OR₃, NO₂, NO, CHR₄R₅, alkyl chain from C1 to C14, COOR₆, CHO, COR₇, COCF₃, COCH₂CF₃, SR₈, SOR₉, SO₂R₁₀, SONR₁₁R₁₂, and SO₂NR₁₃R₁₄;
**[Hetero-Ar]** is independently selected from substituted or unsubstituted heterocycles; selected from pyridyl, triazolyl, triazinyl, pyrimidinyl, pyridazinyl, oxazolyl, furanyl, benzothiophenyl benzofuranyl, thiophenyl, pyrrolyl, imidazoyl, thiazoyl, quinolinyl, isoquinolinyl, benzooxazolyl, benzothiazolyl, indolyl, benzotriazolyl, and benzoimidazolyl, attached through any available ring position, wherein substitution on a heterocycle, when present, is mono substitution by a substituent selected from any of F, Cl, Br, I, CN, NR₄R₅, CF₃, CHCF₂, CH₂F, OCF₃, OCH₂CF₃, OR₆, NO₂, NO, CHR₄R₅, alkyl chain from C1 to C14, COOR₆, CHO, COR₇, COCF₃, COCH₂CF₃, SR₈, SOR₉, SO₂R₁₀, SONR₁₁R₁₂, and SO₂NR₁₃R₁₄;
R₁, R₂, R₃, R₄, R₅, R₆, R₇, R₈, R₉, R₁₀, R₁₁, R₁₂, R₁₃, and R₁₄, are independently mono substituents selected from any of H, linear C1-C10 alkyl, branched C3-C10 alkyl, and unsubstituted phenyl; **[Alicyclic]** is independently selected from unsubstituted cycloalkanes selected from any of cyclopropane, cyclobutane, cyclopentane, cyclohexane, cycloheptane, and cyclooctane, attached through any available ring position.

The compounds of general **Formula 1** are useful as anti-cancer agent.

The compounds of general **Formula 1** inhibit CDK2 and CDK9.

The compounds of general **Formula 1** exhibit an *in vitro* cancer cell line activity and *in-vivo* efficacy. In one embodiment the compound of Formula 1A of general **Formula 1** can be prepared by coupling intermediate **13** with intermediate **3** in the presence of palladium catalyst i.e. tetrakis (triphenylphosphino) palladium (0) in a solvent selected from dry dioxane, or methanol in the presence of base selected from cesium carbonate, or triethyl amine at a temperature in the range of 80 °C to 100 °C for a period in the range of 24 to 49 hrs to obtain the compounds of **1A (1-20)** of general Formula 1.

The compound of Formula 1A of general **Formula 1**, can be prepared by coupling of intermediate **13** with intermediate **4** in the presence of palladium catalyst i.e. tetrakis (triphenylphosphino) palladium (0) in a solvent selected from dioxane, or methanol in the presence of base selected from cesium carbonate, or triethyl amine at a temperature in the range of 80 °C to 100 °C for a period in the range of 24 to 49 hrs to obtain compounds of **1A (21-40)** of general Formula 1.

The compound of Formula 1A of general **Formula 1** can be prepared by coupling of intermediate **13** with intermediate **5** in the presence of palladium catalyst i.e. tetrakis (triphenylphosphino) palladium (0) in a solvent selected from dry dioxane, or methanol in the presence of base selected from cesium carbonate, or triethyl amine at a temperature in the range of 80 °C to 100 °C for a period in the range of 24 to 49 hrs to obtain the compounds of **1A (41-60)** of general Formula 1.

The compounds of Formula 1B of general **Formula 1** can be prepared by coupling of intermediate **13** with intermediate **6** in the presence of palladium catalyst i.e. tetrakis (triphenylphosphino) palladium (0) in a solvent selected from dry dioxane, or methanol in the presence of base selected from cesium carbonate, or triethyl amine at a temperature in the range of 80 °C to 100 °C for a period in the range of 24 to 49 hrs to obtain the compounds of **1B (1-20)** of general Formula 1.

The compounds of Formula 1C(provided for reference) can be prepared by coupling of intermediate **13** with 2-amino-4-chloropyrimidine in the presence of palladium catalyst i.e. tetrakis (triphenylphosphino) palladium (0) in a solvent selected from dry dioxane, or methanol in the presence of base selected from cesium carbonate, or triethyl amine at a temperature in the range of 80 °C to 100 °C for a period in the range of 24 to 49 hrs to obtain the compounds of **1C (1-20)**.

The compounds of Formula 1A and 1B, includes:
*N*-cyclopropyl-4-(5-(4-(trifluoromethyl)phenyl)-1*H*-pyrrolo[2,3-*b*]pyridin-3-yl)pyrimidin-2-amine,(compound **1A1**, Table 1)
*N*-cyclopropyl-4-(5-(4-fluorophenyl)-1*H*-pyrrolo[2,3-*b*]pyridin-3-yl)pyrimidin-2-amine(compound**1A2**, Table 1)
*N*-cyclopropyl-4-(5-(4-(trifluoromethoxy)phenyl)-1*H*-pyrrolo[2,3-*b*]pyridin-3-yl)pyrimidin-2-amine (compound **1A3**, Table 1)
*N*-cyclopropyl-4-(5-(3-(trifluoromethyl)phenyl)-1*H*-pyrrolo[2,3-*b*]pyridin-3-yl)pyrimidin-2-amine (compound **1A4**, Table 1)
*N*-cyclopropyl-4-(5-(3-fluorophenyl)-1*H*-pyrrolo[2,3-*b*]pyridin-3-yl)pyrimidin-2-amine(compound**1A5**, Table 1)
*N*-cyclopropyl-4-(5-(p-tolyl)-1*H*-pyrrolo[2,3-*b*]pyridin-3-yl)pyrimidin-2-amine(compound **1A6**, Table 1)
*N*-cyclopropyl-4-(5-(m-tolyl)-1*H*-pyrrolo[2,3-*b*]pyridin-3-yl)pyrimidin-2-amine(compound **1A7**, Table 1)
*N*-cyclopropyl-4-(5-(4-(methylthio)phenyl)-1*H*-pyrrolo[2,3-*b*]pyridin-3-yl)pyrimidin-2-amine (compound **1A8**, Table 1)
*N*-cyclopropyl-4-(5-(2-(methylthio)phenyl)-1*H*-pyrrolo[2,3-*b*]pyridin-3-yl)pyrimidin-2-amine (compound **1A9**, Table 1)
*N*-cyclopropyl-4-(5-(2-ethylphenyl)-1*H*-pyrrolo[2,3-*b*]pyridin-3-yl)pyrimidin-2-amine(compound**1A10**, Table 1)
*N*-cyclopropyl-4-(5-(4-methoxyphenyl)-1*H*-pyrrolo[2,3-*b*]pyridin-3-yl)pyrimidin-2-amine(compound **1A11**, Table 1) 4-(5-(4-chlorophenyl)-1*H*-pyrrolo[2,3-*b*]pyridin-3-yl)-*N*-cyclopropylpyrimidin-2-amine(compound **1A12**, Table 1)
1-(4-(3-(2-(cyclopropylamino)pyrimidin-4-yl)-1*H*-pyrrolo[2,3-*b*]pyridin-5-yl)phenyl)ethan-1-one (compound **1A13**, Table 1)
*N*-cyclopropyl-4-(5-(naphthalen-1-yl)-1*H*-pyrrolo[2,3-*b*]pyridin-3-yl)pyrimidin-2-amine(compound **1A14**, Table 1)
*N*-cyclopropyl-4-(5-(pyridin-3-yl)-1*H*-pyrrolo[2,3-*b*]pyridin-3-yl)pyrimidin-2-amine (compound **1A15**, Table 1)
*N*-cyclopropyl-4-(5-(furan-3-yl)-1*H*-pyrrolo[2,3-*b*]pyridin-3-yl)pyrimidin-2-amine(compound**1A16**, Table 1)
*N*-cyclopropyl-4-(5-(5-methoxy-1*H*-indol-2-yl)-1*H*-pyrrolo[2,3-*b*]pyridin-3-yl)pyrimidin-2-amine (compound **1A17**, Table 1)
4-(5-(benzofuran-2-yl)-1*H*-pyrrolo[2,3-*b*]pyridin-3-yl)-*N*-cyclopropylpyrimidin-2-amine (compound **1A18**, Table 1)
4-(5-(1*H*-indol-2-yl)-1*H*-pyrrolo[2,3-*b*]pyridin-3-yl)-*N*-cyclopropylpyrimidin-2-amine (compound **1A19**, Table 1)
*N*-cyclopropyl-4-(5-(isoquinolin-1-yl)-1*H*-pyrrolo[2,3-*b*]pyridin-3-yl)pyrimidin-2-amine (compound **1A20**, Table 1)
*N*-cyclopentyl-4-(5-(4-(trifluoromethyl)phenyl)-1*H*-pyrrolo[2,3-*b*]pyridin-3-yl)pyrimidin-2-amine (compound **1A21**, Table 2)
*N*-cyclopentyl-4-(5-(4-fluorophenyl)-1*H*-pyrrolo[2,3-*b*]pyridin-3-yl)pyrimidin-2-amine(compound**1A22**, Table 2)
*N*-cyclopentyl-4-(5-(4-(trifluoromethoxy)phenyl)-1*H*-pyrrolo[2,3-*b*]pyridin-3-yl)pyrimidin-2-amine (compound **1A23**, Table 2)
*N*-cyclopentyl-4-(5-(3-(trifluoromethyl)phenyl)-1*H*-pyrrolo[2,3-*b*]pyridin-3-yl)pyrimidin-2-amine (compound **1A24**, Table 2)
*N*-cyclopentyl-4-(5-(3-fluorophenyl)-1*H*-pyrrolo[2,3-*b*]pyridin-3-yl)pyrimidin-2-amine(compound**1A25**, Table 2)
*N*-cyclopentyl-4-(5-(p-tolyl)-1*H*-pyrrolo[2,3-*b*]pyridin-3-yl)pyrimidin-2-amine(compound **1A26**, Table 2)
*N*-cyclopentyl-4-(5-(m-tolyl)-1*H*-pyrrolo[2,3-*b*]pyridin-3-yl)pyrimidin-2-amine(compound**1A27**, Table 2)
*N*-cyclopentyl-4-(5-(4-(methylthio)phenyl)-1*H*-pyrrolo[2,3-*b*]pyridin-3-yl)pyrimidin-2-amine(compound **1A28**, Table 2)
*N*-cyclopentyl-4-(5-(2-(methylthio)phenyl)-1*H*-pyrrolo[2,3-*b*]pyridin-3-yl)pyrimidin-2-amine(compound **1A29**, Table 2)
*N*-cyclopentyl-4-(5-(2-ethylphenyl)-1*H*-pyrrolo[2,3-*b*]pyridin-3-yl)pyrimidin-2-amine(compound**1A30**, Table 2)
*N*-cyclopentyl-4-(5-(4-methoxyphenyl)-1*H*-pyrrolo[2,3-*b*]pyridin-3-yl)pyrimidin-2-amine (compound **1A31**, Table 2)
4-(5-(4-chlorophenyl)-1*H*-pyrrolo[2,3-*b*]pyridin-3-yl)-*N*-cyclopentylpyrimidin-2-amine(compound**1A32**, Table 2)
4-(3-(2-(cyclopentylamino)pyrimidin-4-yl)-1*H*-pyrrolo[2,3-*b*]pyridin-5-yl)benzaldehyde (compound **1A33**, Table 2)
*N*-cyclopentyl-4-(5-(naphthalen-1-yl)-1*H*-pyrrolo[2,3-*b*]pyridin-3-yl)pyrimidin-2-amine(compound **1A34**, Table 2)
*N*-cyclopentyl-4-(5-(pyridin-3-yl)-1*H*-pyrrolo[2,3-*b*]pyridin-3-yl)pyrimidin-2-amine(compound**1A35**, Table 2)
*N*-cyclopentyl-4-(5-(furan-3-yl)-1*H*-pyrrolo[2,3-*b*]pyridin-3-yl)pyrimidin-2-amine(compound**1A36**, Table 2)
*N*-cyclopentyl-4-(5-(5-methoxy-1*H*-indol-2-yl)-1*H*-pyrrolo[2,3-*b*]pyridin-3-yl)pyrimidin-2-amine (compound **1A37**, Table 2)
4-(5-(benzo[*b*]thiophen-2-yl)-1*H*-pyrrolo[2,3-*b*]pyridin-3-yl)*N*-cyclopentylpyrimidin-2-amine (compound **1A38**, Table 2)
4-(5-(1*H*-indol-2-yl)-1*H*-pyrrolo[2,3-*b*]pyridin-3-yl)-*N*-cyclopentylpyrimidin-2-amine(compound**1A39**, Table 2)
*N*-cyclopentyl-4-(5-(isoquinolin-1-yl)-1*H-*pyrrolo[2,3-*b*]pyridin-3-yl)pyrimidin-2-amine(compound **1A40**, Table 2)
*N*-cycloheptyl-4-(5-(4-(trifluoromethyl)phenyl)-1*H*-pyrrolo[2,3-*b*]pyridin-3-yl)pyrimidin-2-amine (compound **1A41**, Table 3)
*N*-cycloheptyl-4-(5-(4-fluorophenyl)-1*H*-pyrrolo[2,3-*b*]pyridin-3-yl)pyrimidin-2-amine(compound**1A42**, Table 3)
*N*-cycloheptyl-4-(5-(4-(trifluoromethoxy)phenyl)-1*H*-pyrrolo[2,3-*b*]pyridin-3-yl)pyrimidin-2-amine (compound **1A43**, Table 3)
*N*-cycloheptyl-4-(5-(3-(trifluoromethyl)phenyl)-1*H*-pyrrolo[2,3-*b*]pyridin-3-yl)pyrimidin-2-amine (compound **1A44**, Table 3)
*N*-cycloheptyl-4-(5-(3-fluorophenyl)-1*H*-pyrrolo[2,3-*b*]pyridin-3-yl)pyrimidin-2-amine(compound**1A45**, Table 3)
*N*-cycloheptyl-4-(5-(p-tolyl)-1*H*-pyrrolo[2,3-*b*]pyridin-3-yl)pyrimidin-2-amine(compound**1A46**, Table 3)
*N*-cycloheptyl-4-(5-(m-tolyl)-1*H*-pyrrolo[2,3-*b*]pyridin-3-yl)pyrimidin-2-amine(compound**1A47**, Table 3)
*N*-cycloheptyl-4-(5-(4-(methylthio)phenyl)-1*H*-pyrrolo[2,3-*b*]pyridin-3-yl)pyrimidin-2-amine(compound **1A48**, Table 3)
*N*-cycloheptyl-4-(5-(2-(methylthio)phenyl)-1*H*-pyrrolo[2,3-*b*]pyridin-3-yl)pyrimidin-2-amine(compound **1A49**, Table 3)
*N*-cycloheptyl-4-(5-(2-ethylphenyl)-1*H*-pyrrolo[2,3-*b*]pyridin-3-yl)pyrimidin-2-amine(compound**1A50**, Table 3)
*N*-cycloheptyl-4-(5-(4-methoxyphenyl)-1*H*-pyrrolo[2,3-*b*]pyridin-3-yl)pyrimidin-2-amine(compound **1A51**, Table 3)
4-(5-(4-chlorophenyl)-1*H*-pyrrolo[2,3-*b*]pyridin-3-yl)-*N*-cycloheptylpyrimidin-2-amine(compound**1A52**, Table 3)
4-(3-(2-(cycloheptylamino)pyrimidin-4-yl)-1*H*-pyrrolo[2,3-*b*]pyridin-5-yl)benzaldehyde(compound **1A53**, Table 3)
*N*-cycloheptyl-4-(5-(naphthalen-1-yl)-1*H*-pyrrolo[2,3-*b*]pyridin-3-yl)pyrimidin-2-amine(compound **1A54**, Table 3)
*N*-cycloheptyl-4-(5-(pyridin-3-yl)-1*H*-pyrrolo[2,3-*b*]pyridin-3-yl)pyrimidin-2-amine(compound1**A55**, Table 3)
*N*-cycloheptyl-4-(5-(furan-3-yl)-1*H*-pyrrolo[2,3-*b*]pyridin-3-yl)pyrimidin-2-amine(compound**1A56**, Table 3)
*N*-cycloheptyl-4-(5-(5-methoxy-1*H*-indol-2-yl)-1*H*-pyrrolo[2,3-*b*]pyridin-3-yl)pyrimidin-2-amine (compound **1A57**, Table 3)
4-(5-(benzo[*b*]thiophen-2-yl)-1*H*-pyrrolo[2,3-*b*]pyridin-3-yl)-*N*-cycloheptylpyrimidin-2-amine (compound **1A58**, Table 3)
4-(5-(1*H*-indol-2-yl)-1*H*-pyrrolo[2,3-*b*]pyridin-3-yl)-*N*-cycloheptylpyrimidin-2-amine(compound**1A59**, Table 3)
*N*-cycloheptyl-4-(5-(isoquinolin-1-yl)-1*H-*pyrrolo[2,3-*b*]pyridin-3-yl)pyrimidin-2-amine (compound **1A60**, Table 3)
*N*-phenethyl-4-(5-(4-(trifluoromethyl)phenyl)-1*H*-pyrrolo[2,3-*b*]pyridin-3-yl)pyrimidin-2-amine (compound **1B1**, Table 4)
4-(5-(4-fluorophenyl)-1*H*-pyrrolo[2,3-*b*]pyridin-3-yl)-*N*-phenethylpyrimidin-2-amine(compound**1B2**, Table 4)
*N*-phenethyl-4-(5-(4-(trifluoromethoxy)phenyl)-1*H*-pyrrolo[2,3-*b*]pyridin-3-yl)pyrimidin-2-amine (compound **1B3**, Table 4)
*N*-phenethyl-4-(5-(3-(trifluoromethyl)phenyl)-1*H*-pyrrolo[2,3-*b*]pyridin-3-yl)pyrimidin-2-amine (compound **1B4**, Table 4)
4-(5-(3-fluorophenyl)-1*H*-pyrrolo[2,3-*b*]pyridin-3-yl)-*N*-phenethylpyrimidin-2-amine(compound**1B5**, Table 4)
*N*-phenethyl-4-(5-(p-tolyl)-1*H*-pyrrolo[2,3-*b*]pyridin-3-yl)pyrimidin-2-amine (compound **1B6**, Table 4)
*N*-phenethyl-4-(5-(m-tolyl)-1*H*-pyrrolo[2,3-*b*]pyridin-3-yl)pyrimidin-2-amine (compound **1B7**, Table 4)
4-(5-(4-(methylthio)phenyl)-1*H*-pyrrolo[2,3-*b*]pyridin-3-yl)-*N*-phenethylpyrimidin-2-amine(compound **1B8**, Table 4)
4-(5-(2-(methylthio)phenyl)-1*H*-pyrrolo[2,3-*b*]pyridin-3-yl)-*N*-phenethylpyrimidin-2-amine(compound **1B9**, Table 4)
4-(5-(2-ethylphenyl)-1*H*-pyrrolo[2,3-*b*]pyridin-3-yl)-*N-*phenethylpyrimidin-2-amine(compound**1B10**, Table 4)
4-(5-(4-methoxyphenyl)-1*H*-pyrrolo[2,3-*b*]pyridin-3-yl)-*N*-phenethylpyrimidin-2-amine(compound**1B11**, Table 4)
4-(5-(4-chlorophenyl)-1*H*-pyrrolo[2,3-*b*]pyridin-3-yl)-*N*-phenethylpyrimidin-2-amine(compound**1B12**, Table 4)
4-(3-(2-(phenethylamino)pyrimidin-4-yl)-1*H*-pyrrolo[2,3-*b*]pyridin-5-yl)benzaldehyde(compound**1B13**, Table 4)
4-(5-(naphthalen-1-yl)-1*H*-pyrrolo[2,3-*b*]pyridin-3-yl)-*N-*phenethylpyrimidin-2-amine(compound**1B14**, Table 4)
*N*-phenethyl-4-(5-(pyridin-3-yl)-1*H*-pyrrolo[2,3-*b*]pyridin-3-yl)pyrimidin-2-amine(compound**1B15**, Table 4)
4-(5-(furan-3-yl)-1*H*-pyrrolo[2,3-*b*]pyridin-3-yl)-*N-*phenethylpyrimidin-2-amine(compound**1B16**,Table 4)
4-(5-(5-methoxy-1*H*-indol-2-yl)-1*H*-pyrrolo[2,3-*b*]pyridin-3-yl)-*N*-phenethylpyrimidin-2-amine (compound **1B17**, Table 4)
4-(5-(benzo[*b*]thiophen-2-yl)-1*H*-pyrrolo[2,3-*b*]pyridin-3-yl)-*N*-phenethylpyrimidin-2-amine(compound **1B18**, Table 4)
4-(5-(1*H*-indol-2-yl)-1*H*-pyrrolo[2,3-*b*]pyridin-3-yl)-*N-*phenethylpyrimidin-2-amine(compound**1B19**, Table 4)
4-(5-(isoquinolin-1-yl)-1*H*-pyrrolo[2,3-*b*]pyridin-3-yl)-*N*-phenethylpyrimidin-2-amine(compound**1B20**, Table 4)

Compounds of Formula 1C are provided for reference and include:
4-(5-(4-(trifluoromethyl)phenyl)-1*H*-pyrrolo[2,3-*b*]pyridin-3-yl)pyrimidin-2-amine(compound**1C1**,Table 5)
4-(5-(4-fluorophenyl)-1*H*-pyrrolo[2,3-*b*]pyridin-3-yl)pyrimidin-2-amine(compound **1C2**, Table 5)
4-(5-(4-(trifluoromethoxy)phenyl)-1*H*-pyrrolo[2,3-*b*]pyridin-3-yl)pyrimidin-2-amine(compound**1C3**, Table 5)
4-(5-(3-(trifluoromethyl)phenyl)-1*H*-pyrrolo[2,3-*b*]pyridin-3-yl)pyrimidin-2-amine(compound**1C4**, Table 5)
4-(5-(3-fluorophenyl)-1*H*-pyrrolo[2,3-*b*]pyridin-3-yl)pyrimidin-2-amine (compound **1C5**, Table 5)
4-(5-(p-tolyl)-1*H*-pyrrolo[2,3-*b*]pyridin-3-yl)pyrimidin-2-amine (compound **1C6**, Table 5)
4-(5-(m-tolyl)-1*H*-pyrrolo[2,3-*b*]pyridin-3-yl)pyrimidin-2-amine (compound **1C7**, Table 5)
4-(5-(4-(methylthio)phenyl)-1*H*-pyrrolo[2,3-*b*]pyridin-3-yl)pyrimidin-2-amine (compound **1C8**, Table 5)
4-(5-(2-(methylthio)phenyl)-1*H*-pyrrolo[2,3-*b*]pyridin-3-yl)pyrimidin-2-amine (compound **1C9**, Table 5)
4-(5-(2-ethylphenyl)-1*H*-pyrrolo[2,3-*b*]pyridin-3-yl)pyrimidin-2-amine (compound **1C10**, Table 5)
4-(5-(4-methoxyphenyl)-1*H*-pyrrolo[2,3-*b*]pyridin-3-yl)pyrimidin-2-amine (compound **1C11**, Table 5)
4-(5-(4-chlorophenyl)-1*H*-pyrrolo[2,3-*b*]pyridin-3-yl)pyrimidin-2-amine (compound **1C12**, Table 5)
4-(3-(2-aminopyrimidin-4-yl)-1*H*-pyrrolo[2,3-*b*]pyridin-5-yl)benzaldehyde (compound **1C13**, Table 5)
4-(5-(naphthalen-1-yl)-1*H*-pyrrolo[2,3-*b*]pyridin-3-yl)pyrimidin-2-amine (compound **1C14**, Table 5)
4-(5-(pyridin-3-yl)-1*H*-pyrrolo[2,3-*b*]pyridin-3-yl)pyrimidin-2-amine (compound **1C15**, Table 5)
4-(5-(furan-3-yl)-1*H*-pyrrolo[2,3-*b*]pyridin-3-yl)pyrimidin-2-amine (compound **1C16**, Table 5)
4-(5-(5-methoxy-1*H*-indol-2-yl)-1*H*-pyrrolo[2,3-*b*]pyridin-3-yl)pyrimidin-2-amine(compound**1C17**, Table 5)
4-(5-(benzo[*b*]thiophen-2-yl)-1*H*-pyrrolo[2,3-*b*]pyridin-3-yl)pyrimidin-2-amine(compound**1C18**,Table 5)
4-(5-(1*H*-dol-2-yl)-1*H*-pyrrolo[2,3-*b*]pyridin-3-yl)pyrimidin-2-amine (compound **1C19**, Table 5)
4-(5-(isoquinolin-1-yl)-1*H*-pyrrolo[2,3-*b*]pyridin-3-yl)pyrimidin-2-amine (compound **1C20**, Table 5)

### EXAMPLES

The invention is further described by reference to following examples which are intended to illustrate, not to limit the scope of the invention.

### Example 1:

### General procedure for the preparation of compounds (IA1-20):

*meta*-Chloroperbenzoic acid (*m*-CPBA) (1.5 mmol) was added portion wise to an ice cooled solution of 2-thiomethyl-4-chloropyrimidine **1** (1 mmol) in dichloromethane and resulting reaction mixture was stirred at 25⁰C for 6-12 hrs. After completion of reaction, monitored by thin layer chromatography and reaction mixture was washed with saturated solution of sodium bicarbonate, extracted with dichloromethane and concentrated in- vaccuo to obtain beige solid 2, which was used without further purification for the next step. The obtained solid was dissolved in tetrahydrofuran and cyclopropyl amine (1.2 mmol) added, stirred at room temperature at 25 ⁰C for 2 hr. After completion of reaction, solvent was evaporated in-vaccuo and purified chromatographically using silica gel of 60-120 mesh size to obtain **3** (Young-Choon Moon, et al., Synthesis 2013, 45, 1764-1784).

{1,1-Bis(diphenylphosphino)ferrocene dichloride palladium(II)} (0.05 eq.) was added to the suspension of 5-bromo-7-azaindole **8** (1 mmol), substituted boronic acid 9 (1.5 mmol) and potassium carbonate (2.5 mmol) in dioxane and water (2.5: 1 ml) ratio. The resulting mixture was stirred at 80 °C for 6 h. After cooling to at 25 ⁰C, all solvents were evaporated in-vaccuo and the residue was filtered onto thin pad of celite and purified chromatographically on silica gel with hexane/ethyl acetate to obtain **10** (Robert H. Dodd, et al., J. Med. Chem. 2013, 56, 9569-9585).

A solution of iodine (1 mmol) in 20 mL DMF was dropped to the solution of substituted 7-azaindole **10** (1 mmol) and potassium hydroxide (2.5 mmol) in 20 mL DMF at 25 ⁰C and the mixture was stirred for 45 min. The reaction mixture was then poured on 50 ml ice water containing 1 % ammonia and 0.2 % sodium disulfite. The precipitate was filtered, washed with ice water and dried in vacuo to obtain a yellow solid **11**. The obtained solid was used without further purification for the next step. It was suspended in 10 mL dichloromethane, 4-dimethylaminopyridine (0.1 mmol) was added and di-*tert*-butyl dicarbonate (1.2 mmol), dissolved in 20 mL dichloromethane, was added drop wise for 30 min. The mixture was stirred for 30 min. at 25 ⁰C, washed with 20 mL 0.1 *N* HCl*,* and the aqueous phase was extracted with dichloromethane (2 × 50 mL). The combined organic layers were dried with sodium sulphate, the solvents were removed under reduced pressure and the residue was adsorbed onto Celite and purified chromatographically on silica gel with hexane/ethyl acetate to obtain (92 % total yield over two steps) **12**, which solidifies upon storage in refrigerator.

Tetrakis (triphenylphosphane)-palladium (0) (3 mol %) and 5-substituted *tert*-butyl 3-iodo-1*H*-pyrrolo[2,3-*b*]pyridine-1-carboxylate (**12**) (1.00 mmol) were placed under argon atmosphere in a dry screw-cap vessel with septum. Then, 5 mL of dry dioxane were added and the mixture was degassed with argon. Dry triethylamine (10.0 mmol, 10.0 equiv), and 4,4,5,5-tetramethyl-1,3,2-dioxaborolane (1.50 mmol, 1.50 equiv) were successively added to the mixture which was stirred at 80 °C (preheated oil bath) for 3 h to obtain **13** (monitored by TLC). Then, after cooling to at 25 ⁰C (water bath), 5 mL of dry methanol, 1.00 mmol of 4-chloro-N-cyclopropylpyrimidin-2-amine **(3)** and cesium carbonate (2.50 mmol, 2.50 equiv) were successively added and the mixture was stirred at 100 °C for 36 h. Then, after cooling at 25 ⁰C (water bath) the solvents were removed in vaccuo and the residue was absorbed onto Celite and purified chromatographically on silica gel with dichloromethane-methanol-aqueous ammonia (isocratic or stepwise gradient). The obtained bis(hetero)aryls **1A (1-20)** can be further purified by suspending in dichloromethane, sonication in ultrasound bath for 0.5-1.0 h, filtration and drying in vacuo overnight for 12 h to obtained the compounds of Formula **1A (1-20)**

### Example 2:

### General procedure for the preparation of compounds (IA21-40):

*meta*-Chloroperbenzoic acid (*m*-CPBA) (1.5 mmol) was added portion wise to an ice cooled solution of 2-thiomethyl-4-chloropyrimidine **1** (1 mmol) in dichloromethane and resulting reaction mixture was stirred at 25 ⁰Cfor 6-12 hrs. After completion of reaction, monitored by thin layer chromatography and reaction mixture was washed with saturated solution of sodium bicarbonate, extracted with dichloromethane and concentrated in- vaccuo to obtain beige solid **2**, which was used without further purification for the next step. The obtained solid was dissolved in tetrahydrofuran and cyclopentyl amine (1.2 mmol) added, stirred at 25 ⁰Cfor 2 hr. After completion of reaction, solvent was evaporated in-vaccuo and purified chromatographically using silica gel of 60-120 mesh size to obtain **4**. (Young-Choon Moon, et al., Synthesis 2013, 45, 1764-1784)

{1,1-Bis(diphenylphosphino)ferrocene dichloride palladium(II)} (0.05 eq.) was added to the suspension of 5-bromo-7-azaindole **8** (1 mmol), substituted boronic acid 9 (1.5 mmol) and potassium carbonate (2.5 mmol) in dioxane and water (2.5 : 1 ml) ratio. The resulting mixture was stirred at 80 °C for 6 h. After cooling to at 25 ⁰C, all solvents were evaporated in-vaccuo and the residue was filtered onto thin pad of celite and purified chromatographically on silica gel with hexane/ethyl acetate to obtain **10** (Robert H. Dodd, et al., J. Med. Chem. 2013, 56, 9569-9585)

A solution of iodine (1 mmol) in 20 mL DMF was dropped to the solution of substituted 7-azaindole **10** (1 mmol) and potassium hydroxide (2.5 mmol) in 20 mL DMF at 25 ⁰Cand the mixture was stirred for 45 min. The reaction mixture was then poured on 50 ml ice water containing 1 % ammonia and 0.2 % sodium disulfite. The precipitate was filtered, washed with ice water and dried in vaccuo to obtain a yellow solid **11**. The obtained solid was used without further purification for the next step. It was suspended in 10 mL dichloromethane, 4-dimethylaminopyridine (0.1 mmol) was added and di-*tert*-butyl dicarbonate (1.2 mmol), dissolved in 20 mL dichloromethane, was added drop wise for 30 min. The mixture was stirred for 30 min. at 25 ⁰C, washed with 20 mL 0.1 *N* HCl*,* and the aqueous phase was extracted with dichloromethane (2 × 50 mL). The combined organic layers were dried with sodium sulphate, the solvents were removed under reduced pressure and the residue was adsorbed onto Celite and purified chromatographically on silica gel with hexane/ethyl acetate to obtain (92 % total yield over two steps) **12**, which solidifies upon storage in refrigerator.

Tetrakis (triphenylphosphane)-palladium (0) (3 mol %) and 5-substituted *tert*-butyl 3-iodo-1*H-*pyrrolo[2,3-*b*]pyridine-1-carboxylate (**12**) (1.00 mmol) were placed under argon atmosphere in a dry screw-cap vessel with septum. Then, 5 mL of dry dioxane were added and the mixture was degassed with argon. Dry triethylamine (10.0 mmol, 10.0 equiv), and 4,4,5,5-tetramethyl-1,3,2- dioxaborolane (1.50 mmol, 1.50 equiv) were successively added to the mixture which was stirred at 80 °C (preheated oil bath) for 3 h to obtain **13** (monitored by TLC). Then, after cooling to at 25 ⁰C (water bath), 5 mL of dry methanol, 1.00 mmol of 4-chloro-N-cyclopentylpyrimidin-2-amine **(4)** and cesium carbonate (2.50 mmol, 2.50 equiv) were successively added and the mixture was stirred at 100 °C for 36 h. Then, after cooling to room temperature at 25 ⁰C (water bath) the solvents were removed in vaccuo and the residue was absorbed onto Celite and purified chromatographically on silica gel with dichloromethane-methanol-aqueous ammonia (isocratic or stepwise gradient). The obtained bis(hetero)aryls **1A (21-40)** can be further purified by suspending in dichloromethane, sonication in ultrasound bath for 0.5-1.0 h, filtration and drying in vaccuo overnight to obtained the compounds of Formula **1A (21-40).**

### Example 3:

### General procedure for the preparation of compounds (IA41-60):

*meta*-Chloroperbenzoic acid (*m*-CPBA) (1.5 mmol) was added portion wise to an ice cooled solution of 2-thiomethyl-4-chloropyrimidine **1** (1 mmol) in dichloromethane and resulting reaction mixture was stirred at 25 ⁰Cfor 6-12 hrs. After completion of reaction, monitored by thin layer chromatography and reaction mixture was washed with saturated solution of sodium bicarbonate, extracted with dichloromethane and concentrated in- vaccuo to obtain beige solid **2**, which was used without further purification for the next step. The obtained solid was dissolved in tetrahydrofuran and cycloheptyl amine (1.2 mmol) added, stirred at 25 ⁰Cfor 2 hr. After completion of reaction, solvent was evaporated in-vaccuo and purified chromatographically using silica gel of 60-120 mesh size to obtain **5** (Young-Choon Moon, et al., Synthesis 2013, 45, 1764-1784).

{1,1-Bis(diphenylphosphino)ferrocene dichloride palladium(II)} (0.05 eq.) was added to the suspension of 5-bromo-7-azaindole **8** (1 mmol), substituted boronic acid 9 (1.5 mmol) and potassium carbonate (2.5 mmol) in dioxane and water (2.5 : 1 ml) ratio. The resulting mixture was stirred at 80 °C for 6 h to overnight (25 ⁰C). After cooling to room temperature at 25 ⁰C, all solvents were evaporated in-vaccuo and the residue was filtered onto thin pad of celite and purified chromatographically on silica gel with hexane/ethyl acetate to obtain **10** (Robert H. Dodd, et al., J. Med. Chem. 2013, 56, 9569-9585).

A solution of iodine (1 mmol) in 20 mL DMF was dropped to the solution of substituted 7-azaindole **10** (1 mmol) and potassium hydroxide (2.5 mmol) in 20 mL DMF at 25 ⁰C and the mixture was stirred for 45 min. The reaction mixture was then poured on 50 ml ice water containing 1 % ammonia and 0.2 % sodium disulfite. The precipitate was filtered, washed with ice water and dried in vaccuo to obtain a yellow solid **11**. The obtained solid was used without further purification for the next step. It was suspended in 10 mL dichloromethane, 4-dimethylaminopyridine (0.1 mmol) was added and di-*tert*-butyl dicarbonate (1.2 mmol), dissolved in 20 mL dichloromethane, was added drop wise for 30 min. The mixture was stirred for 30 min. at 25 ⁰C, washed with 20 mL 0.1 *N* HCl*,* and the aqueous phase was extracted with dichloromethane (2 × 50 mL). The combined organic layers were dried with sodium sulphate, the solvents were removed under reduced pressure and the residue was adsorbed onto Celite and purified chromatographically on silica gel with hexane/ethyl acetate to obtain (92 % total yield over two steps) **12**, which solidifies upon storage in refrigerator.

Tetrakis (triphenylphosphane)-palladium (0) (3 mol %) and 5-substituted *tert*-butyl 3-iodo-1*H*-pyrrolo[2,3-*b*]pyridine-1-carboxylate (**12**) (1.00 mmol) were placed under argon atmosphere in a dry screw-cap vessel with septum. Then, 5 mL of dry dioxane were added and the mixture was degassed with argon. Dry triethylamine (10.0 mmol, 10.0 equiv), and 4,4,5,5-tetramethyl-1,3,2-dioxaborolane (1.50 mmol, 1.50 equiv) were successively added to the mixture which was stirred at 80 °C (preheated oil bath) for 3 h to obtain **13** (monitored by TLC). Then, after cooling to room temperature (water bath), 5 mL of dry methanol, 1.00 mmol of 4-chloro-N-cycloheptylpyrimidin-2-amine **(5)** and cesium carbonate (2.50 mmol, 2.50 equiv) were successively added and the mixture was stirred at 100 °C for 36 h. Then, after cooling to at 25 ⁰C (water bath) the solvents were removed in vaccuo and the residue was absorbed onto Celite and purified chromatographically on silica gel with dichloromethane-methanol-aqueous ammonia (isocratic or stepwise gradient). The obtained bis(hetero)aryls **1A (41-60)** can be further purified by suspending in dichloromethane, sonication in ultrasound bath for 0.5-1.0 h, filtration and drying in vaccuo overnight 12 h to obtained the compounds of Formula **1A (41-60).**

### Example 4:

### General procedure for the preparation of compounds 1B (1-20):

*meta*-Chloroperbenzoic acid (*m*-CPBA) (1.5 mmol) was added portion wise to an ice cooled solution of 2-thiomethyl-4-chloropyrimidine **1** (1 mmol) in dichloromethane and resulting reaction mixture was stirred at 25 ⁰Cfor 6-12 hrs. After completion of reaction, monitored by thin layer chromatography and reaction mixture was washed with saturated solution of sodium bicarbonate, extracted with dichloromethane and concentrated in- vaccuo to obtain beige solid **2**, which was used without further purification for the next step. The obtained solid was dissolved in tetrahydrofuran and phenethyl amine (1.2 mmol) added, stirred at 25 ⁰Cfor 2 hr. After completion of reaction, solvent was evaporated in-vaccuo and purified chromatographically using silica gel of 60-120 mesh size to obtain **6** (Young-Choon Moon, et al., Synthesis 2013, 45, 1764-1784).

{1,1-Bis(diphenylphosphino)ferrocene dichloride palladium (II)} (0.05 eq.) was added to the suspension of 5-bromo-7-azaindole **8** (1 mmol), substituted boronic acid 9 (1.5 mmol) and potassium carbonate (2.5 mmol) in dioxane and water (2.5 : 1 ml) ratio. The resulting mixture was stirred at 80 °C for 6 h. After cooling to room temperature at 25 ⁰C, all solvents were evaporated in-vaccuo and the residue was filtered onto thin pad of celite and purified chromatographically on silica gel with hexane/ethyl acetate to obtain **10** (Robert H. Dodd, et al., J. Med. Chem. 2013, 56, 9569-9585).

A solution of iodine (1 mmol) in 20 mL DMF was dropped to the solution of substituted 7-azaindole **10** (1 mmol) and potassium hydroxide (2.5 mmol) in 20 mL DMF at 25 ⁰Cand the mixture was stirred for 45 min. The reaction mixture was then poured on 50 ml ice water containing 1 % ammonia and 0.2 % sodium disulfite. The precipitate was filtered, washed with ice water and dried in vaccuo to obtain a yellow solid **11**. The obtained solid was used without further purification for the next step. It was suspended in 10 mL dichloromethane, 4-dimethylaminopyridine (0.1 mmol) was added and di-*tert*-butyl dicarbonate (1.2 mmol), dissolved in 20 mL dichloromethane, was added drop wise for 30 min. The mixture was stirred for 30 min. at 25 ⁰C, washed with 20 mL 0.1 *N* HCl*,* and the aqueous phase was extracted with dichloromethane (2 × 50 mL). The combined organic layers were dried with sodium sulphate, the solvents were removed under reduced pressure and the residue was adsorbed onto Celite and purified chromatographically on silica gel with hexane/ethyl acetate to obtain (92 % total yield over two steps) **12**, which solidifies upon storage in refrigerator.

Tetrakis (triphenylphosphane)-palladium (0) (3 mol %) and 5-substituted *tert*-butyl 3-iodo-1*H*-pyrrolo[2,3-*b*]pyridine-1-carboxylate (**12**) (1.00 mmol) were placed under argon atmosphere in a dry screw-cap vessel with septum. Then, 5 mL of dry dioxane were added and the mixture was degassed with argon. Dry triethylamine (10.0 mmol, 10.0 equiv), and 4,4,5,5-tetramethyl-1,3,2-dioxaborolane (1.50 mmol, 1.50 equiv) were successively added to the mixture which was stirred at 80 °C (preheated oil bath) for 3 h to obtain **13** (monitored by TLC). Then, after cooling to at 25 ⁰C (water bath), 5 mL of dry methanol, 1.00 mmol of 4-chloro-N-phenethylpyrimidin-2-amine **(6)** and cesium carbonate (2.50 mmol, 2.50 equiv) were successively added and the mixture was stirred at 100 °C for 36 h. Then, after cooling to at 25 ⁰C (water bath) the solvents were removed in vacuo and the residue was absorbed onto Celite and purified chromatographically on silica gel with dichloromethane-methanol-aqueous ammonia (isocratic or stepwise gradient). The obtained bis(hetero)aryls **1B (1-20)** can be further purified by suspending in dichloromethane, sonication in ultrasound bath for 0.5-1.0 h, filtration and drying in vaccuo overnight 25 ⁰C to obtained the compounds of Formula **1B (1-20).**

### Example 5:

### General procedure for the preparation of compounds 1C (1-20) (for reference):

{1,1-Bis(diphenylphosphino)ferrocene dichloride palladium(II)} (0.05 eq.) was added to the suspension of 5-bromo-7-azaindole **8** (1 mmol), substituted boronic acid 9 (1.5 mmol) and potassium carbonate (2.5 mmol) in dioxane and water (2.5 : 1 ml) ratio. The resulting mixture was stirred at 80 °C for 6 h. After cooling to at 25 ⁰C, all solvents were evaporated in-vaccuo and the residue was filtered onto thin pad of celite and purified chromatographically on silica gel with hexane/ethyl acetate to obtain **10** (Robert H. Dodd, et al., J. Med. Chem. 2013, 56, 9569-9585).

A solution of iodine (1 mmol) in 20 mL DMF was dropped to the solution of substituted 7-azaindole **10** (1 mmol) and potassium hydroxide (2.5 mmol) in 20 mL DMF at 5 ⁰C and the mixture was stirred for 45 min. The reaction mixture was then poured on 50 ml ice water containing 1 % ammonia and 0.2 % sodium disulfite. The precipitate was filtered, washed with ice water and dried in vaccuo to obtain a yellow solid **11**. The obtained solid was used without further purification for the next step. It was suspended in 10 mL dichloromethane, 4-dimethylaminopyridine (0.1 mmol) was added and di-*tert*-butyl dicarbonate (1.2 mmol), dissolved in 20 mL dichloromethane, was added drop wise for 30 min. The mixture was stirred for 30 min. at 25 ⁰C, washed with 20 mL 0.1 *N* HCl, and the aqueous phase was extracted with dichloromethane (2 × 50 mL). The combined organic layers were dried with sodium sulphate, the solvents were removed under reduced pressure and the residue was adsorbed onto Celite and purified chromatographically on silica gel with hexane/ethyl acetate to obtain ( 94 % yield; 92 % total yield over two steps) of **12**, which solidifies upon storage in refrigerator.

Tetrakis (triphenylphosphane)-palladium (0) (3 mol %) and 5-substituted *tert*-butyl 3-iodo-1*H-*pyrrolo[2,3-*b*]pyridine-1-carboxylate (**12**) (1.00 mmol) were placed under argon atmosphere in a dry screw-cap vessel with septum. Then, 5 mL of dry dioxane were added and the mixture was degassed with argon. Dry triethylamine (10.0 mmol, 10.0 equiv), and 4,4,5,5-tetramethyl-1,3,2- dioxaborolane (1.50 mmol, 1.50 equiv) were successively added to the mixture which was stirred at 80 °C (preheated oil bath) for 3 h to obtain **13** (monitored by TLC). Then, after cooling to at 25 ⁰C (water bath), 5 mL of dry methanol, 1.00 mmol of 2-amino-4-chloropyrimidine and cesium carbonate (2.50 mmol, 2.50 equiv) were successively added and the mixture was stirred at 100 °C for 36 h. Then, after cooling to at 25 ⁰C (water bath) the solvents were removed in vacuo and the residue was absorbed onto Celite and purified chromatographically on silica gel with dichloromethane-methanol-aqueous ammonia (isocratic or stepwise gradient). The obtained bis(hetero)aryls **1C (1-20)** can be further purified by suspending in dichloromethane, sonication in ultrasound bath for 0.5-1.0 h, filtration and drying in vaccuo overnight 12 h to obtained the compounds of Formula **1C (1-20).**

### N-cyclopropyl-4-(5-(4-(trifluoromethyl)phenyl)-1H-pyrrolo[2,3-b]pyridin-3-yl)pyrimidin-2-amine (compound 1A1, Table 1, Fig. 1)

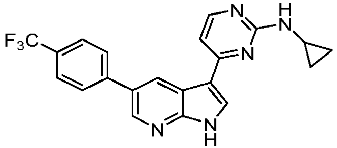

**¹H NMR (400 MHz, DMSO) δ** 12.41 (s, 1H), 9.31 (s, 1H), 8.65 (d, *J* = 2.2 Hz, 1H), 8.49 (d, *J* = 2.7 Hz, 1H), 8.22 (d, *J* = 5.2 Hz, 1H), 7.96 (d, *J* = 7.7 Hz, 2H), 7.86 (d, *J* = 8.3 Hz, 2H), 7.33 (d, *J* = 1.8 Hz, 1H), 7.16 (d, *J* = 5.3 Hz, 1H), 1.23 (s, 1H), 0.74 - 0.65 (m, 2H), 0.58 - 0.49 (m, 2H); MS (ESI+): m\z 395.14.

### N-cyclopropyl-4-(5-(4-fluorophenyl)-1H-pyrrolo[2,3-b]pyridin-3-yl)pyrimidin-2-amine (compound 1A2, Table 1, Fig. 1)

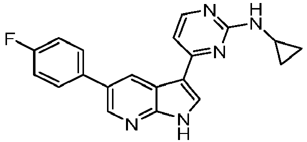

**¹H NMR (500 MHz, DMSO) δ** 12.34 (s, 1H), 9.23 (s, 1H), 8.56 (d, *J* = 2.2 Hz, 1H), 8.46 (s, 1H), 8.21 (s, 1H), 7.75 (s, 2H), 7.34 (d, *J* = 8.5 Hz, 3H), 7.15 (d, *J* = 5.1 Hz, 1H), 1.07 (s, 1H), 0.70 - 0.62 (m, 2H), 0.6 - 0.49 (m, 2H); MS (ESI+): m\z 345.14.

### N-cyclopropyl-4-(5-(4-(trifluoromethoxy)phenyl)-1H-pyrrolo[2,3-b]pyridin-3-yl)pyrimidin-2-amine (compound 1A3, Table 1, Fig. 1)

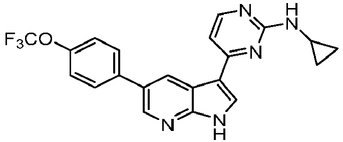

**¹H NMR (400 MHz, DMSO) δ** 12.35 (s, 1H), 9.24 (s, 1H), 8.59 (d, *J* = 2.2 Hz, 1H), 8.45 (d, *J* = 2.6 Hz, 1H), 8.21 (d, *J* = 5.2 Hz, 1H), 7.85 (d, *J* = 8.4Hz, 2H), 7.49 (d, *J* = 8.0 Hz, 2H), 7.30 (s, 1H), 7.15 (d, *J* = 5.3 Hz, 1H), 1.23 (t, *J* = 7.2 Hz, 1H), 0.75 - 0.66 (m, 2H), 0.60 - 0.48 (m, 2H); MS (ESI+): m\z 411.13.

### N-cyclopropyl-4-(5-(3-(trifluoromethyl)phenyl)-1H-pyrrolo[2,3-b]pyridin-3-yl)pyrimidin-2-amine (compound 1A4, Table 1, Fig. 1)

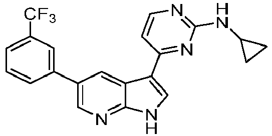

**¹H NMR (400 MHz, DMSO) δ** 12.40 (s, 1H), 9.34 (s, 1H), 8.67 (d, *J* = 1.7 Hz, 1H), 8.49 (d, *J* = 2.3 Hz, 1H), 8.21 (d, *J* = 5.1 Hz, 1H), 8.07 (d, *J* = 4.0 Hz, 1H), 7.98 (s, 1H), 7.75 (d, *J* = 4.7 Hz, 2H), 7.33 (s, 1H), 7.17 (d, *J* = 5.3 Hz, 1H), 1.23- 1.18 (m, 1H), 0.69 - 0.59 (m, 2H), 0.58 - 0.49 (m, 2H); MS (ESI+): m\z 395.14.

### N-cyclopropyl-4-(5-(3-fluorophenyl)-1H-pyrrolo[2,3-b]pyridin-3-yl)pyrimidin-2-amine (compound 1A5, Table 1, Fig. 1)

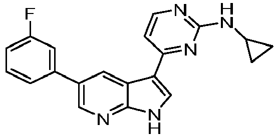

**¹H NMR (400 MHz, DMSO) δ** 12.37 (s, 1H), 9.25 (s, 1H), 8.62 (d, *J* = 2.0 Hz, 1H), 8.46 (d, *J* = 2.4 Hz, 1H), 8.21 (d, *J* = 5.0 Hz, 1H), 7.62 - 7.49 (m, 3H), 7.34 (s, 1H), 7.22 (t, *J* = 7.8 Hz, 1H), 7.16 (d, *J* = 5.2 Hz, 1H), 1.18 (t, *J* = 7.3 Hz, 1H), 0.70 (q, *J* = 6.5 Hz, 2H), 0.56 (s, 2H); MS (ESI+): m\z 345.14.

### N-cyclopropyl-4-(5-(p-tolyl)-1H-pyrrolo[2,3-b]pyridin-3-yl)pyrimidin-2-amine (compound 1A6, Table 1, Fig. 1)

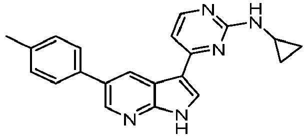

**¹H NMR (500 MHz, DMSO) δ** 12.36 (s, 1H), 9.24 (s, 1H), 8.56 (d, *J* = 2.2 Hz, 1H), 8.44 (s, 1H), 8.20 (s, 1H), 7.80 (s, 2H), 7.36 (d, *J* = 8.5 Hz, 3H), 7.13 (d, *J* = 5.1 Hz, 1H), 2.33 (s, 3H), 1.18 (s, 1H), 0.70 - 0.62 (m, 2H), 0.6 - 0.49 (m, 2H); MS (ESI+): m\z 341.16.

### N-cyclopropyl-4-(5-(m-tolyl)-1H-pyrrolo[2,3-b]pyridin-3-yl)pyrimidin-2-amine (compound 1A7, Table 1, Fig. 1)

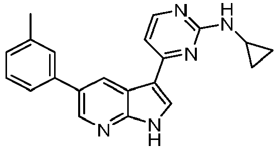

**¹H NMR (500 MHz, DMSO) δ** 12.40 (s, 1H), 9.28 (s, 1H), 8.53 (d, *J* = 2.2 Hz, 1H), 8.40 (s, 1H), 8.19 (s, 1H), 7.79 (s, 2H), 7.34 (d, *J* = 8.5 Hz, 3H), 7.18 (d, *J* = 5.1 Hz, 1H), 2.33 (s, 3H), 1.20 (s, 1H), 0.70 - 0.62 (m, 2H), 0.6 - 0.49 (m, 2H); MS (ESI+): m\z 341.16.

### N-cyclopropyl-4-(5-(4-(methylthio)phenyl)-1H-pyrrolo[2,3-b]pyridin-3-yl)pyrimidin-2-amine (compound 1A8, Table 1, Fig. 1)

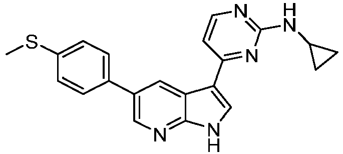

**¹H NMR (400 MHz, DMSO) δ** 12.36 (s, 1H), 9.02 (s, 1H), 8.44 (d, *J* = 2.2 Hz, 1H), 8.24 (s, 1H), 8.19 (d, *J* = 5.0 Hz, 1H), 7.70 (d, *J* = 8.4 Hz, 2H), 7.32 (d, *J* = 8.4 Hz, 3H), 7.03 (d, *J* = 5.3 Hz, 1H), 2.38 (s, 3H), 1.27 - 1.19 (m, 1H), 0.62 - 0.41 (m, 4H); MS (ESI+): m\z 373.13.

### N-cyclopropyl-4-(5-(2-(methylthio)phenyl)-1H-pyrrolo[2,3-b]pyridin-3-yl)pyrimidin-2-amine (compound 1A9, Table 1, Fig. 1)

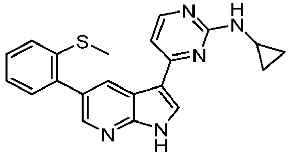

**¹H NMR (400 MHz, DMSO) δ** 12.33 (s, 1H), 9.05 (s, 1H), 8.44 (d, *J* = 2.2 Hz, 1H), 8.25 (s, 1H), 8.19 (d, *J* = 5.0 Hz, 1H), 7.46 - 7.39 (m, 1H), 7.36 (d, *J* = 7.8 Hz, 1H), 7.27 (dd, *J* = 15.7, 7.5 Hz, 3H), 7.13 (d, *J* = 5.3 Hz, 1H), 2.37 (s, 3H), 1.27 - 1.19 (m, 1H), 0.62 - 0.41 (m, 4H); MS (ESI+): m\z 373.13.

### N-cyclopropyl-4-(5-(2-ethylphenyl)-1H-pyrrolo[2,3-b]pyridin-3-yl)pyrimidin-2-amine (compound 1A10, Table 1, Fig. 1)

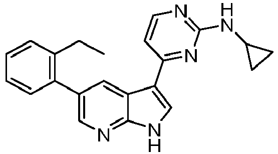

**¹H NMR (400 MHz, DMSO) δ** 12.31 (s, 1H), 8.98 (s, 1H), 8.45 (d, *J* = 2.5 Hz, 1H), 8.20 (dd, *J* = 7.6, 3.4 Hz, 2H), 7.36 (t, *J* = 6.2 Hz, 2H), 7.31 - 7.19 (m, 3H), 7.13 (d, *J* = 5.3 Hz, 1H), 2.60 (dd, *J* = 15.0, 7.5 Hz, 2H), 1.25 (s, 1H), 1.01 (t, *J* = 7.5 Hz, 3H), 0.63 - 0.36 (m, 4H); MS (ESI+): m\z 355.18.

### N-cyclopropyl-4-(5-(4-methoxyphenyl)-1H-pyrrolo[2,3-b]pyridin-3-yl)pyrimidin-2-amine (compound 1A11, Table 1, Fig. 1)

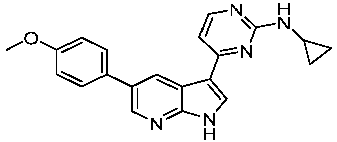

**¹H NMR (400 MHz, DMSO) δ** 12.23 (s, 1H), 9.16 (s, 1H), 8.51 (d, *J* = 2.2 Hz, 1H), 8.39 (s, 1H), 8.18 (d, *J* = 5.2 Hz, 1H), 7.63 (d, *J* = 8.3 Hz, 2H), 7.26 (d, *J* = 2.0 Hz, 1H), 7.12 (d, *J* = 5.3 Hz, 1H), 7.04 (d, *J* = 8.7 Hz, 2H), 3.80 (s, 3H), 1.21 (s, 1H), 0.78 - 0.60 (m, 2H), 0.60 - 0.44 (m, 2H); MS (ESI+): m\z 357.16.

### 4-(5-(4-chlorophenyl)-1H-pyrrolo[2,3-b]pyridin-3-yl)-N-cyclopropylpyrimidin-2-amine (compound 1A12, Table 1, Fig. 1)

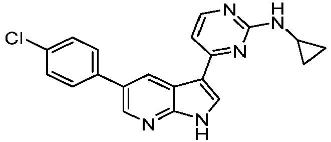

**¹H NMR (400 MHz, DMSO) δ** 12.32 (s, 1H), 9.28 (s, 1H), 9.13 (d, *J* = 1.9 Hz, 1H), 8.60 (d, *J* = 1.9 Hz, 1H), 8.40 (d, *J* = 2.4 Hz, 1H), 8.16 (d, *J* = 5.3 Hz, 1H), 7.84 (d, *J* = 8.4 Hz, 2H), 7.55 (d, *J* = 8.3 Hz, 2H), 7.09 (d, *J* = 15.8 Hz, 1H), 1.23- 1.18 (m, 1H), 0.69 - 0.59 (m, 2H), 0.58 - 0.49 (m, 2H); MS (ESI+): m\z 361.10.

### 4-(3-(2-(cyclopropylamino)pyrimidin-4-yl)-1H-pyrrolo[2,3-b]pyridin-5-yl)benzaldehyde (compound 1A13, Table 1, Fig. 1)

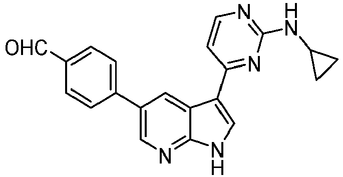

**¹H NMR (400 MHz, DMSO) δ** 12.32 (s, 1H), 9.89 (s, 1H), 9.28 (s, 1H), 9.13 (d, *J* = 1.9 Hz, 1H), 8.60 (d, *J* = 1.9 Hz, 1H), 8.40 (d, *J* = 2.4Hz, 1H), 8.16 (d, *J* = 5.3 Hz, 1H), 8.11 (d, *J* = 8.4 Hz, 2H), 7.63 (d, *J* = 8.3 Hz, 2H), 7.09 (d, *J* = 15.8 Hz, 1H), 1.23- 1.18 (m, 1H), 0.69 - 0.59 (m, 2H), 0.58-0.49 (m, 2H); MS (ESI+): m\z 355.14.

### N-cyclopropyl-4-(5-(naphthalen-1-yl)-1H-pyrrolo[2,3-b]pyridin-3-yl)pyrimidin-2-amine (compound 1A14, Table 1, Fig. 1)

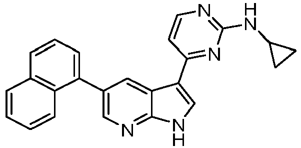

**¹H NMR (400 MHz, DMSO) δ** 12.20 (s, 1H), 9.00 (s, 1H), 8.30 (d, *J* = 2.7 Hz, 1H), 8.19 (s, 1H), 7.98 (d, *J* = 5.2 Hz, 1H), 7.81 (dd, *J* = 14.6, 8.1 Hz, 2H), 7.69 (s, 1H), 7.47 - 7.39 (m, 1H), 7.32 (dt, *J* = 26.2, 8.3 Hz, 3H), 6.96 (dd, *J* = 9.9, 4.1 Hz, 2H), 1.02 (s, 1H), 0.1 - 0.01 (m, 4H); MS (ESI+): m\z 377.16.

### N-cyclopropyl-4-(5-(pyridin-3-yl)-1H-pyrrolo[2,3-b]pyridin-3-yl)pyrimidin-2-amine (compound 1A15, Table 1, Fig. 1)

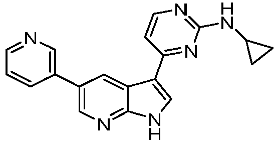

**¹H NMR (400 MHz, DMSO) δ** 12.39 (s, 1H), 9.29 (s, 1H), 8.96 (s, 1H), 8.63 (d, *J* = 2.2 Hz, 1H), 8.60 (d, *J* = 4.6Hz, 1H), 8.48 (s, 1H), 8.22 (d, *J* = 5.1 Hz, 1H), 8.14 (d, *J* = 7.5 Hz, 1H), 7.53 (dd, *J* = 7.7, 4.8 Hz, 1H), 7.35 (s, 1H), 7.16 (d, *J* = 5.3 Hz, 1H), 1.26- 1.18 (m, 1H), 0.69 (q, *J* = 6.6 Hz, 2H), 0.56 (d, *J* = 2.4 Hz, 2H); MS (ESI+): m\z 328.14.

### N-cyclopropyl-4-(5-(furan-3-yl)-1H-pyrrolo[2,3-b]pyridin-3-yl)pyrimidin-2-amine (compound 1A16, Table 1, Fig. 1)

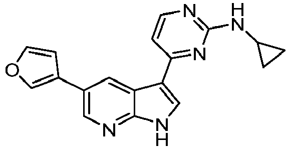

**¹H NMR (400 MHz, DMSO) δ** 12.25 (s, 1H), 9.09 (s, 1H), 8.59 (d, *J* = 2.1 Hz, 1H), 8.39 (d, *J* = 2.7 Hz, 1H), 8.21 (d, *J* = 5.3 Hz, 2H), 7.80 (t, *J* = 1.7 Hz, 1H), 7.30 (s, 1H), 7.14 (d, *J* = 5.3 Hz, 1H), 1.28 - 1.18 (m, 1H), 0.78 - 0.71 (m, 2H), 0.60 - 0.52 (m, 2H); MS (ESI+): m\z 317.12.

### N-cyclopropyl-4-(5-(5-methoxy-1H-indol-2-yl)-1H-pyrrolo[2,3-b]pyridin-3-yl)pyrimidin-2-amine (compound 1A17, Table 1, Fig. 1)

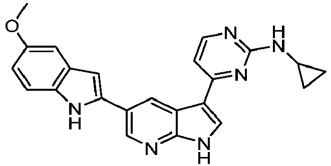

**¹H NMR (400 MHz, DMSO) δ** 12.40 (s, 1H), 11.42 (s, 1H), 9.32 (d, *J* = 1.9 Hz, 1H), 9.20 (s, 1H), 8.89 (d, *J* = 2.1 Hz, 1H), 8.46 (d, *J* = 2.7 Hz, 1H), 8.25 (d, *J* = 5.3 Hz, 1H), 7.40 (d, *J* = 8.7 Hz, 1H), 7.19 (d, *J* = 5.3 Hz, 1H), 7.09 (dd, *J* = 14.0, 1.7 Hz, 2H), 6.82 (dd, *J* = 8.7, 2.4 Hz, 1H), 3.80 (s, 3H), 1.23- 1.18 (m, 1H), 0.69 - 0.59 (m, 2H), 0.58 - 0.49 (m, 2H); MS (ESI+): m\z 396.17.

### 4-(5-(benzo[b]thiophen-2-yl)-1H-pyrrolo[2,3-b]pyridin-3-yl)-N-cyclopropylpyrimidin-2-amine (compound 1A18, Table 1, Fig. 1)

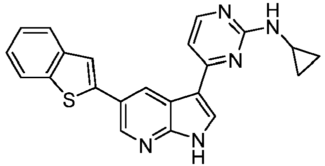

**¹H NMR (400 MHz, DMSO) δ** 12.44 (s, 1H), 9.27 (s, 1H), 9.15 (s, 1H), 8.71 (s, 1H), 8.46 (d, *J* = 1.9 Hz, 1H), 8.18 (d, *J* = 5.2 Hz, 1H), 8.08 - 7.96 (m, 2H), 7.88 (d, *J* = 7.6 Hz, 1H), 7.45- 7.36 (m, 2H), 7.12 (d, *J* = 5.3 Hz, 1H), 1.23- 1.18 (m, 1H), 0.69 - 0.59 (m, 2H), 0.58 - 0.49 (m, 2H); MS (ESI+): m\z 383.12.

### 4-(5-(1H-indol-2-yl)-1H-pyrrolo[2,3-b]pyridin-3-yl)-N-cyclopropylpyrimidin-2-amine (compound 1A19, Table 1, Fig. 1)

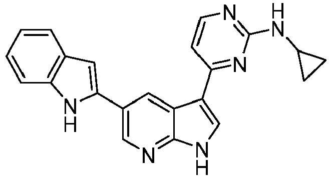

**¹H NMR (400 MHz, DMSO) δ** 12.50 (s, 1H), 11.56 (s, 1H), 9.30 (d, *J* = 2.1 Hz, 1H), 9.23 (s, 1H), 8.89 (d, *J* = 2.1 Hz, 1H), 8.46 (d, *J* = 2.7 Hz, 1H), 8.25 (d, *J* = 5.3 Hz, 1H), 7.40 (d, *J* = 8.7 Hz, 1H), 7.19 (d, *J* = 5.3 Hz, 1H), 7.12 - 7.01 (m, 3H), 6.82 (dd, *J* = 8.7, 2.4 Hz, 1H), 1.23- 1.18 (m, 1H), 0.69 - 0.59 (m, 2H), 0.58 - 0.49 (m, 2H); MS (ESI+): m\z 366.16.

### N-cyclopropyl-4-(5-(isoquinolin-1-yl)-1H-pyrrolo[2,3-b]pyridin-3-yl)pyrimidin-2-amine (compound 1A20, Table 1, Fig. 1)

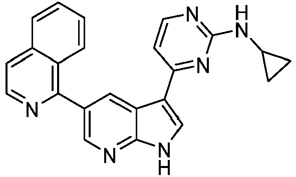

**¹H NMR (400 MHz, CDCl₃) δ** 12.40 (s, 1H), 9.34 (s, 1H), 9.24 (d, *J* = 2.2 Hz, 1H), 8.68 (d, *J* = 1.9 Hz, 1H), 8.37 (d, *J* = 2.0 Hz, 1H), 8.28 (d, *J* = 2.0 Hz, 1H), 8.17 (d, *J* = 8.4 Hz, 1H), 8.07 (d, *J* = 4.0 Hz, 1H), 7.92 (d, *J* = 8.1 Hz, 1H), 7.81 - 7.69 (m, 1H), 7.61 (t, *J* = 7.5 Hz, 1H), 7.45 (d, *J* = 3.3 Hz, 1H), 7.17 (d, *J* = 5.3 Hz, 1H), 1.23- 1.18 (m, 1H), 0.69 - 0.59 (m, 2H), 0.58 - 0.49 (m, 2H); MS (ESI+): m\z 378.16.

### N-cyclopentyl-4-(5-(4-(trifluoromethyl)phenyl)-1H-pyrrolo[2,3-b]pyridin-3-yl)pyrimidin-2-amine (compound 1A21, Table 2, Fig. 2)

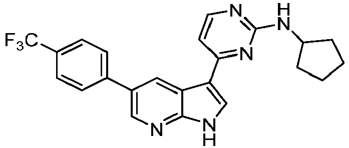

**¹H NMR (400 MHz, DMSO) δ** 12.39 (s, 1H), 9.33 (s, 1H), 8.60 (d, *J* = 2.2 Hz, 1H), 8.45 (d, *J* = 2.7 Hz, 1H), 8.23 (d, *J* = 5.2 Hz, 1H), 7.95 (d, *J* = 7.7 Hz, 2H), 7.88 (d, *J* = 8.3 Hz, 2H), 7.36 (d, *J* = 1.8 Hz, 1H), 7.16 (d, *J* = 5.3 Hz, 1H), 1.80 (s, 1H), 1.34 - 1.15 (m, 8H); MS (ESI+): m\z 423.16.

### N-cyclopentyl-4-(5-(4-fluorophenyl)-1H-pyrrolo[2,3-b]pyridin-3-yl)pyrimidin-2-amine (compound 1A22, Table 2, Fig. 2)

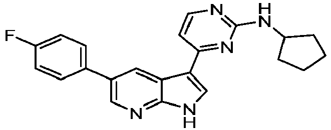

**¹H NMR (500 MHz, DMSO) δ** 12.37 (s, 1H), 9.27 (s, 1H), 8.55 (d, *J* = 2.2 Hz, 1H), 8.48 (s, 1H), 8.24 (s, 1H), 7.76 (s, 2H), 7.33 (d, *J* = 8.5 Hz, 3H), 7.15 (d, *J* = 5.1 Hz, 1H), 1.78 (s, 1H), 1.34 - 1.15 (m, 8H); MS (ESI+): m\z 373.17.

### N-cyclopentyl-4-(5-(4-(trifluoromethoxy)phenyl)-1H-pyrrolo[2,3-b]pyridin-3-yl)pyrimidin-2-amine (compound 1A23, Table 2, Fig. 2)

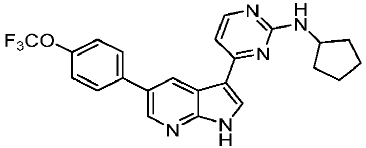

**¹H NMR (400 MHz, DMSO) δ** 12.38 (s, 1H), 9.25 (s, 1H), 8.59 (d, *J* = 2.2 Hz, 1H), 8.48 (d, *J* = 2.6 Hz, 1H), 8.23 (d, *J* = 5.2 Hz, 1H), 7.85 (d, *J* = 8.4 Hz, 2H), 7.49 (d, *J* = 8.0 Hz, 2H), 7.32 (s, 1H), 7.15 (d, *J* = 5.3 Hz, 1H), 1.80 (s, 1H), 1.34 - 1.15 (m, 8H); MS (ESI+): m\z 439.16.

### N-cyclopentyl-4-(5-(3-(trifluoromethyl)phenyl)-1H-pyrrolo[2,3-b]pyridin-3-yl)pyrimidin-2-amine (compound 1A24, Table 2, Fig. 2)

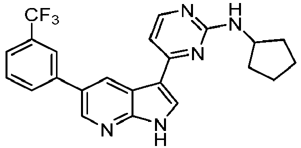

**¹H NMR (400 MHz, DMSO) δ** 12.42 (s, 1H), 9.35 (s, 1H), 8.67 (d, *J* = 1.7 Hz, 1H), 8.49 (d, *J* = 2.3 Hz, 1H), 8.21 (d, *J* = 5.1 Hz, 1H), 8.07 (d, *J* = 4.0 Hz, 1H), 7.96 (s, 1H), 7.75 (d, *J* = 4.7 Hz, 2H), 7.34 (s, 1H), 7.17 (d, *J* = 5.3 Hz, 1H), 1.80 (s, 1H), 1.34 - 1.15 (m, 8H); MS (ESI+): m\z 423.16.

### N-cyclopentyl-4-(5-(3-fluorophenyl)-1H-pyrrolo[2,3-b]pyridin-3-yl)pyrimidin-2-amine (compound 1A25, Table 2, Fig. 2)

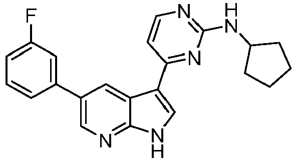

**¹H NMR (500 MHz, DMSO) δ** 12.40 (s, 1H), 9.28 (s, 1H), 8.53 (d, *J* = 2.2 Hz, 1H), 8.40 (s, 1H), 8.19 (s, 1H), 7.79 (s, 2H), 7.34 (d,*J* = 8.5 Hz, 3H), 7.18 (d, *J*= 5.1 Hz, 1H), 1.80 (s, 1H), 1.34- 1.15 (m, 8H); MS (ESI+): m\z 373.17

### N-cyclopentyl-4-(5-(p-tolyl)-1H-pyrrolo[2,3-b]pyridin-3-yl)pyrimidin-2-amine (compound 1A26, Table 2, Fig. 2)

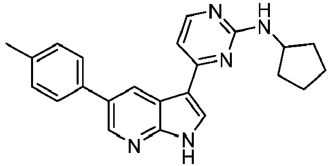

**¹H NMR (500 MHz, DMSO) δ** 12.39 (s, 1H), 9.29 (s, 1H), 8.52 (d, *J* = 2.2 Hz, 1H), 8.49 (s, 1H), 8.23 (s, 1H), 7.85 (s, 2H), 7.34 (d, *J* = 8.5 Hz, 3H), 7.13 (d, *J* = 5.1 Hz, 1H), 2.30 (s, 3H), 1.80 (s, 1H), 1.34 - 1.15 (m, 8H); MS (ESI+): m\z 369.19

### N-cyclopentyl-4-(5-(m-tolyl)-1H-pyrrolo[2,3-b]pyridin-3-yl)pyrimidin-2-amine (compound 1A27, Table 2, Fig. 2)

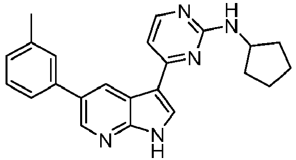

**¹H NMR (500 MHz, DMSO) δ** 12.38 (s, 1H), 9.26 (s, 1H), 8.53 (d, *J* = 2.2 Hz, 1H), 8.42 (s, 1H), 8.19 (s, 1H), 7.79 (s, 2H), 7.34 (d, *J* = 8.5 Hz, 3H), 7.18 (d, *J* = 5.1 Hz, 1H), 2.31 (s, 3H), 1.80 (s, 1H), 1.34 - 1.15 (m, 8H); MS (ESI+): m\z 369.19

### N-cyclopentyl-4-(5-(4-(methylthio)phenyl)-1H-pyrrolo[2,3-b]pyridin-3-yl)pyrimidin-2-amine (compound 1A28, Table 2, Fig. 2)

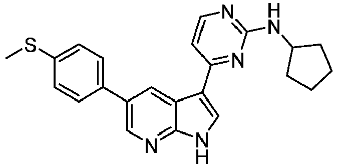

**¹H NMR (400 MHz, DMSO) δ** 12.39 (s, 1H), 9.05 (s, 1H), 8.45 (d, *J* = 2.2 Hz, 1H), 8.26 (s, 1H), 8.19 (d, *J* = 5.0 Hz, 1H), 7.70 (d, *J* = 8.4 Hz, 2H), 7.32 (d, *J* = 8.4 Hz, 3H), 7.03 (d, *J* = 5.3 Hz, 1H), 2.39 (s, 3H), 1.80 (s, 1H), 1.34 - 1.15 (m, 8H); MS (ESI+): m\z 401.16

### N-cyclopentyl-4-(5-(2-(methylthio)phenyl)-1H-pyrrolo[2,3-b]pyridin-3-yl)pyrimidin-2-amine (compound 1A29, Table 2, Fig. 2)

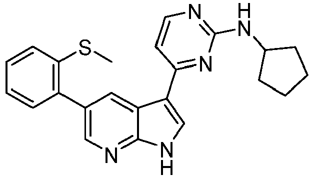

**¹H NMR (400 MHz, DMSO) δ** 12.33 (s, 1H), 9.0 (s, 1H), 8.44 (d, *J* = 2.2 Hz, 1H), 8.23 (s, 1H), 8.19 (d, *J* = 5.0 Hz, 1H), 7.46 - 7.39 (m, 1H), 7.36 (d, *J* = 7.8 Hz, 1H), 7.27 (dd, *J* = 15.7, 7.5 Hz, 3H), 7.13 (d, *J* = 5.3 Hz, 1H), 2.36 (s, 3H), 1.80 (s, 1H), 1.34 - 1.15 (m, 8H); MS (ESI+): m\z 401.16

### N-cyclopentyl-4-(5-(2-ethylphenyl)-1H-pyrrolo[2,3-b]pyridin-3-yl)pyrimidin-2-amine (compound 1A30, Table 2, Fig. 2)

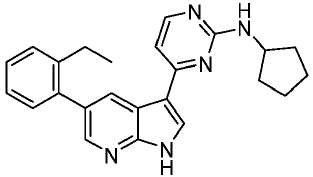

**¹H NMR (400 MHz, DMSO) δ** 12.31 (s, 1H), 8.98 (s, 1H), 8.45 (d, *J* = 2.5 Hz, 1H), 8.20 (dd, *J* = 7.6, 3.4 Hz, 2H), 7.36 (t*, J=* 6.2 Hz, 2H), 7.31 - 7.19 (m, 3H), 7.13 (d, *J* = 5.3 Hz, 1H), 2.60 (dd, *J =* 15.0, 7.5 Hz, 2H), 1.80 (s, 1H), 1.34 - 1.15 (m, 8H), 1.01 (t, *J* = 7.5 Hz, 3H); MS (ESI+): m\z 383.21

### N-cyclopentyl-4-(5-(4-methoxyphenyl)-1H-pyrrolo-[2,3-b]pyridin-3-yl)pyrimidin-2-amine (compound 1A31, Table 2, Fig. 2)

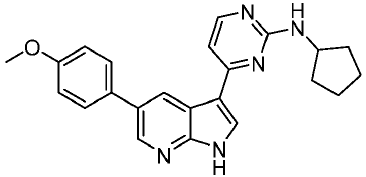

**¹H NMR (400 MHz, DMSO) δ** 12.23 (s, 1H), 9.16 (s, 1H), 8.51 (d, *J* = 2.2 Hz, 1H), 8.39 (s, 1H), 8.18 (d, *J* = 5.2 Hz, 1H), 7.63 (d, *J* = 8.3 Hz, 2H), 7.26 (d, *J* = 2.0 Hz, 1H), 7.12 (d, *J* = 5.3 Hz, 1H), 7.04 (d, *J* = 8.7 Hz, 2H), 3.83 (s, 3H), 1.80 (s, 1H), 1.34 - 1.15 (m, 8H); MS (ESI+): m\z 385.19

### 4-(5-(4-chlorophenyl)-1H-pyrrolo [2,3-b]pyridin-3-yl)-N-cyclopentylpyrimidin-2-amine (compound 1A32, Table 2, Fig. 2)

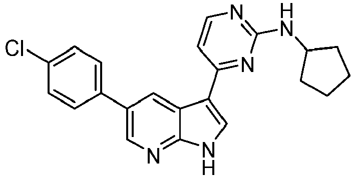

**¹H NMR (400 MHz, DMSO) δ** 12.32 (s, 1H), 9.28 (s, 1H), 9.13 (d*, J* = 1.9 Hz, 1H), 8.60 (d, *J* = 1.9 Hz, 1H), 8.40 (d, *J* = 2.4 Hz, 1H), 8.16 (d, *J* = 5.3 Hz, 1H), 7.84 (d, *J* = 8.4 Hz, 2H), 7.55 (d, *J* = 8.3 Hz, 2H), 7.09 (d, *J=* 15.8 Hz, 1H), 1.80 (s, 1H), 1.34 - 1.15 (m, 8H); MS (ESI+): m\z 389.14

### 4-(3-(2-(cyclopentylamino)pyrimidin-4-yl)-1H-pyrrolo[2,3-b]pyridin-5-yl)benzaldehyde (compound 1A33, Table 2, Fig. 2)

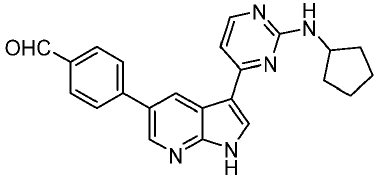

**¹H NMR (400 MHz, DMSO) δ** 12.32 (s, 1H), 9.89 (s, 1H), 9.28 (s, 1H), 9.13 (d, *J* = 1.9 Hz, 1H), 8.60 (d, *J* = 1.9 Hz, 1H), 8.40 (d, *J* = 2.4 Hz, 1H), 8.16 (d, *J* = 5.3 Hz, 1H), 8.11 (d, *J* = 8.4 Hz, 2H), 7.63 (d, *J* = 8.3 Hz, 2H), 7.09 (d, *J* = 15.8 Hz, 1H), 1.80 (s, 1H), 1.34 - 1.15 (m, 8H); MS (ESI+): m\z 383.17

### N-cyclopentyl-4-(5-(naphthalen-1-yl)-1H-pyrrolo[2,3-b]pyridin-3-yl)pyrimidin-2-amine (compound 1A34, Table 2, Fig. 2)

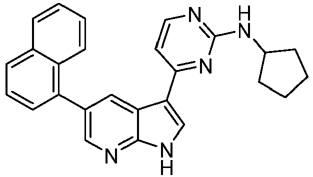

**¹H NMR (400 MHz, DMSO) δ** 12.20 (s, 1H), 9.00 (s, 1H), 8.30 (d, *J* = 2.7 Hz, 1H), 8.19 (s, 1H), 7.98 (d, *J* = 5.2 Hz, 1H), 7.81 (dd, *J* = 14.6, 8.1 Hz, 2H), 7.69 (s, 1H), 7.47 - 7.39 (m, 1H), 7.32 (dt, *J* = 26.2, 8.3 Hz, 3H), 6.96 (dd, *J* = 9.9, 4.1 Hz, 2H), 1.69 (s, 1H), 1.25 - 1.08 (m, 8H); MS (ESI+): m\z 405.19

### N-cyclopentyl-4-(5-(pyridin-3-yl)-1H-pyrrolo[2,3-b]pyridin-3-yl)pyrimidin-2-amine (compound 1A35, Table 2, Fig. 2)

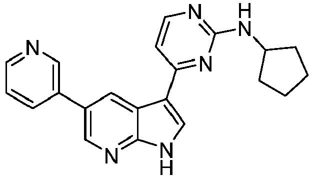

**¹H NMR (400 MHz, DMSO) δ** 12.39 (s, 1H), 9.29 (s, 1H), 8.96 (s, 1H), 8.63 (d, *J* = 2.2 Hz, 1H), 8.60(d, *J* = 4.6 Hz, 1H), 8.48 (s, 1H), 8.22 (d, *J* = 5.1 Hz, 1H), 8.14 (d, *J* = 7.5 Hz, 1H), 7.53 (dd, *J =* 7.7, 4.8 Hz, 1H), 7.35 (s, 1H), 7.16 (d, *J* = 5.3 Hz, 1H), 1.80 (s, 1H), 1.34 - 1.15 (m, 8H); MS (ESI+): m\z 356.17

### N-cyclopentyl-4-(5-(furan-3-yl)-1H-pyrrolo[2,3-b]pyridin-3-yl)pyrimidin-2-amine (compound 1A36, Table 2, Fig. 2)

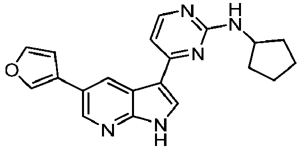

**¹H NMR (400 MHz, DMSO) δ** 12.25 (s, 1H), 9.09 (s, 1H), 8.59 (d, *J* = 2.1 Hz, 1H), 8.39 (d, *J* = 2.7 Hz, 1H), 8.21 (d, *J* = 5.3 Hz, 2H), 7.80 (t, *J* = 1.7 Hz, 1H), 7.30 (s, 1H), 7.14 (d, *J* = 5.3 Hz, 1H), 1.80 (s, 1H), 1.34 - 1.15 (m, 8H); MS (ESI+): m\z 345.15

### N-cyclopentyl-4-(5-(5-methoxy-1H-indol-2-yl)-1H-pyrrolo[2,3-b]pyridin-3-yl)pyrimidin-2-amine (compound 1A37, Table 2, Fig. 2)

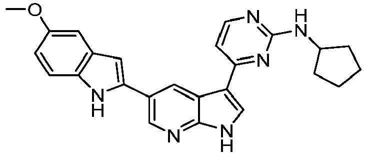

**¹H NMR (400 MHz, DMSO) δ** 12.40 (s, 1H), 11.42 (s, 1H), 9.32 (d, *J* = 1.9 Hz, 1H), 9.20 (s, 1H), 8.89 (d, *J* = 2.1 Hz, 1H), 8.46 (d, *J* = 2.7 Hz, 1H), 8.25 (d, *J* = 5.3 Hz, 1H), 7.40 (d, *J* = 8.7 Hz, 1H), 7.19 (d, *J* = 5.3 Hz, 1H), 7.09 (dd, *J* = 14.0, 1.7 Hz, 2H), 6.82 (dd, *J* = 8.7, 2.4 Hz, 1H), 3.80 (s, 3H), 1.80 (s, 1H), 1.34 - 1.15 (m, 8H); MS (ESI+): m\z 424.20

### 4-(5-(benzo[b]thiophen-2-yl)-1H-pyrrolo[2,3-b]pyridin-3-yl)-N-cyclopentylpyrimidin-2-amine (compound 1A38, Table 2, Fig. 2)

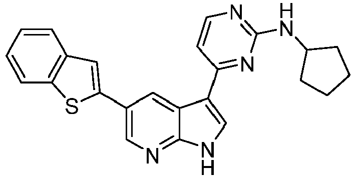

**¹H NMR (400 MHz, DMSO) δ** 12.44 (s, 1H), 9.27 (s, 1H), 9.15 (s, 1H), 8.71 (s, 1H), 8.46 (d, *J* = 1.9 Hz, 1H), 8.18 (d, *J* = 5.2 Hz, 1H), 8.08 - 7.96 (m, 2H), 7.88 (d, *J* = 7.6 Hz, 1H), 7.45- 7.36 (m, 2H), 7.12 (d, *J* = 5.3 Hz, 1H), 1.80 (s, 1H), 1.34 - 1.15 (m, 8H); MS (ESI+): m\z 411.15

### 4-(5-(1H-indol-2-yl)-1H-pyrrolo[2,3-b]pyridin-3-yl)-N-cyclopentylpyrimidin-2-amine (compound 1A39, Table 2, Fig. 2)

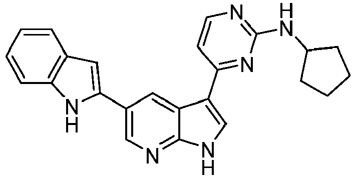

**¹H NMR (400 MHz, DMSO) δ** 12.50 (s, 1H), 11.56 (s, 1H), 9.30 (d, *J* = 2.1 Hz, 1H), 9.23 (s, 1H), 8.89 (d, *J* = 2.1 Hz, 1H), 8.46 (d, *J* = 2.7 Hz, 1H), 8.25 (d, *J* = 5.3 Hz, 1H), 7.40 (d, *J* = 8.7 Hz, 1H), 7.19 (d, *J* = 5.3 Hz, 1H), 7.12 - 7.01 (m, 3H), 6.82 (dd, *J* = 8.7, 2.4 Hz, 1H), 1.33- 1.20 (m, 1H), 0.79 - 0.5 (m, 8H); MS (ESI+): m\z 394.19

### N-cyclopentyl-4-(5-(isoquinolin-1-yl)-1H-pyrrolo[2,3-b]pyridin-3-yl)pyrimidin-2-amine (compound 1A40, Table 2, Fig. 2)

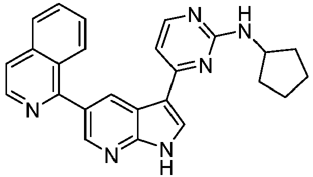

**¹H NMR (400 MHz, CDCl₃**) **δ** 12.35 (s, 1H), 9.32 (s, 1H), 9.24 (d, *J* = 2.2 Hz, 1H), 8.68 (d, *J* = 1.9 Hz, 1H), 8.37 (d, *J* = 2.0 Hz, 1H), 8.28 (d, *J* = 2.0 Hz, 1H), 8.17 (d*, J =* 8.4 Hz, 1H), 8.07 (d, *J* = 4.0 Hz, 1H), 7.92 (d, *J* = 8.1 Hz, 1H), 7.81 - 7.69 (m, 1H), 7.61 (t, *J* = 7.5 Hz, 1H), 7.45 (d, *J* = 3.3 Hz, 1H), 7.17 (d, *J* = 5.3 Hz, 1H), 1.33- 1.20 (m, 1H), 0.79 - 0.5 (m, 8H); MS (ESI+): m\z 406.19

### N-cycloheptyl-4-(5-(4-(trifluoromethyl)phenyl)-1H-pyrrolo[2,3-b]pyridin-3-yl)pyrimidin-2-amine (compound 1A41, Table 3, Fig. 3)

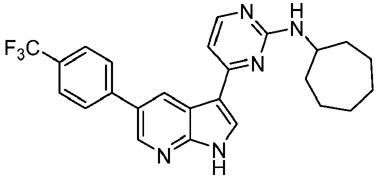

**¹H NMR (400 MHz, DMSO) δ** 12.44 (s, 1H), 9.37 (s, 1H), 8.63 (d, *J* = 2.1 Hz, 1H), 8.48 (d, *J* = 2.3 Hz, 1H), 8.23 (d, *J* = 5.2 Hz, 1H), 7.95 (d, *J* = 7.7 Hz, 2H), 7.88 (d, *J* = 8.3 Hz, 2H), 7.36 (d, *J* = 1.8 Hz, 1H), 7.16 (d, *J* = 5.3 Hz, 1H), 2.08 - 1.91 (m, 1H), 1.72 - 1.30 (m, 12H); MS (ESI+): m\z 451.19

### N-cycloheptyl-4-(5-(4-fluorophenyl)-1H-pyrrolo[2,3-b]pyridin-3-yl)pyrimidin-2-amine (compound 1A42, Table 3, Fig. 3)

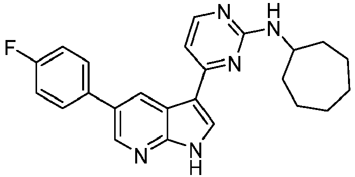

**¹H NMR (500 MHz, DMSO) δ** 12.40 (s, 1H), 9.30 (s, 1H), 8.57 (d, *J=* 2.1 Hz, 1H), 8.48 (s, 1H), 8.28 (s, 1H), 7.79 (s, 2H), 7.35 (d, *J* = 8.2 Hz, 3H), 7.15 (d, *J* = 5.1 Hz, 1H), 2.08 - 1.91 (m, 1H), 1.72 - 1.30 (m, 12H); MS (ESI+): m\z 401.20

### N-cycloheptyl-4-(5-(4-(trifluoromethoxy)phenyl)-1H-pyrrolo[2,3-b]pyridin-3-yl)pyrimidin-2-amine (compound 1A43, Table 3, Fig. 3)

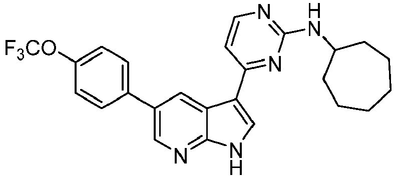

**¹H** NMR (400 MHz, DMSO) **δ** 12.41 (s, 1H), 9.26 (s, 1H), 8.58 (d, *J* = 2.3 Hz, 1H), 8.48 (d, *J* = 2.6 Hz, 1H), 8.23 (d, *J* = 5.2 Hz, 1H), 7.85 (d, *J* = 8.4 Hz, 2H), 7.49 (d, *J* **=** 8.0 Hz, 2H), 7.34 (s, 1H), 7.15 (d, *J=* 5.3 Hz, 1H), 2.08 - 1.91 (m, 1H), 1.72 - 1.30 (m, 12H); MS (ESI+): m\z 467.19

### N-cycloheptyl-4-(5-(3-(trifluoromethyl)phenyl)-1H-pyrrolo[2,3-b]pyridin-3-yl)pyrimidin-2-amine (compound 1A44, Table 3, Fig. 3)

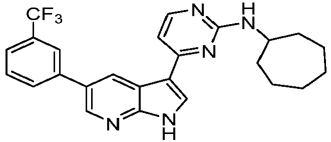

**¹H NMR (400 MHz, DMSO) δ** 12.44 (s, 1H), 9.37 (s, 1H), 8.69 (d, *J* = 1.6 Hz, 1H), 8.49 (d, *J* = 2.3 Hz, 1H), 8.21 (d, *J* = 5.1 Hz, 1H), 8.07 (d, *J* = 4.0 Hz, 1H), 7.95 (s, 1H), 7.75 (d, *J* = 4.7 Hz, 2H), 7.36 (s, 1H), 7.17 (d, *J* = 5.3 Hz, 1H), 2.08 - 1.91 (m, 1H), 1.72 - 1.30 (m, 12H); MS (ESI+): m\z 451.19

### N-cycloheptyl-4-(5-(3-fluorophenyl)-1H-pyrrolo[2,3-b]pyridin-3-yl)pyrimidin-2-amine (compound 1A45, Table 3, Fig. 3)

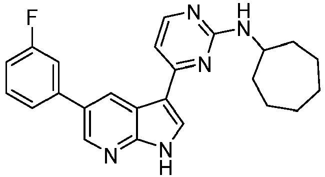

**¹H NMR (500 MHz, DMSO) δ** 12.42 (s, 1H), 9.30 (s, 1H), 8.53 (d, *J=* 2.2 Hz, 1H), 8.41 (s, 1H), 8.20 (s, 1H), 7.80 (s, 2H), 7.34 (d, *J* = 8.5 Hz, 3H), 7.18 (d, *J* = 5.1 Hz, 1H), 2.08 - 1.91 (m, 1H), 1.72 - 1.30 (m, 12H); MS (ESI+): m\z 401.20

### N-cycloheptyl-4-(5-(p-tolyl)-1H-pyrrolo[2,3-b]pyridin-3-yl)pyrimidin-2-amine (compound 1A46, Table 3, Fig. 3)

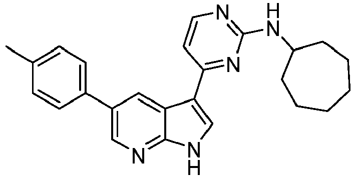

**¹H NMR (500 MHz, DMSO) δ** 12.40 (s, 1H), 9.29 (s, 1H), 8.52 (d, *J* = 2.2 Hz, 1H), 8.49 (s, 1H), 8.23 (s, 1H), 7.85 (s, 2H), 7.34 (d, *J* = 8.5 Hz, 3H), 7.13 (d, *J* = 5.1 Hz, 1H), 2.30 (s, 3H), 2.08 - 1.91 (m, 1H), 1.72 - 1.30 (m, 12H); MS (ESI+): m\z 397.22

### N-cycloheptyl-4-(5-(m-tolyl)-1H-pyrrolo[2,3-b]pyridin-3-yl)pyrimidin-2-amine (compound 1A47, Table 3, Fig. 3)

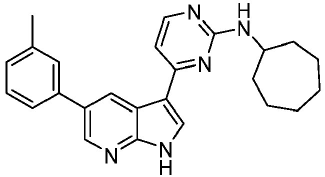

**¹H NMR (500 MHz, DMSO) δ** 12.39 (s, 1H), 9.26 (s, 1H), 8.53 (d, *J=* 2.2 Hz, 1H), 8.42 (s, 1H), 8.20 (s, 1H), 7.79 (s, 2H), 7.34 (d*, J =* 8.5 Hz, 3H), 7.18 (d*, J =* 5.1 Hz, 1H), 2.32 (s, 3H), 2.08 - 1.91 (m, 1H), 1.72 - 1.30 (m, 12H); MS (ESI+): m\z 397.22

### N-cycloheptyl-4-(5-(4-(methylthio)phenyl)-1H-pyrrolo[2,3-b]pyridin-3-yl)pyrimidin-2-amine (compound 1A48, Table 3, Fig. 3)

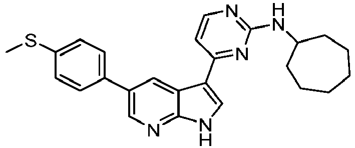

**¹H NMR (400 MHz, DMSO) δ** 12.39 (s, 1H), 9.05 (s, 1H), 8.45 (d, *J* = 2.2 Hz, 1H), 8.26 (s, 1H), 8.19 (d, *J* = 5.0 Hz, 1H), 7.70 (d, *J* = 8.4 Hz, 2H), 7.32 (d, *J* = 8.4 Hz, 3H), 7.03 (d, *J* = 5.3 Hz, 1H), 2.39 (s, 3H), 2.08 - 1.91 (m, 1H), 1.72 - 1.30 (m, 12H); MS (ESI+): m\z 429.19

### N-cycloheptyl-4-(5-(2-(methylthio)phenyl)-1H-pyrrolo[2,3-b]pyridin-3-yl)pyrimidin-2-amine (compound 1A49, Table 3, Fig. 3)

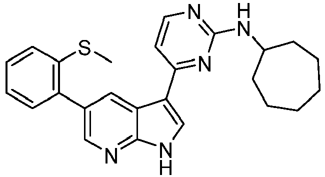

**¹H NMR (400 MHz, DMSO) δ** 12.33 (s, 1H), 9.0 (s, 1H), 8.44 (d, *J* = 2.2 Hz, 1H), 8.23 (s, 1H), 8.19 (d, *J* = 5.0 Hz, 1H), 7.46 - 7.39 (m, 1H), 7.36 (d, *J* = 7.8 Hz, 1H), 7.27 (dd, *J* = 15.7, 7.5 Hz, 3H), 7.13 (d, *J* = 5.3 Hz, 1H), 2.36 (s, 3H), 2.08 - 1.91 (m, 1H), 1.72 - 1.30 (m, 12H); MS (ESI+): m\z 429.19

### N-cycloheptyl-4-(5-(2-ethylphenyl)-1H-pyrrolo[2,3-b]pyridin-3-yl)pyrimidin-2-amine (compound 1A50, Table 3, Fig. 3)

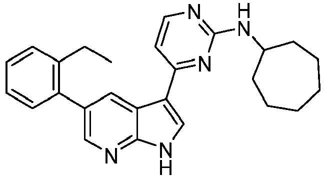

**¹H NMR (400 MHz, DMSO) δ** 12.31 (s, 1H), 8.98 (s, 1H), 8.45 (d, *J* = 2.5 Hz, 1H), 8.20 (dd, *J* = 7.6, 3.4 Hz, 2H), 7.36 (t, *J* = 6.2 Hz, 2H), 7.31 - 7.19 (m, 3H), 7.13 (d, *J* = 5.3 Hz, 1H), 2.60 (dd, *J =* 15.0, 7.5 Hz, 2H), 2.08 - 1.91 (m, 1H), 1.72 - 1.30 (m, 12H), 1.01 (t, *J* = 7.5 Hz, 3H); MS (ESI+): m\z 411.24

### N-cycloheptyl-4-(5-(4-methoxyphenyl)-1H-pyrrolo[2,3-b]pyridin-3-yl)pyrimidin-2-amine (compound 1A51, Table 3, Fig. 3)

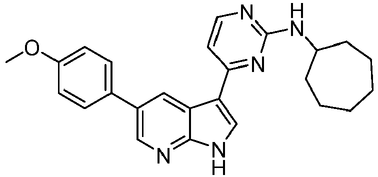

**¹H NMR (400 MHz, DMSO) δ** 12.93 (s, 1H), 8.89 (d, *J* = 23.2 Hz, 2H), 8.61 (s, 1H), 8.39 (s, 1H), 8.20 (d, *J* = 6.0 Hz, 1H), 7.67 (d, *J* = 8.1 Hz, 2H), 7.39 (d, *J* = 5.7 Hz, 1H), 7.07 (d, *J* = 8.0 Hz, 2H), 3.82 (s, 3H), 2.08 - 1.91 (m, 1H), 1.72 - 1.30 (m, 12H); MS (ESI+): m\z 413.22

### 4-(5-(4-chlorophenyl)-1H-pyrrolo[2,3-b]pyridin-3-yl)-N-cycloheptylpyrimidin-2-amine (compound 1A52, Table 3, Fig. 3)

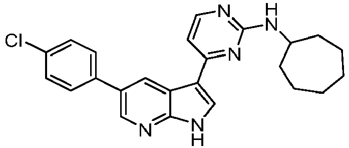

**¹H NMR (400 MHz, DMSO) δ** 12.32 (s, 1H), 9.28 (s, 1H), 9.13 (d, *J* = 1.9 Hz, 1H), 8.60 (d, *J* = 1.9 Hz, 1H), 8.40 (d, *J* = 2.4 Hz, 1H), 8.16 (d, *J* = 5.3 Hz, 1H), 7.84 (d,*J* = 8.4 Hz, 2H), 7.55 (d, *J* = 8.3 Hz, 2H), 7.09 (d, *J* = 15.8 Hz, 1H), 2.08 - 1.91 (m, 1H), 1.72 - 1.30 (m, 12H); MS (ESI+): m\z 417.17

### 4-(3-(2-(cycloheptylamino)pyrimidin-4-yl)-1H-pyrrolo[2,3-b]pyridin-5-yl)benzaldehyde (compound 1A53, Table 3, Fig. 3)

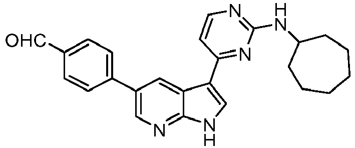

**¹H NMR (400 MHz, DMSO) δ** 12.32 (s, 1H), 9.89 (s, 1H), 9.28 (s, 1H), 9.13 (d, *J* = 1.9 Hz, 1H), 8.60 (d,*J* = 1.9 Hz, 1H), 8.40 (d, *J*=2.4Hz*,* 1H), 8.16 (d, *J* = 5.3 Hz, 1H), 8.11 (d, *J* = 8.4 Hz, 2H), 7.63 (d, *J* = 8.3 Hz, 2H), 7.09 (d, *J* = 15.8 Hz, 1H), 2.08 - 1.91 (m, 1H), 1.72 - 1.30 (m, 12H); MS (ESI+): m\z 411.20

### N-cycloheptyl-4-(5-(naphthalen-1-yl)-1H-pyrrolo[2,3-b]pyridin-3-yl)pyrimidin-2-amine (compound 1A54, Table 3, Fig. 3)

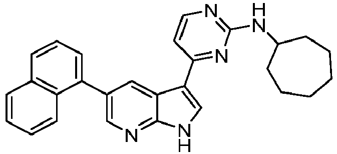

**¹H NMR (400 MHz, DMSO) δ** 12.20 (s, 1H), 9.00 (s, 1H), 8.30 (d, *J=* 2.7 Hz, 1H), 8.19 (s, 1H), 7.98 (d, *J* = 5.2 Hz, 1H), 7.81 (dd, *J* = 14.6, 8.1 Hz, 2H), 7.69 (s, 1H), 7.47 - 7.39 (m, 1H), 7.32 (dt, *J* = 26.2, 8.3 Hz, 3H), 6.96 (dd, *J* = 9.9, 4.1 Hz, 2H), 2.08 - 1.91 (m, 1H), 1.72 - 1.30 (m, 12H); MS (ESI+): m\z 433.22

### N-cycloheptyl-4-(5-(pyridin-3-yl)-1H-pyrrolo[2,3-b]pyridin-3-yl)pyrimidin-2-amine (compound 1A55, Table 3, Fig. 3)

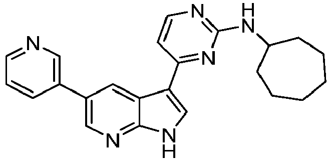

**¹H NMR (400 MHz, DMSO) δ** 12.39 (s, 1H), 9.29 (s, 1H), 8.96 (s, 1H), 8.63 (d, *J* = 2.2 Hz, 1H), 8.60(d, *J* = 4.6 Hz, 1H), 8.48 (s, 1H), 8.22 (d, *J* = 5.1 Hz, 1H), 8.14 (d, *J*= 7.5 Hz, 1H), 7.53 (dd, *J =* 7.7, 4.8 Hz, 1H), 7.35 (s, 1H), 7.16 (d, *J=* 5.3 Hz, 1H), 1.80 (s, 1H), 1.34 - 1.15 (m, 8H), 2.08 - 1.91 (m, 1H), 1.72 - 1.30 (m, 12H); MS (ESI+): m\z 384.20

### N-cycloheptyl-4-(5-(furan-3-yl)-1H-pyrrolo[2,3-b]pyridin-3-yl)pyrimidin-2-amine (compound 1A56, Table 3, Fig. 3)

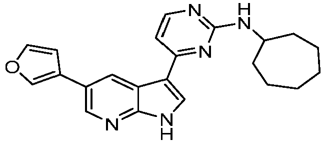

**¹H NMR (400 MHz, DMSO) δ** 12.25 (s, 1H), 9.09 (s, 1H), 8.59 (d, *J* = 2.1 Hz, 1H), 8.39 (d, *J* = 2.7 Hz, 1H), 8.21 (d, *J* = 5.3 Hz, 2H), 7.80 (t, *J* = 1.7 Hz, 1H), 7.30 (s, 1H), 7.14 (d, *J* = 5.3 Hz, 1H), 2.08 - 1.91 (m, 1H), 1.72 - 1.30 (m, 12H); MS (ESI+): m\z 373.19

### N-cycloheptyl-4-(5-(5-methoxy-1H-indol-2-yl)-1H-pyrrolo[2,3-b]pyridin-3-yl)pyrimidin-2-amine (compound 1A57, Table 3, Fig. 3)

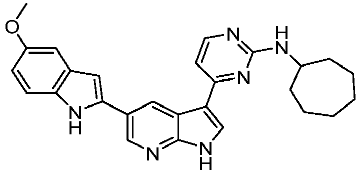

**¹H NMR (400 MHz, DMSO) δ** 12.40 (s, 1H), 11.42 (s, 1H), 9.32 (d, *J* = 1.9 Hz, 1H), 9.20 (s, 1H), 8.89 (d, *J* = 2.1 Hz, 1H), 8.46 (d, *J* = 2.7 Hz, 1H), 8.25 (d, *J* = 5.3 Hz, 1H), 7.40 (d, *J* = 8.7 Hz, 1H), 7.19 (d, *J* = 5.3 Hz, 1H), 7.09 (dd, *J* = 14.0, 1.7 Hz, 2H), 6.82 (dd, *J* = 8.7, 2.4 Hz, 1H), 3.80 (s, 3H), 2.08 - 1.91 (m, 1H), 1.72 - 1.30 (m, 12H); MS (ESI+): m\z 452.23

### 4-(5-(benzo[b]thiophen-2-yl)-1H-pyrrolo[2,3-b]pyridin-3-yl)-N-cycloheptylpyrimidin-2-amine (compound 1A58, Table 3, Fig. 3)

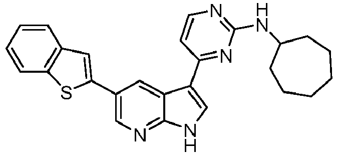

**¹H NMR (400 MHz, DMSO) δ** 12.44 (s, 1H), 9.27 (s, 1H), 9.15 (s, 1H), 8.71 (s, 1H), 8.46 (d, *J* = 1.9 Hz, 1H), 8.18 (d*, J* = 5.2 Hz, 1H), 8.08 - 7.96 (m, 2H), 7.88 (d, *J* = 7.6 Hz, 1H), 7.45- 7.36 (m, 2H), 7.12 (d, *J* = 5.3 Hz, 1H), 1.80 (s, 1H), 1.34 - 1.15 (m, 8H), 2.08 - 1.91 (m, 1H), 1.72 - 1.30 (m, 12H); MS (ESI+): m\z 439.18

### 4-(5-(1H-indol-2-yl)-1H-pyrrolo[2,3-b]pyridin-3-yl)-N-cycloheptylpyrimidin-2-amine (compound 1A59, Table 3, Fig. 3)

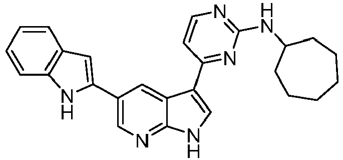

**¹H NMR (400 MHz, DMSO) δ** 12.50 (s, 1H), 11.56 (s, 1H), 9.30 (d, *J* = 2.1 Hz, 1H), 9.23 (s, 1H), 8.89 (d, *J* = 2.1 Hz, 1H), 8.46 (d, *J* = 2.7 Hz, 1H), 8.25 (d, *J* = 5.3 Hz, 1H), 7.40 (d, *J* = 8.7 Hz, 1H), 7.19 (d, *J* = 5.3 Hz, 1H), 7.12 - 7.01 (m, 3H), 6.82 (dd, *J*= 8.7, 2.4 Hz, 1H), 2.08 - 1.91 (m, 1H), 1.72 - 1.30 (m, 12H); MS (ESI+): m\z 422.22

### N-cycloheptyl-4-(5-(isoquinolin-1-yl)-1H-pyrrolo[2,3-b]pyridin-3-yl)pyrimidin-2-amine (compound 1A60, Table 3, Fig. 3)

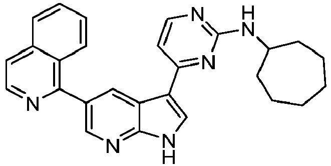

**¹H NMR (400 MHz, CDCl₃**) **δ** 12.35 (s, 1H), 9.32 (s, 1H), 9.24 (d, *J* = 2.2 Hz, 1H), 8.68 (d, *J* = 1.9 Hz, 1H), 8.37 (d, *J* = 2.0 Hz, 1H), 8.28 (d, *J* = 2.0 Hz, 1H), 8.17 (d*, J =* 8.4 Hz, 1H), 8.07 (d, *J* = 4.0 Hz, 1H), 7.92 (d, *J* = 8.1 Hz, 1H), 7.81 - 7.69 (m, 1H), 7.61 (t, *J* = 7.5 Hz, 1H), 7.45 (d, *J* = 3.3 Hz, 1H), 7.17 (d, *J* = 5.3 Hz, 1H), 2.08 - 1.91 (m, 1H), 1.72 - 1.30 (m, 12H); MS (ESI+): m\z 434.22

### N-phenethyl-4-(5-(4-(trifluoromethyl)phenyl)-1H-pyrrolo[2,3-b]pyridin-3-yl)pyrimidin-2-amine (compound 1B1, Table 4, Fig. 4)

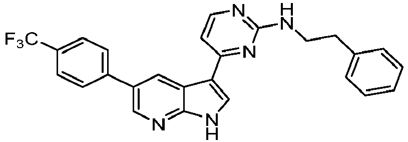

**¹H NMR (400 MHz, DMSO) δ** 12.39 (s, 1H), 9.33 (s, 1H), 8.60 (d, *J* = 2.2 Hz, 1H), 8.45 (d, *J* = 2.7 Hz, 1H), 8.23 (d, *J* = 5.2 Hz, 1H), 7.95 (d, *J* = 7.7 Hz, 2H), 7.88 (d, *J* = 8.3 Hz, 2H), 7.36 - 7.20 (m, 6H), 7.16 (d, *J* = 5.3 Hz, 1H), 3.83 (dd, *J* = 13.3, 6.8 Hz, 2H), 3.02 (t*, J =* 7.1 Hz, 2H); MS (ESI+): m\z 459.16

### 4-(5-(4-fluorophenyl)-1H-pyrrolo[2,3-b]pyridin-3-yl)-N-phenethylpyrimidin-2-amine (compound 1B2, Table 4, Fig. 4)

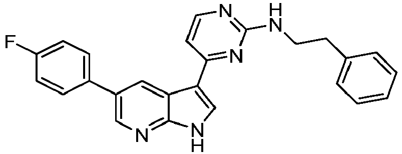

**¹H NMR (500 MHz, DMSO) δ** 12.34 (s, 1H), 9.23 (s, 1H), 8.56 (d, *J* = 2.2 Hz, 1H), 8.46 (s, 1H), 8.21 (s, 1H), 7.75 (s, 2H), 7.36 - 7.20 (m, 8H), 7.15 (d, *J=* 5.1 Hz, 1H), 3.83 (dd, *J=* 13.3, 6.8 Hz, 2H), 3.02 (t, *J* = 7.1 Hz, 2H); MS (ESI+): m\z 409.17

### N-phenethyl-4-(5-(4-(trifluoromethoxy)phenyl)-1H-pyrrolo[2,3-b]pyridin-3-yl)pyrimidin-2-amine (compound 1B3, Table 4, Fig. 4)

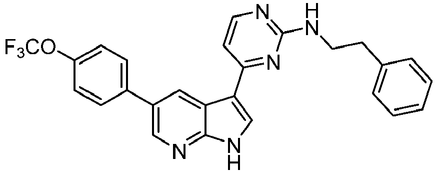

**¹H NMR (400 MHz, DMSO) δ** 12.35 (s, 1H), 9.24 (s, 1H), 8.59 (d, *J* = 2.2 Hz, 1H), 8.45 (d, *J* = 2.6 Hz, 1H), 8.21 (d, *J* = 5.2 Hz, 1H), 7.85 (d, *J* = 8.4 Hz, 2H), 7.49 (d, *J* = 8.0 Hz, 2H), 7.36 - 7.20 (m, 6H), 7.15 (d, *J* = 5.3 Hz, 1H), 3.83 (dd, *J =* 13.3, 6.8 Hz, 2H), 3.02 (t*, J =* 7.1 Hz, 2H); MS (ESI+): m\z 475.16

### N-phenethyl-4-(5-(3-(trifluoromethyl)phenyl)-1H-pyrrolo[2,3-b]pyridin-3-yl)pyrimidin-2-amine (compound 1B4, Table 4, Fig. 4)

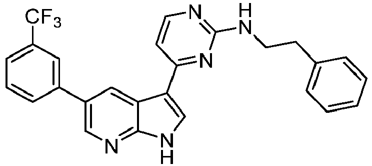

**¹H NMR (400 MHz, DMSO) δ** 12.40 (s, 1H), 9.34 (s, 1H), 8.67 (d, *J* = 1.7 Hz, 1H), 8.49 (d, *J* = 2.3 Hz, 1H), 8.21 (d, *J* = 5.1 Hz, 1H), 8.07 (d, *J* = 4.0 Hz, 1H), 7.98 (s, 1H), 7.75 (d, *J* = 4.7 Hz, 2H), 7.36 - 7.20 (m, 6H), 7.17 (d, *J* = 5.3 Hz, 1H), 3.83 (dd, *J* = 13.3, 6.8 Hz, 2H), 3.02 (t, *J* = 7.1 Hz, 2H); MS (ESI+): m\z 459.16

### 4-(5-(3-fluorophenyl)-1H-pyrrolo[2,3-b]pyridin-3-yl)-N-phenethylpyrimidin-2-amine (compound 1B5, Table 4, Fig. 4)

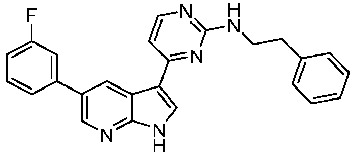

**¹H NMR (400 MHz, DMSO) δ** 12.37 (s, 1H), 9.25 (s, 1H), 8.62 (d, *J* = 2.0 Hz, 1H), 8.46 (d, *J* = 2.4 Hz, 1H), 8.21 (d, *J* = 5.0 Hz, 1H), 7.62 - 7.49 (m, 3H), 7.36 - 7.20 (m, 6H), 7.22 (t*, J=* 7.8 Hz, 1H), 7.16 (d, *J* = 5.2 Hz, 1H), 3.83 (dd, *J* = 13.3, 6.8 Hz, 2H), 3.02 (t, *J* = 7.1 Hz, 2H); MS (ESI+): m\z 409.17

### N-phenethyl-4-(5-(p-tolyl)-1H-pyrrolo[2,3-b]pyridin-3-yl)pyrimidin-2-amine (compound 1B6, Table 4, Fig. 4)

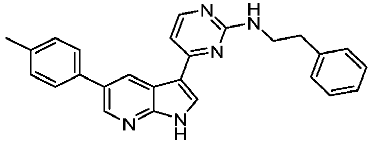

**¹H NMR (500 MHz, DMSO) δ** 12.36 (s, 1H), 9.24 (s, 1H), 8.56 (d, *J=* 2.2 Hz, 1H), 8.44 (s, 1H), 8.20 (s, 1H), 7.80 (s, 2H), 7.36 - 7.20 (m, 8H), 7.13 (d, *J=* 5.1 Hz, 1H), 2.33 (s, 3H), 3.83 (dd, *J* = 13.3, 6.8 Hz, 2H), 3.02 (t, *J* = 7.1 Hz, 2H); MS (ESI+): m\z 405.19

### N-phenethyl-4-(5-(m-tolyl)-1H-pyrrolo[2,3-b]pyridin-3-yl)pyrimidin-2-amine (compound 1B7, Table 4, Fig. 4)

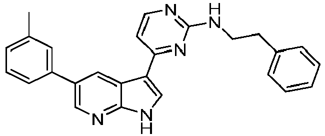

**¹H NMR (500 MHz, DMSO) δ** 12.40 (s, 1H), 9.28 (s, 1H), 8.53 (d, *J* = 2.2 Hz, 1H), 8.40 (s, 1H), 8.19 (s, 1H), 7.79 (s, 2H), 7.35 - 7.18 (m, 9H), 2.33 (s, 3H), 3.83 *(dd, J* = 13.3, 6.8 Hz, 2H), 3.02 (t, *J* = 7.1 Hz, 2H); MS (ESI+): m\z 405.19

### 4-(5-(4-(methylthio)phenyl)-1H-pyrrolo[2,3-b]pyridin-3-yl)-N-phenethylpyrimidin-2-amine (compound 1B8, Table 4, Fig. 4)

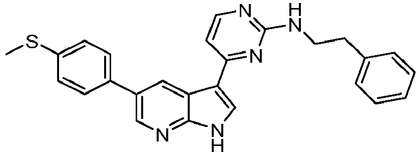

**¹H NMR (400 MHz, DMSO) δ** 12.36 (s, 1H), 9.02 (s, 1H), 8.44 (d, *J* = 2.2 Hz, 1H), 8.24 (s, 1H), 8.19 (d, *J=* 5.0 Hz, 1H), 7.70 (d, *J* = 8.4 Hz, 2H), 7.35 - 7.18 (m, 8H), 7.03 (d, *J=* 5.3 Hz, 1H), 2.38 (s, 3H), 3.83 (dd, *J* = 13.3, 6.8 Hz, 2H), 3.02 (t, *J* = 7.1 Hz, 2H); MS (ESI+): m\z 437.16

### 4-(5-(2-(methylthio)phenyl)-1H-pyrrolo[2,3-b]pyridin-3-yl)-N-phenethylpyrimidin-2-amine (compound 1B9, Table 4, Fig. 4)

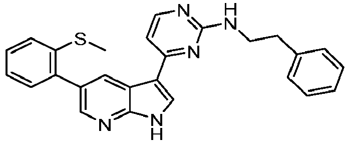

**¹H NMR (400 MHz, DMSO) δ** 12.33 (s, 1H), 9.05 (s, 1H), 8.44 (d, *J* = 2.2 Hz, 1H), 8.25 (s, 1H), 8.19 (d, *J* = 5.0 Hz, 1H), 7.46 - 7.39 (m, 1H), 7.36 - 7.18 (m, 9H), 7.13 (d, *J=* 5.3 Hz, 1H), 2.37 (s, 3H), 3.83 (dd, *J* = 13.3, 6.8 Hz, 2H), 3.02 (t, *J* = 7.1 Hz, 2H); MS (ESI+): m\z 437.16

### 4-(5-(2-ethylphenyl)-1H-pyrrolo[2,3-b]pyridin-3-yl)-N-phenethylpyrimidin-2-amine (compound 1B10, Table 4, Fig. 4)

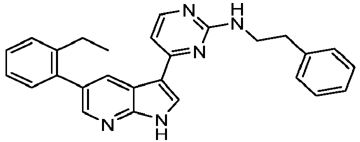

**¹H NMR (400 MHz, DMSO) δ** 12.31 (s, 1H), 8.98 (s, 1H), 8.45 (d, *J* = 2.5 Hz, 1H), 8.20 (dd, *J* = 7.6, 3.4 Hz, 2H), 7.36 - 7.18 (m, 10H), 7.13 (d, *J* = 5.3 Hz, 1H), 3.83 (dd, *J* = 13.3, 6.8 Hz, 2H), 3.02 (t, *J* = 7.1 Hz, 2H), 2.60 (dd, *J* = 15.0, 7.5 Hz, 2H), 1.01 (t, *J* = 7.5 Hz, 3H);
MS (ESI+): m\z 419.21

### 4-(5-(4-methoxyphenyl)-1H-pyrrolo[2,3-b]pyridin-3-yl)-N-phenethylpyrimidin-2-amine (compound 1B11, Table 4, Fig. 4)

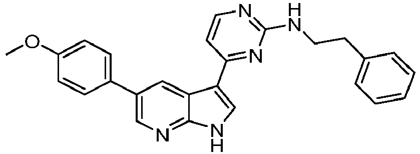

**¹H NMR (400 MHz, DMSO) δ** 12.23 (s, 1H), 9.16 (s, 1H), 8.51 (d, *J* = 2.2 Hz, 1H), 8.39 (s, 1H), 8.18 (d, *J=* 5.2 Hz, 1H), 7.63 (d, *J=* 8.3 Hz, 2H), 7.36 - 7.18 (m, 6H), 7.12 (d, *J* = 5.3 Hz, 1H), 7.04 (d, *J* = 8.7 Hz, 2H), 3.83 - 3.71 (m, 5H), 3.02 (t, *J* = 7.1 Hz, 2H); MS (ESI+): m\z 421.19

### 4-(5-(4-chlorophenyl)-1H-pyrrolo[2,3-b]pyridin-3-yl)-N-phenethylpyrimidin-2-amine (compound 1B12, Table 4, Fig. 4)

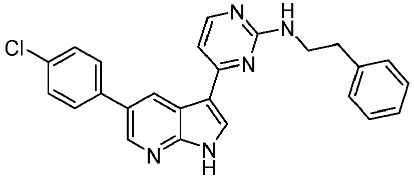

**¹H NMR (400 MHz, DMSO) δ** 12.32 (s, 1H), 9.28 (s, 1H), 9.13 (d, *J* = 1.9 Hz, 1H), 8.60 (d, *J* = 1.9 Hz, 1H), 8.40 (d, *J* = 2.4 Hz, 1H), 8.16 (d, *J* = 5.3 Hz, 1H), 7.84 (d*, J =* 8.4 Hz, 2H), 7.55 (d, *J* = 8.3 Hz, 2H), 7.36 - 7.18 (m, 6H), 3.83 (dd, *J* = 13.3, 6.8 Hz, 2H), 3.02 (t, *J* = 7.1 Hz, 2H); MS (ESI+): m\z 425.14

### 1-(4-(3-(2-(phenethylamino)pyrimidin-4-yl)-1H-pyrrolo[2,3-b]pyridin-5-yl)phenyl)ethan-1-one (compound 1B13, Table 4, Fig. 4)

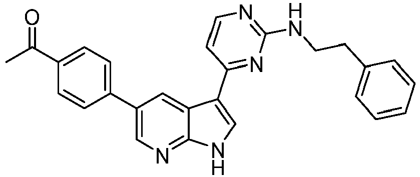

**¹H NMR (400 MHz, DMSO) δ** 12.32 (s, 1H), 9.89 (s, 1H), 9.28 (s, 1H), 9.13 (d, *J* = 1.9 Hz, 1H), 8.60 (d, *J* = 1.9 Hz, 1H), 8.40 (d, *J* = 2.4 Hz*,* 1H), 8.16 (d, *J* = 5.3 Hz, 1H), 8.11 (d, *J* = 8.4 Hz, 2H), 7.63 (d, *J* = 8.3 Hz, 2H), 7.36 - 7.14 (m, 6H), 3.83 (dd, *J* = 13.3, 6.8 Hz, 2H), 3.02 (t, *J* = 7.1 Hz, 2H); MS (ESI+): m\z 433.19

### 4-(5-(naphthalen-1-yl)-1H-pyrrolo[2,3-b]pyridin-3-yl)-N-phenethylpyrimidin-2-amine (compound 1B14, Table 4, Fig. 4)

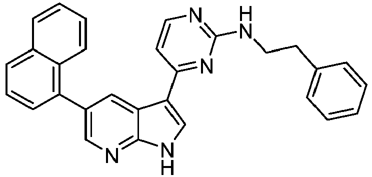

**¹H NMR (400 MHz, DMSO) δ** 12.20 (s, 1H), 9.00 (s, 1H), 8.30 (d, *J=* 2.7 Hz, 1H), 8.19 (s, 1H), 7.98 (d, *J* = 5.2 Hz, 1H), 7.81 (dd, *J* = 14.6, 8.1 Hz, 2H), 7.69 (s, 1H), 7.47 - 7.39 (m, 1H), 7.36 - 7.18 (m, 8H), 6.96 (dd, *J* = 9.9, 4.1 Hz, 2H), 3.83 (dd, *J* = 13.3, 6.8 Hz, 2H), 3.02 (t, *J* = 7.1 Hz, 2H); MS (ESI+): m\z 441.19

### N-phenethyl-4-(5-(pyridin-3-yl)-1H-pyrrolo[2,3-b]pyridin-3-yl)pyrimidin-2-amine (compound 1B15, Table 4, Fig. 4)

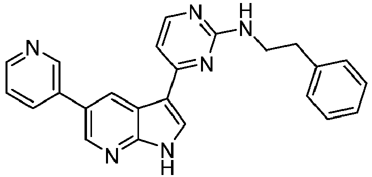

**¹H NMR (400 MHz, DMSO) δ** 12.39 (s, 1H), 9.29 (s, 1H), 8.96 (s, 1H), 8.63 (d, *J* = 2.2 Hz, 1H), 8.60(d, *J* = 4.6 Hz, 1H), 8.48 (s, 1H), 8.22 (d, *J* = 5.1 Hz, 1H), 8.14 (d, *J* = 7.5 Hz, 1H), 7.53 (dd, *J =* 7.7, 4.8 Hz, 1H), 7.36 - 7.18 (m,7H), 3.83 (dd, *J* = 13.3, 6.8 Hz, 2H), 3.02 (t, *J* = 7.1 Hz, 2H); MS (ESI+): m\z 392.17

### 4-(5-(furan-3-yl)-1H-pyrrolo[2,3-b]pyridin-3-yl)-N-phenethylpyrimidin-2-amine (compound 1B16, Table 4, Fig. 4)

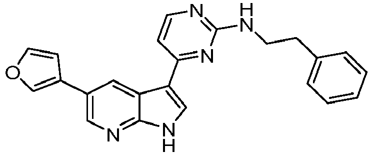

**¹H NMR (400 MHz, DMSO) δ** 12.25 (s, 1H), 9.09 (s, 1H), 8.59 (d, *J* = 2.1 Hz, 1H), 8.39 (d, *J* = 2.7 Hz, 1H), 8.21 (d, *J* = 5.3 Hz, 2H), 7.80 (t, *J* = 1.7 Hz, 1H), 7.36 - 7.18 (m, 8H), 3.83 (dd, *J* = 13.3, 6.8 Hz, 2H), 3.02 (t, *J* = 7.1 Hz, 2H); MS (ESI+): m\z 381.16

### 4-(5-(5-methoxy-1H-indol-2-yl)-1H-pyrrolo[2,3-b]pyridin-3-yl)-N-phenethylpyrimidin-2-amine (compound 1B17, Table 4, Fig. 4)

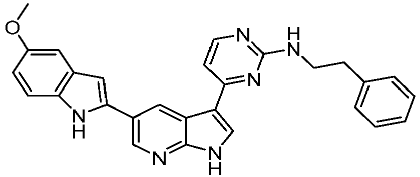

**¹H NMR (400 MHz, DMSO) δ** 12.40 (s, 1H), 11.42 (s, 1H), 9.32 (d, *J* = 1.9 Hz, 1H), 9.20 (s, 1H), 8.89 (d, *J* = 2.1 Hz, 1H), 8.46 (d, *J* = 2.7 Hz, 1H), 8.25 (d, *J* = 5.3 Hz, 1H), 7.40 - 7.20 (m, 7H), 7.09 (dd, *J* = 14.0, 1.7 Hz, 2H), 6.82 (dd, *J* = 8.7, 2.4 Hz, 1H), 3.80 (s, 3H), 3.83 (dd, *J* = 13.3, 6.8 Hz, 2H), 3.02 (t, *J* = 7.1 Hz, 2H); MS (ESI+): m\z 460.20

### 4-(5-(benzo[b]thiophen-2-yl)-1H-pyrrolo[2,3-b]pyridin-3-yl)-N-phenethylpyrimidin-2-amine (compound 1B18, Table 4, Fig. 4)

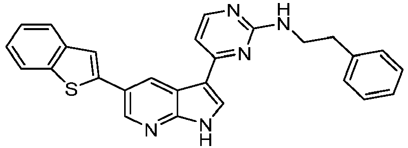

**¹H NMR (400 MHz, DMSO) δ** 12.44 (s, 1H), 9.27 (s, 1H), 9.15 (s, 1H), 8.71 (s, 1H), 8.46 (d, *J* = 1.9 Hz, 1H), 8.18 (d, *J* = 5.2 Hz, 1H), 8.08 - 7.96 (m, 2H), 7.88 (d, *J* = 7.6 Hz, 1H), 7.43- 7.22 (m, 7H), 7.12 (d, *J* = 5.3 Hz, 1H), 3.83 (dd, *J* = 13.3, 6.8 Hz, 2H), 3.02 (t, *J* = 7.1 Hz, 2H); MS (ESI+): m\z 447.15

### 4-(5-(1H-indol-2-yl)-1H-pyrrolo[2,3-b]pyridin-3-yl)-N-phenethylpyrimidin-2-amine (compound 1B19, Table 4, Fig. 4)

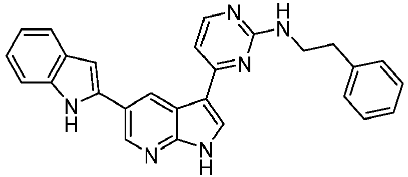

**¹H NMR (400 MHz, DMSO) δ** 12.50 (s, 1H), 11.56 (s, 1H), 9.30 (d, *J* = 2.1 Hz, 1H), 9.23 (s, 1H), 8.89 (d, *J* = 2.1 Hz, 1H), 8.46 (d, *J* = 2.7 Hz, 1H), 8.25 (d, *J* = 5.3 Hz, 1H), 7.40 - 7.18 (m, 7H), 7.12 - 7.01 (m, 3H), 6.82 (dd, *J* = 8.7, 2.4 Hz, 1H), 3.83 (dd, *J* = 13.3, 6.8 Hz, 2H), 3.02 (t, *J* = 7.1 Hz, 2H); MS (ESI+): m\z 430.19

### 4-(5-(isoquinolin-1-yl)-1H-pyrrolo[2,3-b]pyridin-3-yl)-N-phenethylpyrimidin-2-amine (compound 1B20, Table 4, Fig. 4)

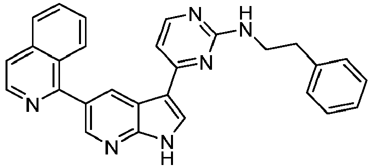

**¹H NMR (400 MHz, CDCl₃) δ** 12.40 (s, 1H), 9.34 (s, 1H), 9.24 (d, *J* = 2.2 Hz, 1H), 8.68 (d, *J* = 1.9 Hz, 1H), 8.37 (d, *J* = 2.0 Hz, 1H), 8.28 (d, *J* = 2.0 Hz, 1H), 8.17 (d, *J* = 8.4 Hz, 1H), 8.07 (d, *J* = 4.0 Hz, 1H), 7.92 (d, *J* = 8.1 Hz, 1H), 7.81 - 7.69 (m, 1H), 7.61 (t, *J* = 7.5 Hz, 1H), 7.45 (d, *J* = 3.3 Hz, 1H), 7.35 - 7.18 (m, 6H), 3.83 (dd, *J* = 13.3, 6.8 Hz, 2H), 3.02 (t, *J* = 7.1 Hz, 2H); MS (ESI+): m\z 442.19

### 4-(5-(4-(trifluoromethyl)phenyl)-1H-pyrrolo[2,3-b]pyridin-3-yl)pyrimidin-2-amine (compound 1C1, Table 5, Fig. 5 - for reference)

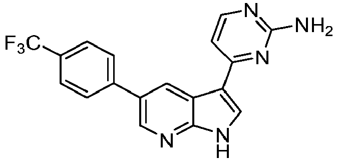

**¹H NMR (400 MHz, DMSO) δ** 12.38 (s, 1H), 9.22 (d, *J* = 2.1 Hz, 1H), 8.67 (d, *J* = 2.2 Hz, 1H), 8.44 (s, 1H), 8.16 (d, *J* = 5.3 Hz, 1H), 8.05 (d, *J* = 8.1 Hz, 2H), 7.85 (d, *J* = 8.2 Hz, 2H), 7.11 (d, *J* = 5.3 Hz, 1H), 6.61 (s, 2H); MS (ESI+): m\z 355.10

### 4-(5-(4-fluorophenyl)-1H-pyrrolo[2,3-b]pyridin-3-yl)pyrimidin-2-amine (compound 1C2, Table 5, Fig. 5 - for reference)

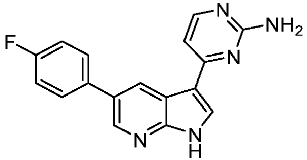

**¹H NMR (400 MHz, DMSO) δ** 12.31 (s, 1H), 9.10 (d, *J* = 2.0Hz, 1H), 8.58 (d, *J* = 2.1 Hz, 1H), 8.40 (d, *J* = 2.7 Hz, 1H), 8.15 (d, *J* = 5.3Hz, 1H), 7.89 - 7.75 (m, 2H), 7.33 (t, *J* = 8.8Hz, 2H), 7.10 (d, *J* = 5.3 Hz, 1H), 6.60 (s, 2H); MS (ESI+): m\z 305.11

### 4-(5-(4-(trifluoromethoxy)phenyl)-1H-pyrrolo[2,3-b]pyridin-3-yl)pyrimidin-2-amine (compound 1C3, Table 5, Fig. 5 - for reference)

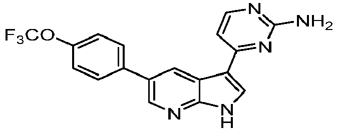

**¹H NMR (400 MHz, DMSO) δ** 12.36 (s, 1H), 9.15 (d, *J* = 1.8 Hz, 1H), 8.63 (d, *J* = 1.9 Hz, 1H), 8.42 (d, *J* = 2.6Hz, 1H), 8.16 (d, *J* = 5.3 Hz, 1H), 7.86 (d, *J* = 7.8Hz, 1H), 7.77 (s, 1H), 7.65 (t, *J* = 8.0 Hz, 1H), 7.40 (d, *J* = 8.0 Hz, 1H), 7.11 (d, *J* = 5.3 Hz, 1H), 6.61 (s, 2H); MS (ESI+): m\z 371.10

### 4-(5-(3-(trifluoromethyl)phenyl)-1H-pyrrolo[2,3-b]pyridin-3-yl)pyrimidin-2-amine (compound 1C4, Table 5, Fig. 5 - for reference)

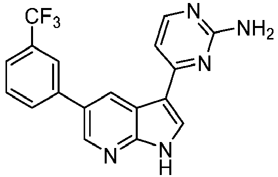

**¹H NMR (400 MHz, DMSO) δ** 12.34 (s, 1H), 9.10 (d, *J* = 1.2 Hz, 1H), 8.60 (s, 1H), 8.37 (s, 1H), 8.14 (d, *J* = 5.3 Hz, 1H), 8.08 - 7.98 (m, 2H), 7.72 (d, *J* = 4.3 Hz, 2H), 7.10 (d, *J* = 5.3 Hz, 1H), 6.55 (s, 2H); MS (ESI+): m\z 355.10

### 4-(5-(3-fluorophenyl)-1H-pyrrolo[2,3-b]pyridin-3-yl)pyrimidin-2-amine (compound 1C5, Table 5, Fig. 5 - for reference)

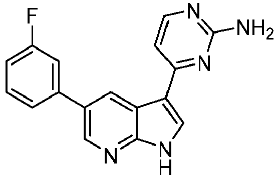

**¹H NMR (400 MHz, DMSO) δ** 12.35 (s, 1H), 9.14 (d, *J* = 2.0 Hz, 1H), 8.64 (d, *J* = 2.1 Hz, 1H), 8.42 (d, *J* = 2.7 Hz, 1H), 8.16 (d, *J* = 5.2 Hz, 1H), 7.70 - 7.62 (m, 2H), 7.55 (dd, *J* = 14.4, 8.0 Hz, 1H), 7.22 (t, *J* = 9.4 Hz, 1H), 7.11 (d, *J* = 5.3 Hz, 1H), 6.64 (s, 2H); MS (ESI+): m\z 305.11 **4-(5-(p-tolyl)-1*H*-pyrrolo[2,3-*b*]pyridin-3-yl)pyrimidin-2-amine (compound 1C6, Table 5,** Fig. 5 - for reference)

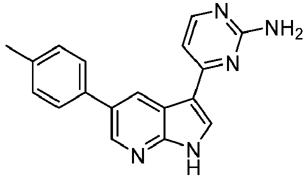

**¹H NMR (500 MHz, DMSO) δ** 12.36 (s, 1H), 9.24 (s, 1H), 8.56 (d, *J* = 2.2 Hz, 1H), 8.44 (s, 1H), 8.20 (s, 1H), 7.80 (s, 2H), 7.36 (d, *J* = 8.5 Hz, 2H), 7.13 (d, *J* = 5.1 Hz, 1H), 6.59 (s, 2H), 2.33 (s, 3H); MS (ESI+): m\z 301.13

### 4-(5-(m-tolyl)-1H-pyrrolo[2,3-b]pyridin-3-yl)pyrimidin-2-amine (compound 1C7, Table 5, Fig. 5 - for reference)

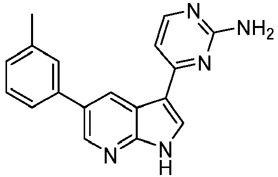

**¹H NMR (400 MHz, DMSO) δ** 12.28 (s, 1H), 9.08 (s, 1H), 8.57 (s, 1H), 8.39 (s, 1H), 8.16 (d, *J* = 4.5 Hz, 1H), 7.58 (d, *J* = 10.2 Hz, 2H), 7.40 (t, *J* = 7.3 Hz, 1H), 7.21 (d, *J* = 7.3 Hz, 1H), 7.10 (d, *J* = 4.5 Hz, 1H), 6.59 (s, 2H), 2.43 (s, 3H); MS (ESI+): m\z 301.13

### 4-(5-(4-(methylthio)phenyl)-1H-pyrrolo[2,3-b]pyridin-3-yl)pyrimidin-2-amine (compound 1C8, Table 5, Fig. 5 - for reference)

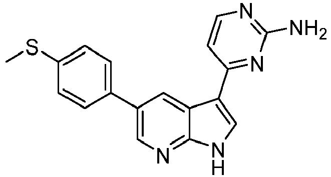

**¹H NMR (400 MHz, DMSO) δ** 12.21 (s, 1H), 9.03 (d, *J* = 2.1 Hz, 1H), 8.52 (d, *J* = 2.2 Hz, 1H), 8.31 (d, *J* = 2.6 Hz, 1H), 8.08 (d, *J* = 5.3 Hz, 1H), 7.70 (d, *J* = 8.4 Hz, 2H), 7.32 (d, *J* = 8.4 Hz, 2H), 7.03 (d, *J* = 5.3 Hz, 1H), 6.50 (s, 2H), 2.47 (s, 3H); MS (ESI+): m\z 333.10

### 4-(5-(2-(methylthio)phenyl)-1H-pyrrolo[2,3-b]pyridin-3-yl)pyrimidin-2-amine (compound 1C9, Table 5, Fig. 5 - for reference)

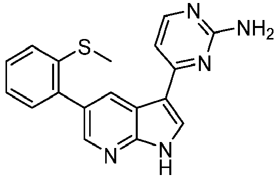

**¹H NMR (400 MHz, DMSO) δ** 12.31 (s, 1H), 8.82 (d, *J* = 2.0 Hz, 1H), 8.40 (d, *J* = 2.7 Hz, 1H), 8.21 (d, *J* = 2.1 Hz, 1H), 8.13 (d, *J* = 5.3 Hz, 1H), 7.44 (dd, *J* = 11.5, 5.1 Hz, 1H), 7.37 (d, *J* = 7.7 Hz, 1H), 7.32 - 7.25 (m, 2H), 7.07 (d, *J* = 5.3 Hz, 1H), 6.45 (s, 2H), 2.38 (s, 3H); MS (ESI+): m\z 333.10

### 4-(5-(2-ethylphenyl)-1H-pyrrolo[2,3-b]pyridin-3-yl)pyrimidin-2-amine (compound 1C10, Table 5, Fig. 5 - for reference)

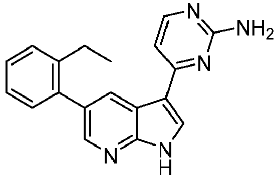

**¹H NMR (400 MHz, DMSO) δ** 12.29 (s, 1H), 8.83 (d, *J* = 2.1 Hz, 1H), 8.41 (d, *J* = 1.5 Hz, 1H), 8.20 (d, *J* = 2.1 Hz, 1H), 8.13 (d, *J* = 5.3 Hz, 1H), 7.41 - 7.35 (m, 2H), 7.31 - 7.23 (m, 2H), 7.08 (d, *J* = 5.3 Hz, 1H), 6.44 (s, 2H), 2.59 (q, *J* = 7.5 Hz, 2H), 1.09 - 1.01 (m, 3H); MS (ESI+): m\z 315.15

### 4-(5-(4-methoxyphenyl)-1H-pyrrolo[2,3-b]pyridin-3-yl)pyrimidin-2-amine (compound 1C11, Table 5, Fig. 5 - for reference)

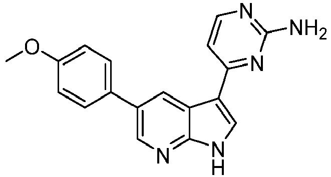

**¹H NMR (400 MHz, DMSO) δ** 12.24 (s, 1H), 9.05 (s, 1H), 8.55 (d, *J* = 2.2 Hz, 1H), 8.37 (d, *J* = 2.8 Hz, 1H), 8.15 (d, *J* = 5.3 Hz, 1H), 7.74 (d, *J* = 8.7 Hz, 2H), 7.08 (t, *J* = 7.5 Hz, 3H), 6.57 (s, 2H), 3.82 (s, 3H); MS (ESI+): m\z 317.12

### 4-(5-(4-chlorophenyl)-1H-pyrrolo[2,3-b]pyridin-3-yl)pyrimidin-2-amine (compound 1C12, Table 5, Fig. 5 - for reference)

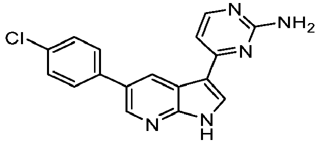

**¹H NMR (400 MHz, DMSO) δ** 12.32 (s, 1H), 9.13 (d, *J* = 1.9 Hz, 1H), 8.60 (d, *J* = 1.9 Hz, 1H), 8.40 (d, *J* = 2.4 Hz, 1H), 8.16 (d, *J* = 5.3 Hz, 1H), 7.84 (d, *J* = 8.4 Hz, 2H), 7.55 (d, *J* = 8.3 Hz, 2H), 7.09 (d, *J* = 15.8 Hz, 1H), 6.59 (s, 2H); MS (ESI+): m\z 321.078

### 4-(3-(2-aminopyrimidin-4-yl)-1H-pyrrolo[2,3-b]pyridin-5-yl)benzaldehyde (compound 1C13, Table 5, Fig. 5 - for reference)

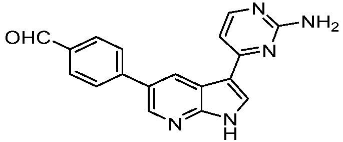

**¹H NMR (400 MHz, DMSO) δ** 12.32 (s, 1H), 9.89 (s, 1H), 9.13 (d, *J* = 1.9 Hz, 1H), 8.60 (d, *J* = 1.9 Hz, 1H), 8.40 (d, *J* = 2.4 Hz, 1H), 8.16 (d, *J* = 5.3 Hz, 1H), 8.11 (d, *J* = 8.4 Hz, 2H), 7.63 (d, *J* = 8.3 Hz, 2H), 7.09 (d, *J* = 15.8 Hz, 1H), 6.59 (s, 2H); MS (ESI+): m\z 315.11

### 4-(5-(naphthalen-1-yl)-1H-pyrrolo[2,3-b]pyridin-3-yl)pyrimidin-2-amine (compound 1C14, Table 5, Fig. 5 - for reference)

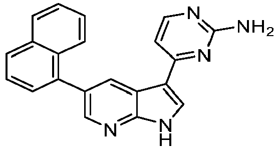

**¹H NMR (400 MHz, DMSO) δ** 12.39 (s, 1H), 8.95 (d, *J* = 2.1 Hz, 1H), 8.46 (d, *J* = 2.7 Hz, 1H), 8.32 (d, *J* = 2.6 Hz, 1H), 8.09 (dd, *J* = 44.1, 7.8 Hz, 3H), 7.75 (d, *J* = 7.3 Hz, 1H), 7.70 - 7.46 (m, 4H), 7.10 (d, *J=* 2.5 Hz, 1H), 6.40 (s, 2H); MS (ESI+): m\z 337.13

### 4-(5-(pyridin-3-yl)-1H-pyrrolo[2,3-b]pyridin-3-yl)pyrimidin-2-amine (compound 1C15, Table 5, Fig. 5 - for reference)

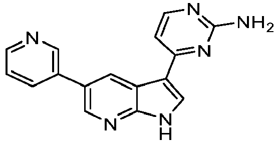

**¹H NMR (400 MHz, DMSO) δ** 12.40 (s, 1H), 9.21 (s, 1H), 9.04 (s, 1H), 8.73 - 8.54 (m, 2H), 8.46 (s, 1H), 8.19 (dd, *J* = 25.9, 6.3 Hz, 2H), 7.60 - 7.47 (m, 1H), 7.13 (d, *J* = 5.1 Hz, 1H), 6.70 (s, 2H); MS (ESI+): m\z 288.11

### 4-(5-(furan-3-yl)-1H-pyrrolo[2,3-b]pyridin-3-yl)pyrimidin-2-amine (compound 1C16, Table 5, Fig. 5 - for reference)

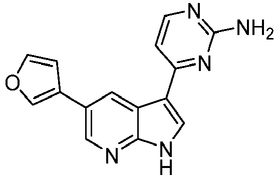

**¹H NMR (400 MHz, DMSO) δ** 12.24 (s, 1H), 9.07 (s, 1H), 8.62 (d, *J* = 1.5 Hz, 1H), 8.37 (s, 2H), 8.15 (d, *J* = 5.2 Hz, 1H), 7.81 (s, 1H), 7.22 (s, 1H), 7.08 (d, *J* = 5.3 Hz, 1H), 6.64 (s, 2H); MS (ESI+): m\z 277.09

### 4-(5-(5-methoxy-1H-indol-2-yl)-1H-pyrrolo[2,3-b]pyridin-3-yl)pyrimidin-2-amine (compound 1C17, Table 5, Fig. 5 - for reference)

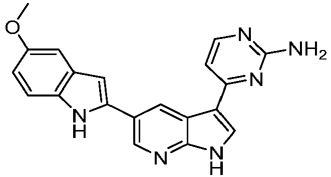

**¹H NMR (400 MHz, DMSO) δ** 12.38 (s, 1H), 11.45 (s, 1H), 9.32 (d, *J* = 1.9 Hz, 1H), 8.89 (d, *J* = 2.1 Hz, 1H), 8.46 (d, *J* = 2.7 Hz, 1H), 8.25 (d, *J* = 5.3 Hz, 1H), 7.40 (d, *J* = 8.7 Hz, 1H), 7.19 (d, *J* = 5.3 Hz, 1H), 7.09 (dd, *J* = 14.0, 1.7 Hz, 2H), 6.82 (dd, *J* = 8.7, 2.4 Hz, 1H), 6.72 (s, 2H), 3.84 (s, 3H); MS (ESI+): m\z 356.13

### 4-(5-(benzo[b]thiophen-2-yl)-1H-pyrrolo[2,3-b]pyridin-3-yl)pyrimidin-2-amine (compound 1C18, Table 5, Fig. 5 - for reference)

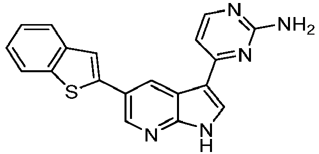

**¹H NMR (400 MHz, DMSO) δ** 12.44 (s, 1H), 9.27 (s, 1H), 8.71 (s, 1H), 8.46 (d, *J* = 1.9 Hz, 1H), 8.18 (d, *J* = 5.2 Hz, 1H), 8.08 - 7.96 (m, 2H), 7.88 (d, *J* = 7.6 Hz, 1H), 7.45- 7.36 (m, 2H), 7.12 (d, *J* = 5.3 Hz, 1H), 6.67 (s, 2H); MS (ESI+): m\z 343.09

### 4-(5-(1H-indol-2-yl)-1H-pyrrolo[2,3-b]pyridin-3-yl)pyrimidin-2-amine (compound 1C19, Table 5, Fig. 5 - for reference)

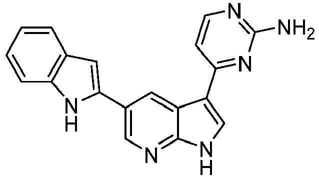

**¹H NMR (400 MHz, DMSO) δ** 12.50 (s, 1H), 11.56 (s, 1H), 9.30 (d, *J* = 2.1 Hz, 1H), 9.23 (s, 1H), 8.89 (d, *J* = 2.1 Hz, 1H), 8.46 (d, *J* = 2.7 Hz, 1H), 8.25 (d, *J* = 5.3 Hz, 1H), 7.40 (d, *J* = 8.7 Hz, 1H), 7.19 (d, *J* = 5.3 Hz, 1H), 7.12 - 7.01 (m, 3H), 6.82 (dd, *J* = 8.7, 2.4 Hz, 1H), 6.64 (s, 2H); MS (ESI+): m\z 326.12

### 4-(5-(isoquinolin-1-yl)-1H-pyrrolo[2,3-b]pyridin-3-yl)pyrimidin-2-amine (compound 1C20, Table 5, Fig. 5 - for reference)

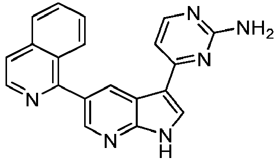

**¹H NMR (400 MHz, CDCl₃) δ** 12.40 (s, 1H), 9.34 (s, 1H), 9.24 (d, *J* = 2.2 Hz, 1H), 8.68 (d, *J* = 1.9 Hz, 1H), 8.37 (d, *J* = 2.0 Hz, 1H), 8.28 (d, *J* = 2.0 Hz, 1H), 8.17 (d, *J* = 8.4 Hz, 1H), 8.07 (d, *J* = 4.0 Hz, 1H), 7.92 (d, *J* = 8.1 Hz, 1H), 7.81 - 7.69 (m, 1H), 7.61 (t, *J* = 7.5 Hz, 1H), 7.45 (d, *J* = 3.3 Hz, 1H), 7.17 (d, *J* = 5.3 Hz, 1H), 6.76 (s, 2H); MS (ESI+): m\z 338.13.

### In vitro cell line activity:

**Cell culture, growth conditions and treatments.** Human breast cancer cell lines MDAMB231, MFC-7, human prostate carcinoma cell line PC-3, HCT-116 and neuroblastoma cell line SHSY-5Y was obtained from European Collection of Cell Cultures (ECACC). Cells were grown in RPMI-1640 medium supplemented with 10% fetal bovine serum (FBS), penicillin (100 units/ml), streptomycin (100 µg/ml), L-glutamine (0.3 mg/ml), sodium pyruvate (550 mg/ml), and NaHCO₃ (2mg/ml). Cells were grown in CO₂ incubator (Thermocon Electron Corporation, USA) at 37°C in an atmosphere of 95% air and 5% CO₂ with 98% humidity. Different molecules were dissolved in DMSO and were delivered to cell cultures in complete medium.

**Results:** Different derivatives were screened for their antiproliferative activity against two breast cancer cell lines MCF-7, MDAMB-435, one prostate cancer cell line PC-3, colon cancer cell line HCT-116 and neuroblastoma cell line SHSY-5Y. Many molecules displayed potent anti proliferative activities in in-vitro cancer cell lines assay. The results for compounds 1C are provided for reference. Compounds are 1A2, 1A5, 1A11, 1A12, 1A14, 1A15 shows the greater than 70% of inhibition against MCF-7, MDAMB-435, HCT-116 and SHSY-5Y cell lines at 10 µM. 1A2, 1A11, 1A12, 1A15, 1A31, 1A42 and 1B5 these compounds are shown greater than 50% of inhibition against PC-3 cell lines at 10µM. 1A2, 1A5, 1A11, 1A12, 1A14, 1A15, 1A51, 1C2, 1C3, 1C4, 1C5, 1C6, 1C9, 1C10, 1C12, 1C15, 1C16, 1C17 and 1C19 shows the greater than 70% of inhibition against MCF-7 cell lines at 10µM. Compounds are 1A16, 1A22, 1A23, 1A41, 1B1, 1B5, 1B11, 1B15, 1C1, 1C7, 1C8, 1C11, 1C13, 1C14 and 1C20 shown the greater than 50% of inhibition against MCF-7 cell lines at 10µM. 1A31, 1A42 and 1A55 are shown the less than 50% of inhibition against MCF-7 cell lines at 10µM. 1A2, 1A5, 1A11, 1A12, 1A14, 1A15, 1A22, 1A23, 1A31, 1A41, 1A42, 1A55, 1B1, 1B2, 1B5, 1B12, 1B15, 1C2, 1C3, 1C4, 1C5, 1C6, 1C7, 1C9, 1C10, 1C11, 1C12, 1C15, 1C16, 1C18 and 1C19 these compounds are shown the greater than 70% of inhibition against SHSY-5Y cell lines at 10µM.

Standard drug is shown the greater than 70% of inhibition against SHSY-5Y cell line.

### Bio-chemical assay (CDK inhibition assays).

Compounds were evaluated for their ability to inhibit CDK2 and CDK9 as given below.

**CDK-2/cyclin** A (5-20 mU diluted in 50 mM Hepes pH 7.5, 1 mM DTT, 0.02% Brij35, 100 mM NaCl) was assayed against Histone HI in a final volume of 25.5 µl containing 50 mM Hepes pH7.5, 1 mM DTT, 0.02% Brij35, 100 mM NaCl, Histone HI (1 mg/ml), 10 mM magnesium acetate and 0.02 mM [33P-g-ATP](500-1000 cpm/pmole) and incubated for 30 min at room temperature. Assays were stopped by addition of 5 µl of 0.5 M (3%) orthophosphoric acid and then harvested onto P81 Unifilter plates with a wash buffer of 50 mM orthophosphoric acid.

**CDK-9/Cyclin T1** (5-20mU diluted in 50 mM Tris pH 7.5, 0.1 mM EGTA, 1 mg/ml BSA, 0.1% Mercaptoethanol) was assayed against a substrate peptide (YSPTSPSYSPTSPSYSPTSPKKK) in a final volume of 25.5 µl containing 50 mM Tris pH 7.5, 0.1mM EDTA, 10mM DTT, 1mg/ml BSA, 0.3 mM YSPTSPSYSPTSPSYSPTSPKKK, 10 mM magnesium acetate and 0.05 mM [33P-γ-ATP] (50-1000 cpm/pmole) and incubated for 30 min at room temperature. Assays were stopped by addition of 5 µl of 0.5 M (3%) orthophosphoric acid and then harvested onto P81 Unifilter plates with a wash buffer of 50 mM orthophosphoric acid.

**Results**: CDKs % of inhibition values of a drug measures the effectiveness of a compound in inhibiting biological or biochemical function. The determination of enzyme based % of inhibition values helps in early analysis and estimation of the drug activities in order to narrow down drug candidates for further experimental purpose. The results for compounds 1C are provided for reference. The compounds 1A2, 1A3, 1A4, 1A5, 1A6, 1A7, 1A8, 1A9, 1A10, 1A11, 1A12, 1A13, 1A16, 1A18, 1A19, 1A29, 1A50, 1A51, 1A52, 1A53, 1A54, 1A55, 1A56, 1A57, 1A58, 1A59, 1A60, 1B4, 1B6, 1B7, 1B12, 1B15, 1B16, 1C1, 1C2, 1C3, 1C4, 1C5, 1C6, 1C7, 1C8, 1C9, 1C10, 1C11, 1C12, 1C13, 1C14 and 1C16 are shown the greater than 70% inhibition at 500 nM against CDK2 and CDK9. These compounds are 1A14, 1A15, 1A17, 1A18, 1A20, 1A24,

1A26, 1A27, 1A28, 1A30, 1A31, 1A32, 1A35, 1A36, 1A38, 1A39, 1A40, 1A42, 1A43, 1A46, 1A47, 1A48, 1B2, 1B3, 1B8, 1B9, 1B10, 1B11, 1B13, 1B14, 1B17, 1B18, 1B19, 1B20 and 1C15 are shown the greater than 50% inhibition at 500 nM against CDK2 and CDK9. The standard drug (table 6) showed greater than 70 % inhibition of CDK2 and CDK9 at 500 nM concentration. Finally, 52 compounds are shown the % of inhibition near to standard drug % of inhibition.

**Table 1**

| **Entry** | **Code** | **Structure** | **Entry** | **Code** | **Structure** |
|---|---|---|---|---|---|
| 1 | **1A1** | 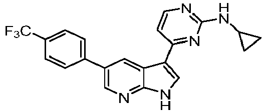 | 11 | **1A11** | 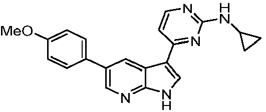 |
| 2 | **1A2** | 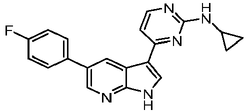 | 12 | **1A12** | 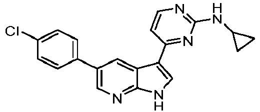 |
| 3 | **1A3** | 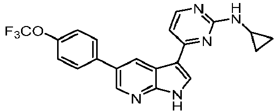 | 13 | **1A13** | 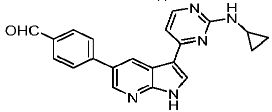 |
| 4 | **1A4** | 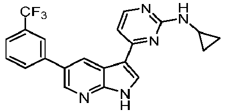 | 14 | **1A14** | 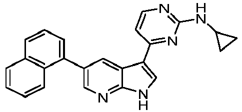 |
| 5 | **1A5** | 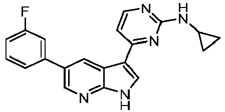 | 15 | **1A15** | 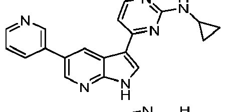 |
| 6 | **1A6** | 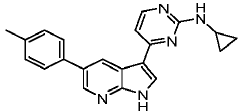 | 16 | **1A16** | 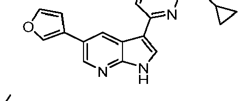 |
| 7 | **1A7** | 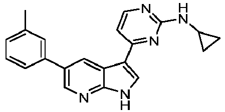 | 17 | **1A17** | 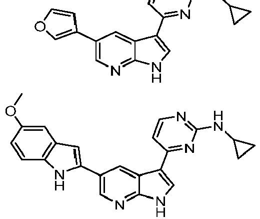 |
| 8 | **1A8** | 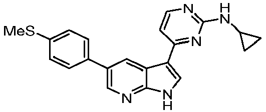 | 18 | **1A18** | 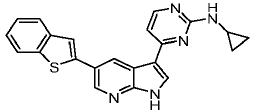 |
| 9 | **1A9** | 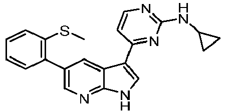 | 19 | **1A19** | 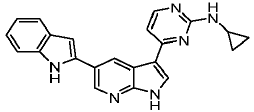 |
| 10 | **1A10** | 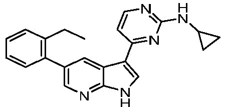 | 20 | **1A20** | 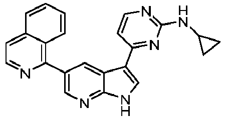 |

**Table 2**

| **Entry** | **Code** | **Structure** | **Entry** | **Code** | **Structure** |
|---|---|---|---|---|---|
| 1 | **1A21** | 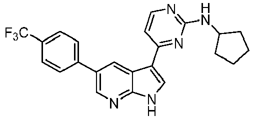 | 11 | **1A31** | 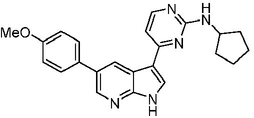 |
| 2 | **1A22** | 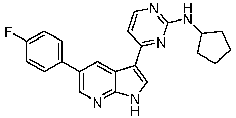 | 12 | **1A32** | 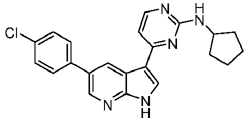 |
| 3 | **1A23** | 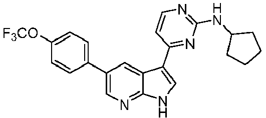 | 13 | **1A33** | 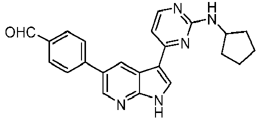 |
| 4 | **1A24** | 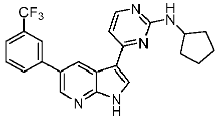 | 14 | **1A34** | 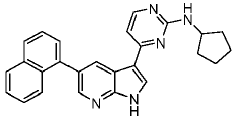 |
| 5 | **1A25** | 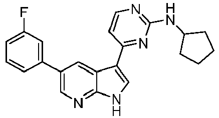 | 15 | **1A35** | 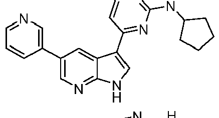 |
| 6 | **1A26** | 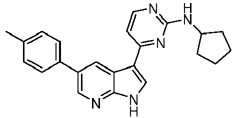 | 16 | **1A36** | 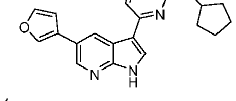 |
| 7 | **1A27** | 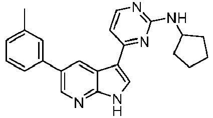 | 17 | **1A37** | 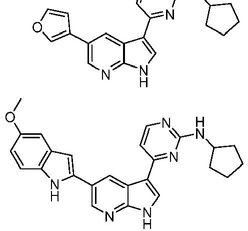 |
| 8 | **1A28** | 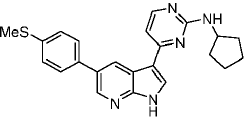 | 18 | **1A38** | 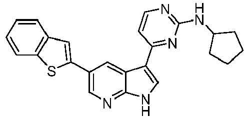 |
| 9 | **1A29** | 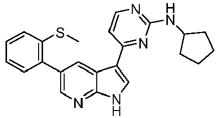 | 19 | **1A39** | 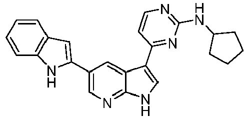 |
| 10 | **1A30** | 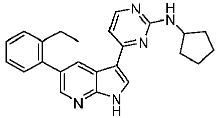 | 20 | **1A40** | 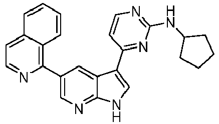 |

**Table 3**

| **Entry** | **Code** | **Structure** | **Entry** | **Code** | **Structure** |
|---|---|---|---|---|---|
| 1 | **1A41** | 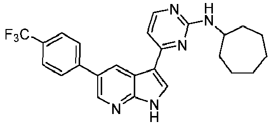 | 11 | **1A51** | 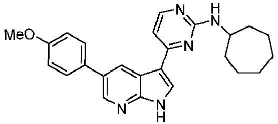 |
| 2 | **1A42** | 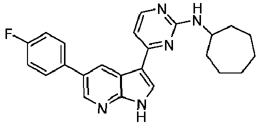 | 12 | **1A52** | 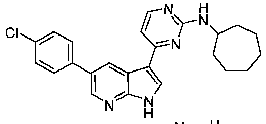 |
| 3 | **1A43** | 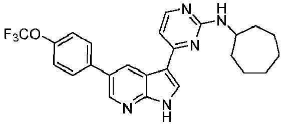 | 13 | **1A53** | 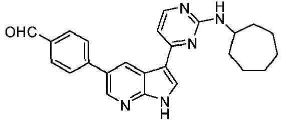 |
| 4 | **1A44** | 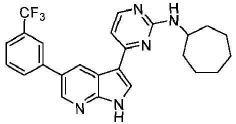 | 14 | **1A54** | 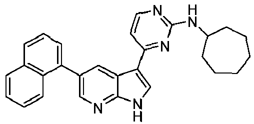 |
| 5 | **1A45** | 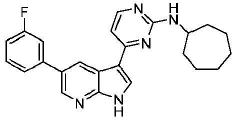 | 15 | **1A55** | 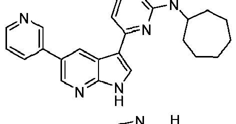 |
| 6 | **1A46** | 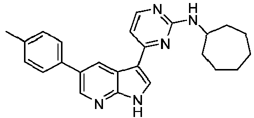 | 16 | **1A56** | 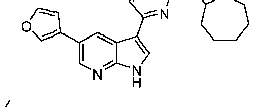 |
| 7 | **1A47** | 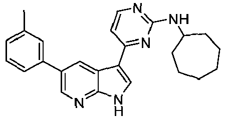 | 17 | **1A57** | 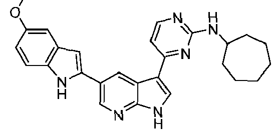 |
| 8 | **1A48** | 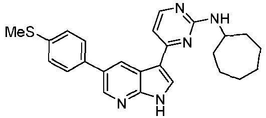 | 18 | **1A58** | 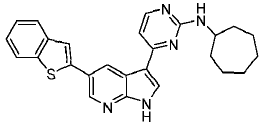 |
| 9 | **1A49** | 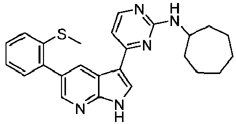 | 19 | **1A59** | 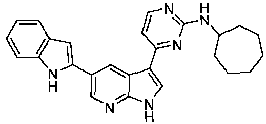 |
| 10 | **1A50** | 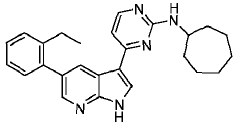 | 20 | **1A60** | 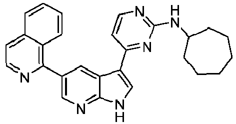 |

**Table 4**

| **Entry** | **Code** | **Structure** | **Entry** | **Code** | **Structure** |
|---|---|---|---|---|---|
| 1 | **1B1** | 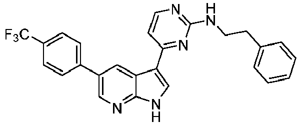 | 11 | **1B11** | 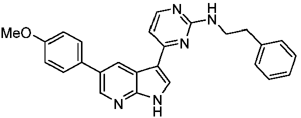 |
| 2 | **1B2** | 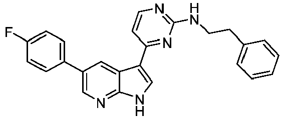 | 12 | **1B12** | 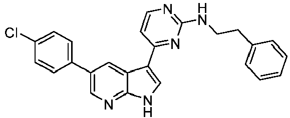 |
| 3 | **1B3** | 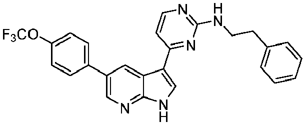 | 13 | **1B13** | 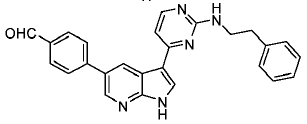 |
| 4 | **1B4** | 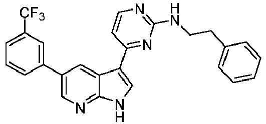 | 14 | **1B14** | 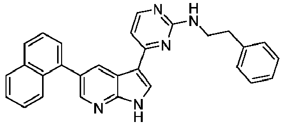 |
| 5 | **1B5** | 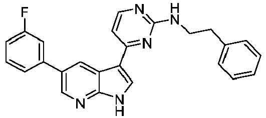 | 15 | **1B15** | 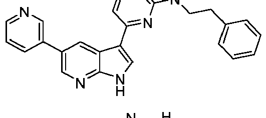 |
| 6 | **1B6** | 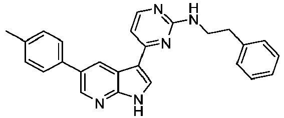 | 16 | **1B16** | 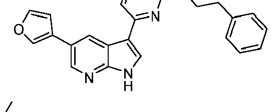 |
| 7 | **1B7** | 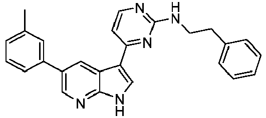 | 17 | **1B17** | 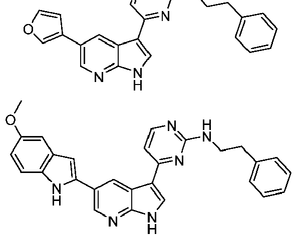 |
| 8 | **1B8** | 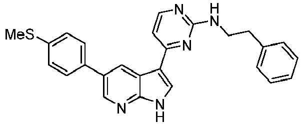 | 18 | **1B18** | 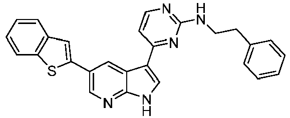 |
| 9 | **1B9** | 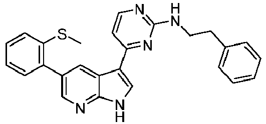 | 19 | **1B19** | 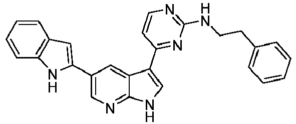 |
| 10 | **1B10** | 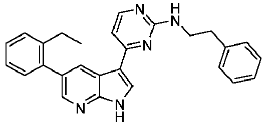 | 20 | **1B20** | 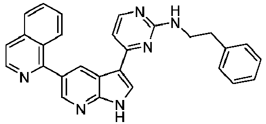 |

**Table 5 (for reference)**

| **Entry** | **Code** | **Structure** | **Entry** | **Code** | **Structure** |
|---|---|---|---|---|---|
| 1 | **1C1** | 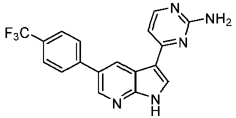 | 11 | **1C11** | 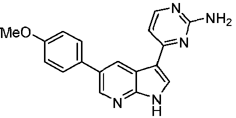 |
| 2 | **1C2** | 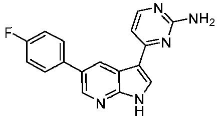 | 12 | **1C12** | 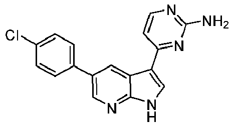 |
| 3 | **1C3** | 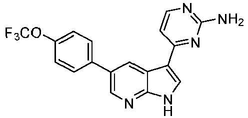 | 13 | **1C13** | 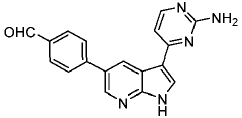 |
| 4 | **1C4** | 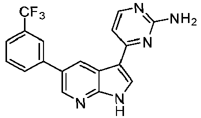 | 14 | **1C14** | 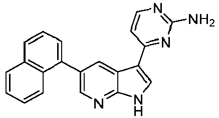 |
| 5 | **1C5** | 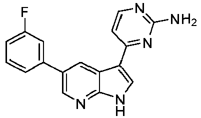 | 15 | **1C15** | 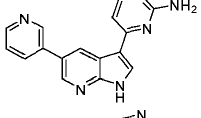 |
| 6 | 1C6 | 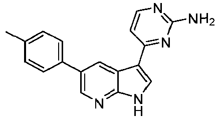 | 16 | **1C16** | 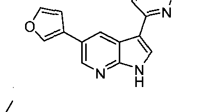 |
| 7 | **1C7** | 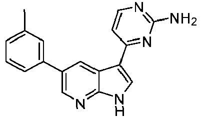 | 17 | **1C17** | 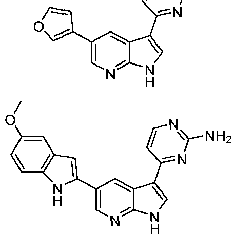 |
| 8 | **1C8** | 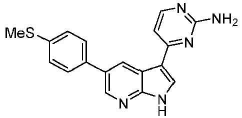 | 18 | **1C18** | 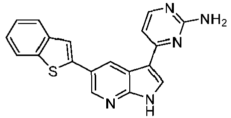 |
| 9 | **1C9** | 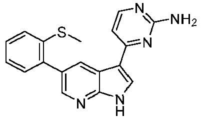 | 19 | **1C19** | 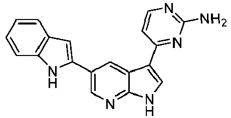 |
| 10 | **1C10** | 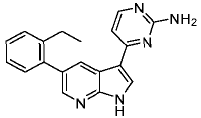 | 20 | **1C20** | 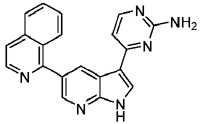 |

**Table-6**

| **Entry** | **Code** | **In vitro CDK2 % of inhibition** | **In vitro CDK9 % of inhibition** | **Entry** | **Code** | **In vitro CDK2 % of inhibition** | **In vitro CDK9 % of inhibition** |
|---|---|---|---|---|---|---|---|
| 1. | **1A1** | + | + | 26. | **1A26** | ++ | ++ |
| 2. | **1A2** | +++ | +++ | 27. | **1A27** | ++ | ++ |
| 3. | **1A3** | +++ | +++ | 28. | **1A28** | ++ | ++ |
| 4. | **1A4** | +++ | +++ | 29. | **1A29** | +++ | +++ |
| 5. | **1A5** | +++ | +++ | 30. | **1A30** | ++ | ++ |
| 6. | **1A6** | +++ | +++ | 31. | **1A31** | ++ | ++ |
| 7. | **1A7** | +++ | +++ | 32. | **1A32** | ++ | ++ |
| 8. | **1A8** | +++ | +++ | 33. | **1A33** | +++ | ++ |
| 9. | **1A9** | +++ | +++ | 34. | **1A34** | ++ | ++ |
| 10. | **1A10** | +++ | +++ | 35. | **1A35** | ++ | ++ |
| 11. | **1A11** | +++ | +++ | 36. | **1A36** | ++ | ++ |
| 12. | **1A12** | +++ | +++ | 37. | **1A37** | +++ | +++ |
| 13. | **1A13** | +++ | +++ | 38. | **1A38** | ++ | ++ |
| 14. | **1A14** | ++ | ++ | 39. | **1A39** | ++ | ++ |
| 15. | **1A15** | ++ | ++ | 40. | **1A40** | ++ | ++ |
| 16. | **1A16** | +++ | +++ | 41. | **1A41** | + | + |
| 17. | **1A17** | ++ | ++ | 42. | **1A42** | ++ | ++ |
| 18. | **1A18** | +++ | +++ | 43. | **1A43** | ++ | ++ |
| 19. | **1A19** | +++ | +++ | 44. | **1A44** | +++ | ++ |
| 20. | **1A20** | ++ | + | 45. | **1A45** | +++ | +++ |
| 21. | **1A21** | + | + | 46. | **1A46** | ++ | ++ |
| 22. | **1A22** | ++ | ++ | 47. | **1A47** | ++ | ++ |
| 23 | **1A23** | +++ | ++ | 48. | **1A48** | ++ | ++ |
| 24 | **1A24** | ++ | ++ | 49. | **Meriolin 1** | +++ | +++ |
| 25. | **1A25** | +++ | +++ | 50. | **Flavopyr idol** | +++ | +++ |

**In vitro CDK2 and CDK9 kinase inhibition at 500 nM; + indicates less than 50% of inhibition (+ < 50% of inhibition), ++ indicates greater than 50% of inhibition (++ >50% of inhibition) and +++ indicates greater than 70% of inhibition (+++ >70% of inhibition).**

**Table-7**

| **Entry** | **Code** | **In vitro CDK2 % of inhibition** | **In vitro CDK9 % of inhibition** | **Entry** | **Code** | **In vitro CDK2 % of inhibition** | **In vitro CDK9 % of inhibition** |
|---|---|---|---|---|---|---|---|
| 1. | **1A49** | + | + | 25. | **1B13** | ++ | ++ |
| 2. | **1A50** | +++ | +++ | 26. | **1B14** | ++ | ++ |
| 3. | **1A51** | +++ | +++ | 27. | **1B15** | +++ | +++ |
| 4. | **1A52** | +++ | +++ | 28. | **1B16** | +++ | +++ |
| 5. | **1A53** | +++ | +++ | 29. | **1B17** | ++ | ++ |
| 6. | **1A54** | +++ | +++ | 30. | **1B18** | ++ | ++ |
| 7. | **1A55** | +++ | +++ | 31. | **1B19** | ++ | ++ |
| 8. | **1A56** | +++ | +++ | 32. | **1B20** | ++ | ++ |
| 9. | **1A57** | +++ | +++ | 33. | **1C1** | +++ | +++ |
| 10. | **1A58** | +++ | +++ | 34. | **1C2** | +++ | +++ |
| 11. | **1A59** | +++ | +++ | 35. | **1C3** | +++ | +++ |
| 12. | **1A60** | +++ | +++ | 36. | **1C4** | +++ | +++ |
| 13. | **1B1** | + | + | 37. | **1C5** | +++ | +++ |
| 14. | **1B2** | ++ | ++ | 38. | **1C6** | +++ | +++ |
| 15. | **1B3** | ++ | ++ | 39. | **1C7** | +++ | +++ |
| 16. | **1B4** | +++ | +++ | 40. | **1C8** | +++ | +++ |
| 17. | **1B5** | ++ | ++ | 41. | **1C9** | +++ | +++ |
| 18. | **1B6** | +++ | +++ | 42. | **1C10** | +++ | +++ |
| 19. | **1B7** | +++ | +++ | 43. | **1C11** | +++ | +++ |
| 20. | **1B8** | ++ | ++ | 44. | **1C12** | +++ | +++ |
| 21. | **1B9** | ++ | ++ | 45. | **1C13** | +++ | +++ |
| 22. | **1B10** | ++ | ++ | 46. | **1C14** | +++ | +++ |
| 23 | **1B11** | ++ | ++ | 47 | **1C15** | ++ | ++ |
| 24 | **1B12** | +++ | +++ | 48 | **1C16** | +++ | +++ |

**In vitro CDK2 and CDK9 kinase inhibition at 500 nM; + indicates less than 50% of inhibition (+ < 50% of inhibition), ++ indicates greater than 50% of inhibition (++ >50% of inhibition) and +++ indicates greater than 70% of inhibition (+++ >70% of inhibition).**

**Table 8**

| **Entry** | **Code** | **MCF-7** | **PC-3** | **MDA-MB435** | **HCT-116** | **SHSY-5Y** |
|---|---|---|---|---|---|---|
| 1. | **1A2** | +++ | ++ | +++ | +++ | +++ |
| 2. | **1A5** | +++ | +++ | +++ | +++ | +++ |
| 3. | **1A11** | +++ | ++ | +++ | +++ | +++ |
| 4. | **1A12** | +++ | ++ | +++ | +++ | +++ |
| 5. | **1A14** | +++ | +++ | +++ | +++ | +++ |
| 6. | **1A15** | +++ | ++ | +++ | +++ | +++ |
| 7. | **1A16** | ++ | +++ | +++ | +++ | ++ |
| 8. | **1A22** | ++ | ++ | + | ++ | +++ |
| 9. | **1A23** | ++ | ++ | + | ++ | +++ |
| 10. | **1A31** | + | ++ | ++ | ++ | +++ |
| 11. | **1A41** | ++ | + | + | ++ | +++ |
| 12. | **1A42** | + | ++ | +++ | ++ | +++ |
| 13. | **1A51** | +++ | + | + | ++ | ++ |
| 14. | **1A55** | + | ++ | ++ | ++ | +++ |
| 15. | **1B1** | ++ | +++ | + | ++ | +++ |
| 16. | **1B2** | ++ | + | ++ | ++ | +++ |
| 17. | **1B5** | ++ | ++ | ++ | ++ | +++ |
| 18. | **1B11** | ++ | + | ++ | ++ | ++ |
| 19. | **1B12** | ++ | +++ | + | ++ | +++ |
| 20. | **1B15** | ++ | + | + | ++ | +++ |
| 21. | **Meriolin 1** | ++ | ++ | ++ | ++ | +++ |

**In vitro cell inhibition at 10µM; + indicates less than 50% of inhibition (+ < 50% of inhibition), ++ indicates greater than 50% of inhibition (++ >50% of inhibition) and +++ indicates greater than 70% of inhibition (+++ >70% of inhibition).**

**Table 9 (for reference)**

| **Entry** | **Code** | **MCF-7** | **PC-3** | **MDA-MB435** | **HCT-116** | **SHSY-5Y** |
|---|---|---|---|---|---|---|
| 1. | **1C1** | ++ | ++ | + | ++ | ++ |
| 2. | **1C2** | +++ | + | +++ | +++ | +++ |
| 3. | **1C3** | +++ | ++ | +++ | +++ | +++ |
| 4. | **1C4** | +++ | + | +++ | +++ | +++ |
| 5. | **1C5** | +++ | + | +++ | +++ | +++ |
| 6. | **1C6** | +++ | ++ | +++ | +++ | +++ |
| 7. | **1C7** | ++ | ++ | +++ | +++ | +++ |
| 8. | **1C8** | ++ | + | +++ | +++ | ++ |
| 9. | **1C9** | +++ | +++ | +++ | ++ | +++ |
| 10. | **1C10** | +++ | +++ | +++ | +++ | +++ |
| 11. | **1C11** | ++ | + | +++ | +++ | +++ |
| 12. | **1C12** | +++ | +++ | +++ | +++ | +++ |
| 13. | **1C13** | ++ | ++ | ++ | ++ | ++ |
| 14. | **1C14** | ++ | +++ | ++ | +++ | ++ |
| 15. | **1C15** | +++ | + | +++ | ++ | +++ |
| 16. | **1C16** | +++ | + | +++ | +++ | +++ |
| 17. | **1C17** | +++ | ++ | +++ | +++ | + |
| 18. | **1C18** | +++ | +++ | + | +++ | +++ |
| 19. | **1C19** | ++ | + | ++ | ++ | +++ |
| 20. | **1C20** | ++ | ++ | ++ | +++ | ++ |
| 21. | **Meriolin 1** | ++ | ++ | ++ | ++ | +++ |

**In vitro cell inhibition at 10µM; + indicates less than 50% of inhibition (+ < 50% of inhibition), ++ indicates greater than 50% of inhibition (++ > 50% of inhibition) and +++ indicates greater than 70% of inhibition (+++ >70% of inhibition).**

## Claims

1. A compound of general **Formula 1**, 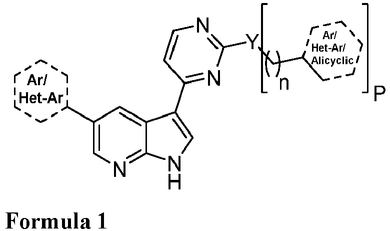
wherein, 'Y' is independently selected from any of NH, S, O, SO, SO₂;
'n' is independently selected from any of 0, 1, 2;
Pis 1;
**[Ar]** is independently selected from any of substituted or unsubstituted phenyl and substituted or unsubstituted aryl, attached through any available ring position, wherein substitution on phenyl or aryl, when present, is mono substitution by a substituent selected from any of F, Cl, Br, I, CN, NR₁R₂, CF₃, CHCF₂, CH₂F, OCF₃, OCH₂CF₃, OR₃, NO₂, NO, CHR₄R₅, alkyl chain from C1 to C14, COOR₆, CHO, COR₇, COCF₃, COCH₂CF₃, SR₈, SOR₉, SO₂R₁₀, SONR₁₁R₁₂ and SO₂NR₁₃R₁₄;
[Hetero-Ar] is independently selected from substituted or unsubstituted heterocycles selected from pyridyl, triazolyl, triazinyl, pyrimidinyl, pyridazinyl, oxazolyl, furanyl, benzothiophenyl benzofuranyl, thiophenyl, pyrrolyl, imidazoyl, thiazoyl, quinolinyl, isoquinolinyl, benzooxazolyl, benzothiazolyl, indolyl, benzotriazolyl, and benzoimidazolyl, attached through any available ring position, wherein substitution on a heterocycle, when present, is mono substitution by a substituent selected from any of F, Cl, Br, I, CN, NR₄R₅, CF₃, CHCF₂, CH₂F, OCF₃, OCH₂CF₃, OR₆, NO₂, NO, CHR₄R₅, alkyl chain from C1 to C14, COOR₆, CHO, COR₇, COCF₃, COCH₂CF₃, SR₈, SOR₉, SO₂R₁₀, SONR₁₁R₁₂, and SO₂NR₁₃R₁₄;
R₁, R₂, R₃, R₄, R₅, R₆, R₇, R₈, R₉, R₁₀, R₁₁, R₁₂, R₁₃, and R₁₄, are independently mono substituents selected from any of H, linear C1-C10 alkyl, branched C3-C10 alkyl, and unsubstituted phenyl; [Alicyclic] is independently selected from unsubstituted cycloalkanes selected from any of cyclopropane, cyclobutane, cyclopentane, cyclohexane, cycloheptane and cyclooctane, attached through any available ring position.

2. The compound of general **Formula 1** as claimed in claim 1, wherein the compounds of Formula 1 is selected from:
*N*-cyclopropyl-4-(5-(4-(trifluoromethyl)phenyl)-1*H*-pyrrolo[2,3-*b*]pyridin-3-yl)pyrimidin-2-amine,(compound **1A1**, Table 1)
*N*-cyclopropyl-4-(5-(4-fluorophenyl)-1*H*-pyrrolo[2,3-*b*]pyridin-3-yl)pyrimidin-2-amine (compound **1A2**, Table 1)
*N*-cyclopropyl-4-(5-(4-(trifluoromethoxy)phenyl)-1*H*-pyrrolo[2,3-*b*]pyridin-3-yl)pyrimidin-2-amine (compound **1A3**, Table 1)
N-cyclopropyl-4-(5-(3-(trifluoromethyl)phenyl)-1*H*-pyrrolo[2,3-*b*]pyridin-3-yl)pyrimidin-2-amine (compound **1A4,** Table 1)
*N*-cyclopropyl-4-(5-(3-fluorophenyl)-1*H*-pyrrolo[2,3-*b*]pyridin-3-yl)pyrimidin-2-amine (compound **1A5**, Table 1)
*N*-cyclopropyl-4-(5-(p-tolyl)-1*H*-pyrrolo[2,3-*b*]pyridin-3-yl)pyrimidin-2-amine (compound **1A6,** Table 1)
*N*-cyclopropyl-4-(5-(m-tolyl)-1*H*-pyrrolo[2,3-*b*]pyridin-3-yl)pyrimidin-2-amine (compound **1A7,** Table 1)
*N*-cyclopropyl-4-(5-(4-(methylthio)phenyl)-1*H*-pyrrolo[2,3-*b*]pyridin-3-yl)pyrimidin-2-amine (compound **1A8,** Table 1)
*N*-cyclopropyl-4-(5-(2-(methylthio)phenyl)-1*H*-pyrrolo[2,3-*b*]pyridin-3-yl)pyrimidin-2-amine (compound **1A9**, Table 1)
*N*-cyclopropyl-4-(5 -(2-ethylphenyl)-1*H*-pyrrolo[2,3-*b*]pyridin-3-yl)pyrimidin-2-amine (compound **1A10,** Table 1)
*N*-cyclopropyl-4-(5-(4-methoxyphenyl)-1*H*-pyrrolo[2,3-*b*]pyridin-3-yl)pyrimidin-2-amine (compound **1A11,** Table 1)
4-(5-(4-chlorophenyl)-1*H*-pyrrolo[2,3-*b*]pyridin-3-yl)-*N*-cyclopropylpyrimidin-2-amine (compound **1A12,** Table 1)
1-(4-(3-(2-(cyclopropylamino)pyrimidin-4-yl)-1*H*-pyrrolo[2,3-*b*]pyridin-5-yl)phenyl)ethan-1-one (compound **1A13,** Table 1)
*N*-cyclopropyl-4-(5-(naphthalen-1-yl)-1*H*-pyrrolo[2,3-*b*]pyridin-3-yl)pyrimidin-2-amine (compound **1A14,** Table 1)
*N-*cyclopropyl-4-(5-(pyridin-3-yl)-1*H*-pyrrolo[2,3-*b*]pyridin-3-yl)pyrimidin-2-amine (compound **1A15,** Table 1)
*N*-cyclopropyl-4-(5-(furan-3-yl)-1*H*-pyrrolo[2,3-*b*]pyridin-3-yl)pyrimidin-2-amine (compound **1A16,** Table 1)
*N*-cyclopropyl-4-(5-(5-methoxy-1*H*-indol-2-yl)-1*H*-pyrrolo[2,3-*b*]pyridin-3-yl)pyrimidin-2-amine (compound **1A17,** Table 1)
4-(5-(benzofuran-2-yl)-1*H*-pyrrolo[2,3-*b*]pyridin-3-yl)-*N*-cyclopropylpyrimidin-2-amine (compound **1A18,** Table 1)
4-(5-(1*H*-indol-2-yl)-1*H*-pyrrolo[2,3-*b*]pyridin-3-yl)-*N*-cyclopropylpyrimidin-2-amine (compound **1A19,** Table 1)
*N*-cyclopropyl-4-(5-(isoquinolin-1-yl)-1*H*-pyrrolo[2,3-*b*]pyridin-3-yl)pyrimidin-2-amine (compound **1A20,** Table 1)
*N*-cyclopentyl-4-(5-(4-(trifluoromethyl)phenyl)-1*H*-pyrrolo[2,3-*b*]pyridin-3-yl)pyrimidin-2-amine (compound **1A21,** Table 2)
*N*-cyclopentyl-4-(5-(4-fluorophenyl)-1*H*-pyrrolo[2,3-*b*]pyridin-3-yl)pyrimidin-2-amine (compound **1A22,** Table 2)
*N*-cyclopentyl-4-(5-(4-(trifluoromethoxy)phenyl)-1*H*-pyrrolo[2,3-*b*]pyridin-3-yl)pyrimidin-2-amine (compound **1A23,** Table 2)
*N*-cyclopentyl-4-(5-(3-(trifluoromethyl)phenyl)-1*H*-pyrrolo[2,3-*b*]pyridin-3-yl)pyrimidin-2-amine (compound **1A24,** Table 2)
*N*-cyclopentyl-4-(5-(3-fluorophenyl)-1*H*-pyrrolo[2,3-*b*]pyridin-3-yl)pyrimidin-2-amine (compound **1A25,** Table 2)
*N*-cyclopentyl-4-(5-(p-tolyl)-1*H*-pyrrolo[2,3-*b*]pyridin-3-yl)pyrimidin-2-amine (compound **1A26,** Table 2)
*N*-cyclopentyl-4-(5-(m-tolyl)-1*H*-pyrrolo[2,3-*b*]pyridin-3-yl)pyrimidin-2-amine (compound **1A27,** Table 2)
*N*-cyclopentyl-4-(5-(4-(methylthio)phenyl)-1*H*-pyrrolo[2,3-*b*]pyridin-3-yl)pyrimidin-2-amine (compound **1A28,** Table 2)
*N*-cyclopentyl-4-(5-(2-(methylthio)phenyl)-1*H*-pyrrolo[2,3-*b*]pyridin-3-yl)pyrimidin-2-amine (compound **1A29,** Table 2)
*N*-cyclopentyl-4-(5-(2-ethylphenyl)-1*H*-pyrrolo[2,3-*b*]pyridin-3-yl)pyrimidin-2-amine (compound **1A30,** Table 2)
*N*-cyclopentyl-4-(5-(4-methoxyphenyl)-1*H*-pyrrolo[2,3-b]pyridin-3-yl)pyrimidin-2-amine (compound **1A31,** Table 2)
4-(5-(4-chlorophenyl)-1*H*-pyrrolo[2,3-*b*]pyridin-3-yl)-*N*-cyclopentylpyrimidin-2-amine (compound **1A32,** Table 2)
4-(3-(2-(cyclopentylamino)pyrimidin-4-yl)-1*H*-pyrrolo[2,3-*b*]pyridin-5-yl)benzaldehyde (compound **1A33,** Table 2)
*N*-cyclopentyl-4-(5-(naphthalen-1-yl)-1*H*-pyrrolo[2,3-*b*]pyridin-3-yl)pyrimidin-2-amine (compound **1A34,** Table 2)
*N*-cyclopentyl-4-(5-(pyridin-3-yl)-1*H*-pyrrolo[2,3-*b*]pyridin-3-yl)pyrimidin-2-amine (compound **1A35,** Table 2)
*N*-cyclopentyl-4-(5-(furan-3-yl)-1*H*-pyrrolo[2,3-*b*]pyridin-3-yl)pyrimidin-2-amine (compound **1A36,** Table 2)
*N*-cyclopentyl-4-(5-(5-methoxy-1*H*-indol-2-yl)-1*H*-pyrrolo[2,3-*b*]pyridin-3-yl)pyrimidin-2-amine (compound **1A37**, Table 2)
4-(5-(benzo[*b*]thiophen-2-yl)-1*H*-pyrrolo[2,3-*b*]pyridin-3-yl)-*N*-cyclopentylpyrimidin-2-amine (compound **1A38,** Table 2)
4-(5-(1*H*-indol-2-yl)-1*H*-pyrrolo[2,3-*b*]pyridin-3-yl)-*N*-cyclopentylpyrimidin-2-amine (compound **1A39,** Table 2)
*N*-cyclopentyl-4-(5-(isoquinolin-1-yl)-1*H*-pyrrolo[2,3-*b*]pyridin-3-yl)pyrimidin-2-amine (compound **1A40**, Table 2)
*N*-cycloheptyl-4-(5-(4-(trifluoromethyl)phenyl)-1*H*-pyrrolo[2,3-*b*]pyridin-3-yl)pyrimidin-2-amine (compound **1A41,** Table 3)
*N*-cycloheptyl-4-(5-(4-fluorophenyl)-1*H*-pyrrolo[2,3-*b*]pyridin-3-yl)pyrimidin-2-amine(compound **1A42,** Table 3)
*N*-cycloheptyl-4-(5-(4-(trifluoromethoxy)phenyl)-1*H*-pyrrolo[2,3-*b*]pyridin-3-yl)pyrimidin-2-amine (compound **1A43,** Table 3)
*N*-cycloheptyl-4-(5-(3-(trifluoromethyl)phenyl)-1*H*-pyrrolo[2,3-*b*]pyridin-3-yl)pyrimidin-2-amine (compound **1A44,** Table 3)
*N*-cycloheptyl-4-(5-(3-fluorophenyl)-1*H*-pyrrolo[2,3-*b*]pyridin-3-yl)pyrimidin-2-amine (compound **1A45,** Table 3)
*N*-cycloheptyl-4-(5-(p-tolyl)-1*H*-pyrrolo[2,3-*b*]pyridin-3-yl)pyrimidin-2-amine (compound **1A46**, Table 3)
*N*-cycloheptyl-4-(5-(m-tolyl)-1*H*-pyrrolo[2,3-*b*]pyridin-3-yl)pyrimidin-2-amine (compound **1A47**, Table 3)
*N*-cycloheptyl-4-(5-(4-(methylthio)phenyl)-1*H*-pyrrolo[2,3-*b*]pyridin-3-yl)pyrimidin-2-amine (compound **1A48,** Table 3)
*N*-cycloheptyl-4-(5-(2-(methylthio)phenyl)-1*H*-pyrrolo[2,3-*b*]pyridin-3-yl)pyrimidin-2-amine (compound **1A49,** Table 3)
*N*-cycloheptyl-4-(5-(2-ethylphenyl)-1*H*-pyrrolo[2,3-*b*]pyridin-3-yl)pyrimidin-2-amine (compound **1A50,** Table 3)
*N*-cycloheptyl-4-(5-(4-methoxyphenyl)-1*H*-pyrrolo[2,3-*b*]pyridin-3-yl)pyrimidin-2-amine (compound **1A51,** Table 3)
4-(5-(4-chlorophenyl)-1*H*-pyrrolo[2,3-*b*]pyridin-3-yl)-*N*-cycloheptylpyrimidin-2-amine (compound **1A52,** Table 3)
4-(3-(2-(cycloheptylamino)pyrimidin-4-yl)-1*H*-pyrrolo[2,3-*b*]pyridin-5-yl)benzaldehyde (compound **1A53,** Table 3)
*N*-cycloheptyl-4-(5-(naphthalen-1-yl)-1*H*-pyrrolo[2,3-*b*]pyridin-3-yl)pyrimidin-2-amine (compound **1A54,** Table 3)
*N*-cycloheptyl-4-(5-(pyridin-3-yl)-1*H*-pyrrolo[2,3-*b*]pyridin-3-yl)pyrimidin-2-amine (compound **1A55,** Table 3)
*N*-cycloheptyl-4-(5-(furan-3-yl)-1*H*-pyrrolo[2,3-*b*]pyridin-3-yl)pyrimidin-2-amine (compound **1A56,** Table 3)
*N*-cycloheptyl-4-(5-(5-methoxy-1*H*-indol-2-yl)-1*H*-pyrrolo[2,3-*b*]pyridin-3-yl)pyrimidin-2-amine (compound **1A57,** Table 3)
4-(5-(benzo[*b*]thiophen-2-yl)-1*H*-pyrrolo[2,3-*b*]pyridin-3-yl)-*N*-cycloheptylpyrimidin-2-amine (compound **1A58,** Table 3)
4-(5-(1*H*-indol-2-yl)-1*H*-pyrrolo[2,3-*b*]pyridin-3-yl)-*N*-cycloheptylpyrimidin-2-amine (compound **1A59,** Table 3)
*N*-cycloheptyl-4-(5-(isoquinolin-1-yl)-1*H-*pyrrolo[2,3-*b*]pyridin-3-yl)pyrimidin-2-amine (compound **1A60**, Table 3)
*N*-phenethyl-4-(5-(4-(trifluoromethyl)phenyl)-1*H*-pyrrolo[2,3-*b*]pyridin-3-yl)pyrimidin-2-amine (compound **1B1**, Table 4)
4-(5-(4-fluorophenyl)-1*H*-pyrrolo[2,3-*b*]pyridin-3-yl)-*N*-phenethylpyrimidin-2-amine (compound **1B2,** Table 4)
*N*-phenethyl-4-(5-(4-(trifluoromethoxy)phenyl)-1*H*-pyrrolo[2,3-*b*]pyridin-3-yl)pyrimidin-2-amine (compound **1B3,** Table 4)
*N*-phenethyl-4-(5-(3-(trifluoromethyl)phenyl)-1*H*-pyrrolo[2,3-*b*]pyridin-3-yl)pyrimidin-2-amine (compound **1B4,** Table 4)
4-(5 -(3 -fluorophenyl)-1*H*-pyrrolo[2,3-*b*]pyridin-3-yl)-*N*-phenethylpyrimidin-2-amine (compound **1B5,** Table 4)
*N*-phenethyl-4-(5-(p-tolyl)-1*H*-pyrrolo[2,3-*b*]pyridin-3-yl)pyrimidin-2-amine (compound **1B6,** Table 4)
*N*-phenethyl-4-(5-(m-tolyl)-1*H*-pyrrolo[2,3-*b*]pyridin-3-yl)pyrimidin-2-amine (compound **1B7,** Table 4)
4-(5-(4-(methylthio)phenyl)-1*H*-pyrrolo[2,3-*b*]pyridin-3-yl)-*N*-phenethylpyrimidin-2-amine (compound **1B8**, Table 4)
4-(5-(2-(methylthio)phenyl)-1*H*-pyrrolo[2,3-*b*]pyridin-3-yl)-*N*-phenethylpyrimidin-2-amine (compound **1B9,** Table 4)
4-(5-(2-ethylphenyl)-1*H*-pyrrolo[2,3-*b*]pyridin-3-yl)-*N*-phenethylpyrimidin-2-amine (compound **1B10,** Table 4)
4-(5-(4-methoxyphenyl)-1*H*-pyrrolo[2,3-*b*]pyridin-3-yl)-*N*-phenethylpyrimidin-2-amine (compound **1B11,** Table 4)
4-(5-(4-chlorophenyl)-1*H*-pyrrolo[2,3-*b*]pyridin-3-yl)-*N*-phenethylpyrimidin-2-amine (compound **1B12,** Table 4)
4-(3-(2-(phenethylamino)pyrimidin-4-yl)-1*H*-pyrrolo[2,3-*b*]pyridin-5-yl)benzaldehyde (compound **1B13,** Table 4)
4-(5-(naphthalen-1-yl)-1*H*-pyrrolo[2,3-*b*]pyridin-3-yl)-*N*-phenethylpyrimidin-2-amine (compound **1B14,** Table 4)
*N*-phenethyl-4-(5-(pyridin-3-yl)-1*H*-pyrrolo[2,3-*b*]pyridin-3-yl)pyrimidin-2-amine (compound **1B15,** Table 4)
4-(5-(furan-3-yl)-1*H*-pyrrolo[2,3-*b*]pyridin-3-yl)-*N*-phenethylpyrimidin-2-amine (compound **1B16,** Table 4)
4-(5-(5-methoxy-1*H*-indol-2-yl)-1*H*-pyrrolo[2,3-*b*]pyridin-3-yl)-*N*-phenethylpyrimidin-2-amine (compound **1B17,** Table 4)
4-(5-(benzo[*b*]thiophen-2-yl)-1*H*-pyrrolo[2,3-*b*]pyridin-3-yl)-*N*-phenethylpyrimidin-2-amine (compound **1B18,** Table 4)
4-(5-(1*H*-indol-2-yl)-1*H*-pyrrolo[2,3-*b*]pyridin-3-yl)-*N*-phenethylpyrimidin-2-amine (compound **1B19,** Table 4)
4-(5-(isoquinolin-1-yl)-1*H*-pyrrolo[2,3-*b*]pyridin-3-yl)-*N*-phenethylpyrimidin-2-amine (compound **1B20,** Table 4)

3. A compound of general **Formula 1** as claimed in claim 1, for use in treating cancer.

4. The compound for use of claim 3, wherein said compound inhibits CDK2 and CDK9.

5. The compound of general **Formula 1** as claimed in claim 1, wherein said compound inhibits an *in vitro* cancer cell line activity.

6. A process for the preparation of the compound of general **Formula 1** as claimed in claim 1, the process comprising;
reacting intermediate compound of Formula 13 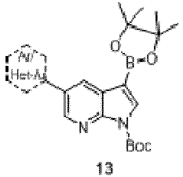 wherein,
**[Ar]** is independently selected from any of substituted or unsubstituted phenyl and substituted or unsubstituted aryl, attached through any available ring position, wherein substitution on phenyl or aryl, when present, is mono substitution by a substituent selected from any of F, Cl, Br, I, CN, NR₁R₂, CF₃, CHCF₂, CH₂F, OCF₃, OCH₂CF₃, OR₃, NOz, NO, CHR₄R₅, alkyl chain from C1 to C14, COOR₆, CHO, COR₇, COCF₃, COCH₂CF₃, SR₈, SOR₉, SO₂R₁₀, SONR₁₁R₁₂ and SO₂NR₁₃R₁₄;
**[Hetero-Ar]** is independently selected from substituted or unsubstituted heterocycles selected from pyridyl, triazolyl, triazinyl, pyrimidinyl, pyridazinyl, oxazolyl, furanyl, benzothiophenyl benzofuranyl, thiophenyl, pyrrolyl, imidazoyl, thiazoyl, quinolinyl, isoquinolinyl, benzooxazolyl, benzothiazolyl, indolyl, benzotriazolyl, and benzoimidazolyl, attached through any available ring position, wherein substitution on a heterocycle, when present, ismono substitution by a substituent selected from any of F, Cl, Br, I, CN, NR₄R₅, CF₃, CHCF₂, CH₂F, OCF₃, OCH₂CF₃, OR₆, NO₂, NO, CHR₄R₅, alkyl chain from C1 to C14, COOR₆, CHO, COR₇, COCF₃, COCH₂CF₃, SR₈, SOR₉, SO₂R₁₀, SONR₁₁R₁₂, and SO₂NR₁₃R₁₄;
R₁, R₂, R₃, R₄, R₅, R₆, R₇, R₈, R₉, R₁₀, R₁₁, R₁₂, R₁₃, and R₁₄, are independently mono substituents selected from any of H, linear C1-C10) alkyl, branched C3-C10 alkyl, and unsubstituted phenyl,
with a substituted chloropyrimidine intermediate compound in the presence of catalyst in a solvent followed by addition of the base at a temperature in the range of 80 °C to 100 °C for a period in the range of 24 to 49 hrs to obtain a compound of general **Formula 1.**

7. The process as claimed in claim 6, wherein the substituted chloropyrimidine intermediate compound is selected from the group consisting of 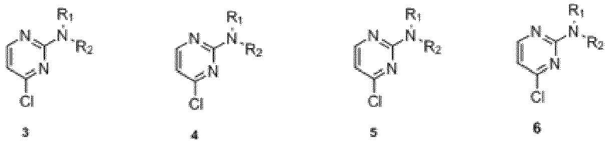 wherein,
R1=H; R2=cyclopropyl, cyclopentyl, cycloheptyl or 2-phenylethyl

8. The process as claimed in claim 6, wherein the catalyst used is tetrakis(triphenylphosphino) palladium(0).

9. The process as claimed in claim 6, wherein the solvent is selected from the group consisting of dry dioxane and methanol.

10. The process as claimed in claim 6, wherein the base is selected from the group consisting cesium carbonate and triethyl amine.

## Patentansprüche

1. Verbindung der allgemeinen Formel 1, 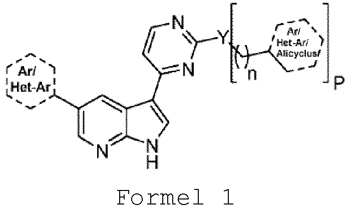
wobei "Y" unabhängig aus einem von NH, S, O, SO, SO₂ ausgewählt ist;
"n" unabhängig aus einem von 0, 1 oder 2 ausgewählt ist;
P für 1 steht;
[Ar] unabhängig aus einem von substituiertem oder unsubstituiertem Phenyl und substituiertem oder unsubstituiertem Aryl, das über eine beliebige verfügbare Ringposition gebunden ist, ausgewählt ist, wobei es sich bei der Substitution an Phenyl oder Aryl, wenn vorhanden, um Monosubstitution durch einen Substituenten, der aus einem von F, Cl, Br, I, CN, NR₁R₂, CF₃, CHCF₂, CH₂F, OCF₃, OCH₂CF₃, OR₃, NO₂, NO, CHR₄R₅, einer C1- bis C14-Alkylkette, COOR₆, CHO, COR₇, COCF₃, COCH₂CF₃, SR₈, SOR₉, SO₂R₁₀, SONR₁₁R₁₂ und SO₂NR₁₃R₁₄ ausgewählt ist, handelt;
[Hetero-Ar] unabhängig aus substituierten oder unsubstituierten Heterocyclen, die aus Pyridyl, Triazolyl, Triazinyl, Pyrimidinyl, Pyridazinyl, Oxazolyl, Furanyl, Benzothiophenyl, Benzofuranyl, Thiophenyl, Pyrrolyl, Imidazolyl, Thiazolyl, Chinolinyl, Isochinolinyl, Benzooxazolyl, Benzothiazolyl, Indolyl, Benzotriazolyl und Benzoimidazolyl ausgewählt sind und über eine beliebige verfügbare Ringposition gebunden sind, ausgewählt ist, wobei es sich bei der Substitution an einem Heterocyclus, wenn vorhanden, um Monosubstitution durch einen Substituenten, der aus einem von F, Cl, Br, I, CN, NR₄R₅, CF₃, CHCF₂, CH₂F, OCF₃, OCH₂CF₃, OR₆, NO₂, NO, CHR₄R₅, einer C1- bis C14-Alkylkette, COOR₆, CHO, COR₇, COCF₃, COCH₂CF₃, SR₈, SOR₉, SO₂R₁₀, SONR₁₁R₁₂ und SO₂NR₁₃R₁₄ ausgewählt ist, handelt;
R₁, R₂, R₃, R₆, R₇, R₈, R₉, R₁₀, R₁₁, R₁₂, R₁₃ und R₁₄ unabhängig für Monosubstituenten, die aus einem von H, linearem C1-C10-Alkyl, verzweigtem C3-C10-Alkyl und unsubstituiertem Phenyl ausgewählt sind, stehen; [Alicyclus] unabhängig aus unsubstituierten Cycloalkanen, die aus einem von Cyclopropan, Cyclobutan, Cyclopentan, Cyclohexan, Cycloheptan und Cyclooctan ausgewählt sind und über eine beliebige verfügbare Ringposition gebunden sind, ausgewählt ist.

2. Verbindung der allgemeinen Formel 1 nach Anspruch 1, wobei die Verbindungen der Formel 1 ausgewählt sind aus:
*N*-Cyclopropyl-4-(5-(4-(trifluormethyl)phenyl)-1*H-*pyrrolo [2,3-*b*]pyridin-3-yl)pyrimidin-2-amin, (Verbindung 1A1, Tabelle 1)
*N*-Cyclopropyl-4-(5-(4-fluorphenyl)-1*H*-pyrrolo[2,3-*b*]pyridin-3-yl)pyrimidin-2-amin (Verbindung 1A2, Tabelle 1)
*N*-Cyclopropyl-4-(5-(4-(trifluormethoxy)phenyl)-1*H-*pyrrolo[2,3-*b*]pyridin-3-yl)pyrimidin-2-amin (Verbindung 1A3, Tabelle 1)
*N*-Cyclopropyl-4-(5-(3-(trifluormethyl)phenyl)-1*H-*pyrrolo[2,3-*b*]pyridin-3-yl)pyrimidin-2-amin (Verbindung 1A4, Tabelle 1)
*N*-Cyclopropyl-4-(5-(3-fluorphenyl)-1*H*-pyrrolo[2,3-*b*]pyridin-3-yl)pyrimidin-2-amin(Verbindung 1A5, Tabelle 1)
*N*-Cyclopropyl-4-(5-(p-tolyl)-1*H*-pyrrolo[2,3-*b*]pyridin-3-yl)pyrimidin-2-amin (Verbindung 1A6, Tabelle 1)
*N*-Cyclopropyl-4-(5-(m-tolyl)-1*H*-pyrrolo[2,3-b]pyridin-3-yl)pyrimidin-2-amin (Verbindung 1A7, Tabelle 1)
*N*-Cyclopropyl-4-(5-(4-(methylthio)phenyl)-1*H-*pyrrolo[2,3-*b*]pyridin-3-yl)pyrimidin-2-amin (Verbindung 1A8, Tabelle 1)
*N*-(Cyclopropyl-4-(5-(2-(methylthio)phenyl)-1*H-*pyrrolo[2,3-*b*]pyridin-3-yl)pyrimidin-2-amin (Verbindung 1A9, Tabelle 1)
*N*-Cyclopropyl-4-(5-(2-ethylphenyl)-1*H*-pyrrolo[2,3-*b*]pyridin-3-yl)pyrimidin-2-amin (Verbindung 1A10, Tabelle 1)
*N*-Cyclopropyl-4-(5-(4-methoxyphenyl)-1*H*-pyrrolo[2,3-*b*]pyridin-3-yl)pyrimidin-2-amin (Verbindung 1A11, Tabelle 1)
4-(5-(4-Chlorphenyl)-1*H*-pyrrolo[2,3-*b*]pyridin-3-yl)-*N-*cyclopropylpyrimidin-2-amin (Verbindung 1A12, Tabelle 1)
1-(4-(3-(2-(Cyclopropylamino)pyrimidin-4-yl)-1*H-*pyrrolo[2,3-*b*]pyridin-5-yl)phenyl)ethan-1-on (Verbindung 1A13, Tabelle 1)
*N*-Cyclopropyl-4-(5-(naphthalin-1-yl)-1*H*-pyrrolo[2,3-*b*]pyridin-3-yl)pyrimidin-2-amin (Verbindung 1A14, Tabelle 1)
*N*-Cyclopropyl-4-(5-(pyridin-3-yl-1*H*-pyrrolo[2,3-*b*]pyridin-3-yl)pyrimidin-2-amin (Verbindung 1A15, Tabelle 1)
*N*-CyclopropyI-4-(5-(furan-3-yl)-1*H*-pyrrolo[2,3-*b*]pyridin-3-yl)pyrimidin-2-amin (Verbindung 1A16, Tabelle 1)
*N*-Cyclopropyl-4-(5-(5-methoxy-1*H*-indol-2-yl)-1*H-*pyrrolo[2,3-*b*]pyridin-3-yl)pyrimidin-2-amin (Verbindung 1A17, Tabelle 1)
4-(5-(Benzofuran-2-yl)-1*H*-pyrrolo[2,3-*b*]pyridin-3-yl)-N-cyclopropylpyrimidin-2-amin (Verbindung 1A18, Tabelle 1)
4-(5-(1H-indol-2-yl)-1*H*-pyrrolo[2,3-*b*]pyridin-3-yl)-*N-*cyclopropylpyrimidin-2-amin (Verbindung 1A19, Tabelle 1)
N-Cyclopropyl-4-(5-(isochinolin-1-yl)4H-pyrrolo[2,3-*b*]pyridin-3-yl)pyrimidin-2-amin (Verbindung 1A20, Tabelle 1)
*N*-Cyclopentyl-4-(5-(4-(trifluormethyl)phenyl)-1*H-*pyrrolo[2,3-*b*]pyridin-3-yl)pyrimidin-2-amin (Verbindung 1A21, Tabelle 2)
*N*-Cyclopentyl-4-(5-(4-fluorphenyl)-1*H*-pyrrolo[2,3-*b*]pyridin-3-yl)pyrimidin-2-amin (Verbindung 1A22, Tabelle 2)
*N*-Cyclopentyl-4-(5-(4-(trifluormethoxy)phenyl)-1*H-*pyrrolo[2,3-*b*]pyridin-3-yl)pyrimidin-2-amin (Verbindung 1A23, Tabelle 2)
*N*-Cyclopentyl-4-(5-(3-(trifluormethyl)phenyl)-1*H-*pyrrolo[2,3-*b*]pyridin-3-yl)pyrimidin-2-amin (Verbindung 1A24, Tabelle 2)
*N*-Cyclopentyl-4-(5-(3-fluorphenyl)-1*H*-pyrrolo[2,3-*b*]pyridin-3-yl)pyrimidin-2-amin (Verbindung 1A25, Tabelle 2)
*N*-Cyclopentyl-4-(5-(p-tolyl)-1*H*-pyrrolo[2,3-*b*]pyridin-3-yl)pyrimidin-2-amin (Verbindung 1A26, Tabelle 2)
*N*-Cyclopentyl-4-(5-(m-tolyl)-1*H*-pyrrolo[2,3-*b*]pyridin-3-yl)pyrimidin-2-amin (Verbindung 1A27, Tabelle 2)
*N*-Cyclopentyl-4-(5-(4-(methylthio)phenyl)-1*H-*pyrrolo[2,3-*b*]pyridin-3-yl)pyrimidin-2-amin (Verbindung 1A28, Tabelle 2)
*N*-Cyclopentyl-4-5-(2-(methylthio)phenyl)-1*H-*pyrrolo[2,3-*b*]pyridin-3-yl)pyrimidin-2-amin (Verbindung 1A29, Tabelle 2)
*N*-Cyclopentyl-4-(5-(2-ethylphenyl)-1*H*-pyrrolo[2,3-*b*]pyridin-3-yl)pyrimidin-2-amin (Verbindung 1A30, Tabelle 2)
*N*-Cyclopentyl-4-(5-(4-methoxyphenyl)-1*H*-pyrrolo[2,3-*b*]pyridin-3-yl)pyrimidin-2-amin (Verbindung 1A31, Tabelle 2)
4-(5-(4-Chlorphenyl)-1*H*-pyrrolo[2,3-*b*]pyridin-3-yl)-N-cyclopentylpyrimidin-2-amin (Verbindung 1A32, Tabelle 2)
4-(3-(2-(Cyclopentylamino)pyrimidin-4-yl)-1*H-*pyrrolo[2,3-*b*]pyridin-5-yl)benzaldehyd (Verbindung 1A33, Tabelle 2)
*N*-Cyclopentyl-4-(5-(naphthalin-1-yl)-1*H*-pyrrolo[2,3-*b*]pyridin-3-yl)pyrimidin-2-amin (Verbindung 1A34, Tabelle 2)
*N-*Cyclopentyl-4-(5-(pyridin-3-yl)-1*H*-pyrrolo[2,3-*b*]pyridin-3-yl)pyrimidin-2-amin (Verbindung 1A35, Tabelle 2)
*N*-Cyclopentyl-4-(5-(furan-3-yl)-1*H*-pyrrolo[2,3-*b*]pyridin-3-yl)pyrimidin-2-amin (Verbindung 1A36, Tabelle 2)
*N*-Cyclopentyl-4-(5-(5-methoxy-1*H*-indol-2-yl)-1*H-*pyrrolo[2,3-*b*]pyridin-3-yl)pyrimidin-2-amin (Verbindung 1A37, Tabelle 2)
4-(5-(Benzo[b]thiophen-2-yl)-1*H*-pyrrolo[2,3-*b*]pyridin-3-yl)*-N*-cyclopentylpyrimidin-2-amin (Verbindung 1A38, Tabelle 2)
4-(5-(1*H*-Indol-2-yl)-1*H*-pyrrolo[2,3-*b*]pyridin-3-yl)-*N-*cyclopentylpyrimidin-2-amin (Verbindung 1A39, Tabelle 2)
*N*-Cyclopentyl-4-(5-(isochinolin-1-yl)-1*H*-pyrrolo[2,3-*b*]pyridin-3-yl)pyrimidin-2-amin (Verbindung 1A40, Tabelle 2)
*N*-Cycloheptyl-4-(5-(4-(trifluormethyl)phenyl)-1*H-*pyrrolo[2,3-*b*]pyridin-3-yl)pyrimidin-2-amin (Verbindung 1A41, Tabelle 3)
*N*-Cycloheptyl-4-(5-(4-fluorphenyl)-1*H*-pyrrolo[2,3-*b*]pyridin-3-yl)pyrimidin-2-amin (Verbindung 1A42, Tabelle 3)
*N*-Cycloheptyl-4-(5-(4-(trifluormethoxy)phenyl)-1*H-*pyrrolo[2,3-*b*]pyridin-3-yl)pyrimidin-2-amin (Verbindung 1A43, Tabelle 3)
*N*-Cycloheptyl-4-(5-(3-(trifluormethyl)phenyl)-1*H-*pyrrolo[2,3-*b*]pyridin-3-yl)pyrimidin-2-amin (Verbindung 1A44, Tabelle 3)
*N*-Cycloheptyl-4-(5-(3-fluorphenyl)-1*H*-pyrrolo[2,3-*b*]pyridin-3-yl)pyrimidin-2-amin (Verbindung 1A45, Tabelle 3)
*N*-Cycloheptyl-4-(5-(p-tolyl)-1*H*-pyrrolo[2,3-*b*]pyridin-3-yl)pyrimidin-2-amin (Verbindung 1A46, Tabelle 3)
*N*-Cycloheptyl-4-(5-(m-tolyl)-1*H*-pyrrolo[2,3-*b*]pyridin-3-yl)pyrimidin-2-amin (Verbindung 1A47, Tabelle 3)
*N*-Cycloheptyl-4-(5-(4-(methylthio)phenyl)-1*H-*pyrrolo[2,3-*b*]pyridin-3-yl)pyrimidin-2-amin (Verbindung 1A48, Tabelle 3)
*N*-Cycloheptyl-4-(5-(2-(methylthio)phenyl)-1*H-*pyrrolo[2,3-*b*]pyridin-3-yl)pyrimidin-2-amin (Verbindung 1A49, Tabelle 3)
*N*-Cycloheptyl-4-(5-(2-ethylphenyl)-1*H*-pyrrolo[2,3-*b*]pyridin-3-yl)pyrimidin-2-amin (Verbindung 1A50, Tabelle 3)
*N*-Cycloheptyl-4-(5-(4-methoxyphenyl)-1*H*-pyrrolo[2,3-*b*]pyridin-3-yl)pyrimidin-2-amin (Verbindung 1A51, Tabelle 3)
4-(5-(4-Chlorphenyl)-1*H*-pyrrolo[2,3-*b*]pyridin-3-yl)-*N-*cycloheptylpyrimidin-2-amin (Verbindung 1A52, Tabelle 3)
4-(3-(2-(Cycloheptylamino)pyrimidin-4-yl)-1*H-*pyrrolo[2,3-b]pyridin-5-yl)benzaldehyd (Verbindung 1A53, Tabelle 3)
N-Cycloheptyl-4-(5-(naphthalin-1-yl)-1H-pyrrolo[2,3-*b*]pyridin-3-yl)pyrimidin-2-amin (Verbindung 1A54, Tabelle 3)
*N*-Cycloheptyl-4-(5-(pyridin-3-yl)-1*H*-pyrrolo[2,3-*b*]pyridin-3-yl)pyrimidin-2-amin (Verbindung 1A55, Tabelle 3)
*N*-Cycloheptyl-4-(5-(furan-3-yl)-1*H*-pyrrolo[2,3-*b*]pyridin-3-yl)pyrimidin-2-amin (Verbindung 1A56, Tabelle 3)
*N*-Cycloheptyl-4-(5-(5-methoxy-1*H*-indol-2-yl)-1*H-*pyrrolo[2,3-b]pyridin-3-yl)-2-amin (Verbindung 1A57, Tabelle 3)
4-(5-(Benzo[b]thiophen-2-yl)-1*H*-pyrrolo[2,3-*b*]pyridin-3-yl)-*N*-cycloheptylpyrimidin-2-amin (Verbindung 1A58, Tabelle 3)
4-(5-(1*H*-Indol-2-yl)-1*H*-pyrrolo[2,3-*b*]pyridin-3-yl)-*N-*cycloheptylpyrimidin-2-amin (Verbindung 1A59, Tabelle 3)
*N*-Cycloheptyl-4-(5-(isochinolin-1-yl)-1*H*-pyrrolo[2,3-*b*]pyridin-3-yl)pyrimidin-2-amin (Verbindung 1A60, Tabelle 3)
*N*-Phenethyl-4-(5-(4-(trifluormethyl)phenyl)-1*H-*pyrrolo[2,3-*b*]pyridin-3-yl)pyrimidin-2-amin (Verbindung 1B1, Tabelle 4)
4-(5-(4-Fluorphenyl)-1*H*-pyrrolo[2,3-*b*]pyridin-3-yl)-*N-*phenethylpyrimidin-2-amin (Verbindung 1B2, Tabelle 4)
*N*-Phenethyl-4-(5-(4-(trifluormethoxy)phenyl)-1*H-*pyrrolo[2,3-*b*]pyridin-3-yl)pyrimidin-2-amin (Verbindung 1B3, Tabelle 4)
*N*-Phenethyl-4-(5-(3-(trifluormethyl)phenyl)-1*H-*pyrrolo[2,3-*b*]pyridin-3-yl)pyrimidin-2-amin (Verbindung 1B4, Tabelle 4)
4-(5-(3-Fluorphenyl)-1*H*-pyrrolo[2,3-*b*]pyridin-3-yl)-*N-*phenethylpyrimidin-2-amin (Verbindung 1B5, Tabelle 4)
*N*-Phenethyl-4-(5-(p-tolyl)-1*H*-pyrrolo[2,3-*b*]pyridin-3-yl)pyrimidin-2-amin (Verbindung 1B6, Tabelle 4)
*N*-Phenethyl-4-(5-(m-tolyl)-1*H*-pyrrolo[2,3-*b*]pyridin-3-yl)pyrimidin-2-amin (Verbindung 1B7, Tabelle 4)
4-(5-(4-(Methylthio)phenyl)-1*H*-pyrrol[2,3-*b*]pyridin-3-yl)-N-phenethylpyrimidin-2-amin Verbindung 1B8**,** Tabelle 4)
4-(5-(2-(Methylthio)phenyl)-1*H*-pyrrolo[2,3-*b*]pyridin-3-yl)-*N*-phenethylpyrimidin-2-amin (Verbindung 1B9, Tabelle 4)
4-(5-(2-Ethylphenyl)-1*H*-pyrrolo[2,3-*b*]pyridin-3-yl-*N-*phenethylpyrimidin-2-amin (Verbindung 1B10, Tabelle 4)
4-(5-(4-Methoxyphenyl)-1*H*-pyrrolo[2,3-*b*]pyridin-3-yl)-*N*-phenethylpyrimidin-2-amin (Verbindung 1B11, Tabelle 4)
4-(5-(4-Chlorphenyl)-1*H*-pyrrolo[2,3-*b*]pyridin-3-yl)-*N-*phenethylpyrimidin-2-amin (Verbindung 1B12, Tabelle 4)
4-(3-(2-(Phenethylamino)pyrimidin-4-yl)-1*H*-pyrrolo[2,3-*b*]pyridin-5-yl)benzaldehyd (Verbindung 1B13, Tabelle 4)
4-(5-(Naphthalin-1-yl)-1*H*-pyrrolo[2,3-*b*]pyridin-3-yl)-*N*-phenethylpyrimidin-2-amin (Verbindung 1B14, Tabelle 4)
*N*-Phenethyl-4-(5-(pyridin-3-yl)-1*H*-pyrrolo[2,3-*b*]pyridin-3-yl)pyrimidin-2-amin (Verbindung 1B15, Tabelle 4)
4-(5-(Furan-3-yl)-1*H*-pyrrolo[2,3-*b*]pyridin-3-yl)-*N-*phenethylpyrimidin-2-amin (Verbindung 1B16, Tabelle 4)
4-(5-(5-Methoxy-1*H*-indol-2-yl)-1*H*-pyrrolo[2,3-*b*]pyridin-3-yl)-*N*-phenethylpyrimidin-2-amin (Verbindung 1B17, Tabelle 4)
4-(5-(Benzo[b]thiophen-2-yl)-1*H*-pyrrolo[2,3-*b*]pyridin-3-yl)-*N*-phenethylpyrimidin-2-amin (Verbindung 1B18, Tabelle 4)
4-(5-(1H-Indol-2-yl)-1*H*-pyrrolo[2,3-*b*]pyridin-3-yl)-*N-*phenethylpyrimidin-2-amin (Verbindung 1B19, Tabelle 4)
4-(5-(Isochinolin-1-yl)-1*H*-pyrrolo[2,3-*b*]pyridin-3-yl)-*N*-phenethylpyrimidin-2-amin (Verbindung 1B20, Tabelle 4).

3. Verbindung der allgemeinen Formel 1 nach Anspruch 1 zur Verwendung bei der Behandlung von Krebs.

4. Verbindung zur Verwendung nach Anspruch 3, wobei die Verbindung CDK2 und CDK9 inhibiert.

5. Verbindung der allgemeinen Formel 1 nach Anspruch 1, wobei die Verbindung eine in-vitro-Krebszelllinienaktivität inhibiert.

6. Verfahren zur Herstellung der Verbindung der allgemeinen Formel 1 nach Anspruch 1, wobei das Verfahren Folgendes umfasst:
Umsetzen der Zwischenverbindung der Formel 13 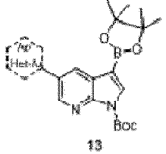 wobei
[Ar] unabhängig aus einem von substituiertem oder unsubstituiertem Phenyl und substituiertem oder unsubstituiertem Aryl, das über eine beliebige verfügbare Ringposition gebunden ist, ausgewählt ist, wobei es sich bei der Substitution an Phenyl oder Aryl, wenn vorhanden, um Monosubstitution durch einen Substituenten, der aus einem von F, Cl, Br, I, CN, NR₁R₂, CF₃, CHCF₂, CH₂F, OCF₃, OCH₂CF₃, OR₃, NO₂, NO, CHR₄R₅, einer C1- bis C14-Alkylkette, COOR₆, CHO, COR₇, COCF₃, COCH₂CF₃, SR₈, SOR₉, SO₂R₁₀, SONR₁₁R₁₂ und SO₂NR₁₃R₁₄ ausgewählt ist, handelt;
[Hetero-Ar] unabhängig aus substituierten oder unsubstituierten Heterocyclen, die aus Pyridyl, Triazolyl, Triazinyl, Pyrimidinyl, Pyridazinyl, Oxazolyl, Furanyl, Benzothiophenyl, Benzofuranyl, Thiophenyl, Pyrrolyl, Imidazolyl, Thiazolyl, Chinolinyl, Isochinolinyl, Benzooxazolyl, Benzothiazolyl, Indolyl, Benzotriazolyl und Benzoimidazolyl ausgewählt sind und über eine beliebige verfügbare Ringposition gebunden sind, ausgewählt ist, wobei es sich bei der Substitution an einem Heterocyclus, wenn vorhanden, um Monosubstitution durch einen Substituenten, der aus einem von F, Cl, Br, I, CN, NR₄R₅, CF₃, CHCF₂, CH₂F, OCF₃, OCH₂CF₃, OR₆, NO₂, NO, CHR₄R₅, einer C1- bis C14-Alkylkette, COOR₆, CHO, COR₇, COCF₃, COCH₂CF₃, SR₈, SOR₉, SO₂R₁₀, SONR₁₁R₁₂ und SO₂NR₁₃R₁₄ ausgewählt ist, handelt;
R₁, R₂, R₃, R₆, R₇, R₈, R₉, R₁₀, R₁₁, R₁₂, R₁₃ und R₁₄ unabhängig für Monosubstituenten, die aus einem von H, linearem C1-C10-Alkyl, verzweigtem C3-C10-Alkyl und unsubstituiertem Phenyl ausgewählt sind, stehen;
mit einer substituierten Chlorpyrimidin-Zwischenverbindung in Gegenwart eines Katalysators in einem Lösungsmittel mit anschließender Zugabe der Base bei einer Temperatur im Bereich von 80 °C bis 100 °C über einen Zeitraum im Bereich von 24 bis 49 h zum Erhalt einer Verbindung der allgemeinen Formel 1.

7. Verfahren nach Anspruch 6, wobei die substituierte Chlorpyrimidin-Zwischenverbindung aus der Gruppe bestehend aus 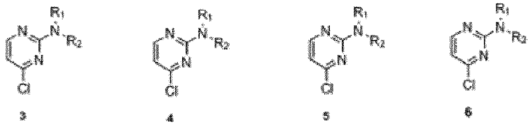 ausgewählt ist, wobei
R1 = H, R2 = Cyclopropyl, Cyclopentyl, Cycloheptyl oder 2-Phenylethyl.

8. Verfahren nach Anspruch 6, wobei es sich bei dem verwendeten Katalysator um Tetrakis(triphenyl-phosphino)palladium(0) handelt.

9. Verfahren nach Anspruch 6, wobei das Lösungsmittel aus der Gruppe bestehend aus trockenem Dioxan und Methanol ausgewählt wird.

10. Verfahren nach Anspruch 6, wobei die Base aus der Gruppe bestehend aus Caesiumcarbonat und Triethylamin ausgewählt wird.

## Revendications

1. Composé de Formule générale 1, 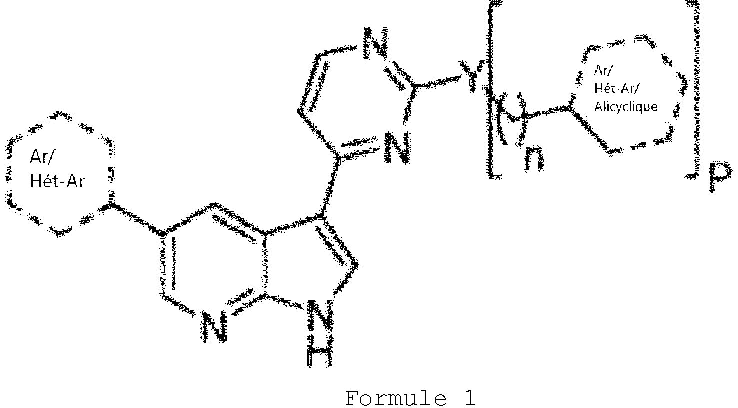
« Y » étant indépendamment choisi parmi l'un quelconque parmi NH, S, O, SO, SO₂ ;
« n » étant indépendamment choisi parmi l'un quelconque parmi 0, 1, 2 ;
P étant 1 ;
[Ar] étant indépendamment choisi parmi l'un quelconque parmi phényle substitué ou non substitué et aryle substitué ou non substitué, fixé par une quelconque position de cycle disponible, une substitution sur le phényle ou l'aryle, lorsqu'elle est présente, étant une monosubstitution par un substituant choisi parmi l'un quelconque parmi F, Cl, Br, I, CN, NR₁R₂, CF₃, CHCF₂, CH₂F, OCF₃, OCH₂CF₃, OR₃, NO₂, NO, CHR₄R₅, une chaîne alkyle de C1 à C14, COOR₆, CHO, COR₇, COCF₃, COCH₂CF₃, SR₈, SOR₉, SO₂R₁₀, SONR₁₁R₁₂, et SO₂NR₁₃R₁₄ ;
[Hétéro-Ar] étant indépendamment choisi parmi des hétérocycles substitués ou non substitués choisis parmi pyridinyle, triazolyle, triazinyle, pyrimidinyle, pyridazinyle, oxazolyle, furannyle, benzothiophényle, benzofurannyle, thiophényle, pyrrolyle, imidazoyle, thiazoyle, quinoléinyle, isoquinoléinyle, benzooxazolyle, benzothiazolyle, indolyle, benzotriazolyle, et benzoimidazolyle, fixé par une quelconque position de cycle disponible, une substitution sur un hétérocycle, lorsqu'elle est présente, étant une monosubstitution par un substituant choisi parmi l'un quelconque parmi F, Cl, Br, I, CN, NR₄R₅, CF₃, CHCF₂, CH₂F, OCF₃, OCH₂CF₃, OR₆, NO₂, NO, CHR₄R₅, une chaîne alkyle de C1 à C14, COOR₆, CHO, COR₇, COCF₃, COCH₂CF₃, SR₈, SOR₉, SO₂R₁₀, SONR₁₁R₁₂, et SO₂NR₁₃R₁₄ ;
R₁, R₂, R₃, R₄, R₅, R₆, R₇, R₈, R₉, R₁₀, R₁₁, R₁₂, R₁₃, et R₁₄, étant indépendamment des monosubstituants choisis parmi l'un quelconque parmi H, C1-C10 alkyle linéaire, C3-C10 alkyle ramifié, et phényle non substitué ; [Alicyclique] étant indépendamment choisi parmi des cycloalcanes non substitués choisis parmi l'un quelconque parmi cyclopropane, cyclobutane, cyclopentane. cyclohexane, cycloheptane et cyclooctane, fixé par une quelconque position de cycle disponible.

2. Composé de Formule générale 1 selon la revendication 1, les composés de Formule 1 étant choisis parmi :
N-cyclopropyl-4-(5-(4-(trifluorométhyl)phényl)-1H-pyrrolo[2,3-b]pyridin-3-yl)pyrimidin-2-amine, (composé 1A1, Tableau 1)
N-cyclopropyl-4-(5-(4-fluorophényl)-1*H*-pyrrolo[2,3-b]pyridin-3-yl)pyrimidin-2-amine (composé 1A2, Tableau 1)
N-cyclopropyl-4-(5-(4-(trifluorométhoxy)phényl)-1H-pyrrolo[2,3-b]pyridin-3-yl)pyrimidin-2-amine (composé 1A3, Tableau 1)
N-cyclopropyl-4-(5-(3-(trifluorométhyl)phényl)-1H-pyrrolo[2,3-b]pyridin-3-yl)pyrimidin-2-amine (composé 1A4, Tableau 1)
N-cyclopropyl-4-(5-(3-fluorophényl)-1H-pyrrolo[2,3-b]pyridin-3-yl)pyrimidin-2-amine (composé 1A5, Tableau 1)
N-cyclopropyl-4-(5-(p-tolyl)-1H-pyrrolo[2,3-b]pyridin-3-yl)pyrimidin-2-amine (composé 1A6, Tableau 1)
N-cyclopropyl-4-(5-(m-tolyl)-1H-pyrrolo[2,3-b]pyridin-3-yl)pyrimidin-2-amine (composé 1A7, Tableau 1)
N-cyclopropyl-4-(5-(4-(méthylthio)phényl)-1H-pyrrolo[2,3-b]pyridin-3-yl)pyrimidin-2-amine (composé 1A8, Tableau 1)
N-(cyclopropyl-4-(5-(2-(méthylthio)phényl)-1H-pyrrolo[2,3-b]pyridin-3-yl)pyrimidin-2-amine (composé 1A9, Tableau 1)
N-cyclopropyl-4-(5-(2-éthylphényl)-1H-pyrrolo[2,3-b]pyridin-3-yl)pyrimidin-2-amine (composé 1A10, Tableau 1)
N-cyclopropyl-4-(5-(4-méthoxyphényl)-1H-pyrrolo[2,3-b]pyridin-3-yl)pyrimidin-2-amine (composé 1A11, Tableau 1)
4-(5-(4-chlorophényl)-1H-pyrrolo[2,3-b]pyridin-3-yl)-N-cyclopropylpyrimidin-2-amine (composé 1A12, Tableau 1)
1-(4-(3-(2-(cyclopropylamino)pyrimidin-4-yl)-1H-pyrrolo[2,3-b]pyridin-5-yl)phényl)éthan-1-one (composé 1A13, Tableau 1)
N-cyclopropyl-4-(5-(naphtalén-1-yl)-1H-pyrrolo[2,3-b]pyridin-3-yl)pyrimidin-2-amine (composé 1A14, Tableau 1)
N-cyclopropyl-4-(5-(pyridin-3-yl)-1H-pyrrolo[2,3-b]pyridin-3-yl)pyrimidin-2-amine (composé 1A15, Tableau 1)
N-cyclopropyI-4-(5-(furan-3-yl)-1H-pyrrolo[2,3-b]pyridin-3-yl)pyrimidin-2-amine (composé 1A16, Tableau 1)
N-cyclopropyl-4-(5-(5-méthoxy-1H-indol-2-yl)-1H-pyrrolo[2,3-b]pyridin-3-yl)pyrimidin-2-amine (composé 1A17, Tableau 1)
4-(5-(benzofuran-2-yl)-1H-pyrrolo[2,3-b]pyridin-3-yl)-N-cyclopropylpyrimidin-2-amine (composé 1A18, Tableau 1)
4-(5-(1H-indol-2-yl)-1H-pyrrolo[2,3-b]pyridin-3-yl)-N-cyclopropylpyrimidin-2-amine (composé 1A19, Tableau 1)
N-cyclopropyl-4-(5-(isoquinoléin-1-yl)1H-pyrrolo[2,3-b]pyridin-3-yl)pyrimidin-2-amine (composé 1A20, Tableau 1)
N-cyclopentyl-4-(5-(4-(trifluorométhyl)phényl)-1H-pyrrolo[2,3-b]pyridin-3-yl)pyrimidin-2-amine (composé 1A21, Tableau 2)
N-cyclopentyl-4-(5-(4-fluorophényl)-1H-pyrrolo[2,3-b]pyridin-3-yl)pyrimidin-2-amine (composé 1A22, Tableau 2)
N-cyclopentyl-4-(5-(4-(trifluorométhoxy)phényl)-1H-pyrrolo[2,3-b]pyridin-3-yl)pyrimidin-2-amine (composé 1A23, Tableau 2)
N-cyclopentyl-4-(5-(3-(trifluorométhyl)phényl)-1H-pyrrolo[2,3-b]pyridin-3-yl)pyrimidin-2-amine (composé 1A24, Tableau 2)
N-cyclopentyl-4-(5-(3-fluorophényl)-1H-pyrrolo[2,3-b]pyridin-3-yl)pyrimidin-2-amine (composé 1A25, Tableau 2)
N-cyclopentyl-4-(5-(p-tolyl)-1H-pyrrolo[2,3-b]pyridin-3-yl)pyrimidin-2-amine (composé 1A26, Tableau 2)
N-cyclopentyl-4-(5-(m-tolyl)-1H-pyrrolo[2,3-b]pyridin-3-yl)pyrimidin-2-amine (composé 1A27, Tableau 2)
N-cyclopentyl-4-(5-(4-(méthylthio)phényl)-1H-pyrrolo[2,3-b]pyridin-3-yl)pyrimidin-2-amine (composé 1A28, Tableau 2)
N-cyclopentyl-4-(5-(2-(méthylthio)phényl)-1H-pyrrolo[2,3-b]pyridin-3-yl)pyrimidin-2-amine (composé 1A29, Tableau 2)
N-cyclopentyl-4-(5-(2-éthylphényl)-1H-pyrrolo[2,3-b]pyridin-3-yl)pyrimidin-2-amine (composé 1A30, Tableau 2)
N-cyclopentyl-4-(5-(4-méthoxyphényl)-1H-pyrrolo[2,3-b]pyridin-3-yl)pyrimidin-2-amine (composé 1A31, Tableau 2)
4-(5-(4-chlorophényl)-1H-pyrrolo[2,3-b]pyridin-3-yl)-N-cyclopentylpyrimidin-2-amine (composé 1A32, Tableau 2)
4-(3-(2-(cyclopentylamino)pyrimidin-4-yl)-1H-pyrrolo[2,3-b]pyridin-5-yl)benzaldéhyde (composé 1A33, Tableau 2)
N-cyclopentyl-4-(5-(naphtalén-1-yl)-1H-pyrrolo[2,3-b]pyridin-3-yl)pyrimidin-2-amine (composé 1A34, Tableau 2)
N-cyclopentyl-4-(5-(pyridin-3-yl)-1H-pyrrolo[2,3-b]pyridin-3-yl)pyrimidin-2-amine (composé 1A35, Tableau 2)
N-cyclopentyl-4-(5-(furan-3-yl)-1H-pyrrolo[2,3-b]pyridin-3-yl)pyrimidin-2-amine (composé 1A36, Tableau 2)
N-cyclopentyl-4-(5-(5-méthoxy-1H-indol-2-yl)-1H-pyrrolo[2,3-b]pyridin-3-yl)pyrimidin-2-amine (composé 1A37, Tableau 2)
4-(5-(benzo[b]thiophén-2-yl)-1H-pyrrolo[2,3-b]pyridin-3-yl)-N-cyclopentylpyrimidin-2-amine (composé 1A38, Tableau 2)
4-(5-(1H-indol-2-yl)-1H-pyrrolo[2,3-b]pyridin-3-yl)-N-cyclopentylpyrimidin-2-amine (composé 1A39, Tableau 2)
N-cyclopentyl-4-(5-(isoquinoléin-1-yl)-1H-pyrrolo[2,3-b]pyridin-3-yl)pyrimidin-2-amine (composé 1A40, Tableau 2)
N-cycloheptyl-4-(5-(4-(trifluorométhyl)phényl)-1H-pyrrolo[2,3-b]pyridin-3-yl)pyrimidin-2-amine (composé 1A41, Tableau 3)
N-cycloheptyl-4-(5-(4-fluorophényl)-1H-pyrrolo[2,3-b]pyridin-3-yl)pyrimidin-2-amine(composé 1A42, Tableau 3)
N-cycloheptyl-4-(5-(4-(trifluorométhoxy)phényl)-1H-pyrrolo[2,3-b]pyridin-3-yl)pyrimidin-2-amine (composé 1A43, Tableau 3)
N-cycloheptyl-4-(5-(3-(trifluorométhyl)phényl)-1H-pyrrolo[2,3-b]pyridin-3-yl)pyrimidin-2-amine (composé 1A44, Tableau 3)
N-cycloheptyl-4-(5-(3-fluorophényl)-1H-pyrrolo[2,3-b]pyridin-3-yl)pyrimidin-2-amine (composé 1A45, Tableau 3)
N-cycloheptyl-4-(5-(p-tolyl)-1H-pyrrolo[2,3-b]pyridin-3-yl)pyrimidin-2-amine (composé 1A46, Tableau 3)
N-cycloheptyl-4-(5-(m-tolyl)-1H-pyrrolo[2,3-b]pyridin-3-yl)pyrimidin-2-amine (composé 1A47, Tableau 3)
N-cycloheptyl-4-(5-(4-(méthylthio)phényl)-1H-pyrrolo[2,3-b]pyridin-3-yl)pyrimidin-2-amine (composé 1A48, Tableau 3)
N-cycloheptyl-4-(5-(2-(méthylthio)phényl)-1H-pyrrolo[2,3-b]pyridin-3-yl)pyrimidin-2-amine (composé 1A49, Tableau 3)
N-cycloheptyl-4-(5-(2-éthylphényl)-1H-pyrrolo[2,3-b]pyridin-3-yl)pyrimidin-2-amine (composé 1A50, Tableau 3)
N-cycloheptyl-4-(5-(4-méthoxyphényl)-1H-pyrrolo[2,3-b]pyridin-3-yl)pyrimidin-2-amine (composé 1A51, Tableau 3)
4-(5-(4-chlorophényl)-1H-pyrrolo[2,3-b]pyridin-3-yl)-N-cycloheptylpyrimidin-2-amine (composé 1A52, Tableau 3)
4-(3-(2-(cycloheptylamino)pyrimidin-4-yl)-1H-pyrrolo[2,3-b]pyridin-5-yl)benzaldéhyde (composé 1A53, Tableau 3)
N-cycloheptyl-4-(5-(naphtalén-1-yl)-1H-pyrrolo[2,3-b]pyridin-3-yl)pyrimidin-2-amine (composé 1A54, Tableau 3)
N-cycloheptyl-4-(5-(pyridin-3-yl)-1H-pyrrolo[2,3-b]pyridin-3-yl)pyrimidin-2-amine (composé 1A55, Tableau 3)
N-cycloheptyl-4-(5-(furan-3-yl)-1H-pyrrolo[2,3-b]pyridin-3-yl)pyrimidin-2-amine (composé 1A56, Tableau 3)
N-cycloheptyl-4-(5-(5-méthoxy-1H-indol-2-yl)-1H-pyrrolo[2,3-b]pyridin-3-yl) pyrimidin-2-amine (composé 1A57, Tableau 3)
4-(5-(benzo[b]thiophén-2-yl)-1H-pyrrolo[2,3-b]pyridin-3-yl)-N-cycloheptylpyrimidin-2-amine (composé 1A58, Tableau 3)
4-(5-(1H-indol-2-yl)-1H-pyrrolo[2,3-b]pyridin-3-yl)-N-cycloheptylpyrimidin-2-amine (composé 1A59, Tableau 3)
N-cycloheptyl-4-(5-(isoquinoléin-1-yl)-1H-pyrrolo[2,3-b]pyridin-3-yl)pyrimidin-2-amine (composé 1A60, Tableau 3)
N-phénéthyl-4-(5-(4-(trifluorométhyl)phényl)-1H-pyrrolo[2,3-b]pyridin-3-yl)pyrimidin-2-amine (composé 1B1, Tableau 4)
4-(5-(4-fluorophényl)-1H-pyrrolo[2,3-b]pyridin-3-yl)-N-phénéthylpyrimidin-2-amine (composé 1B2, Tableau 4)
N-phénéthyl-4-(5-(4-(trifluorométhoxy)phényl)-1H-pyrrolo[2,3-b]pyridin-3-yl)pyrimidin-2-amine (composé 1B3, Tableau 4)
N-phénéthyl-4-(5-(3-(trifluorométhyl)phényl)-1H-pyrrolo[2,3-b]pyridin-3-yl)pyrimidin-2-amine (composé 1B4, Tableau 4)
4-(5-(3-fluorophényl)-1H-pyrrolo[2,3-b]pyridin-3-yl)-N-phénéthylpyrimidin-2-amine (composé 1B5, Tableau 4)
N-phénéthyl-4-(5-(p-tolyl)-1H-pyrrolo[2,3-b]pyridin-3-yl)pyrimidin-2-amine (composé 1B6, Tableau 4)
N-phénéthyl-4-(5-(m-tolyl)-1H-pyrrolo[2,3-b]pyridin-3-yl)pyrimidin-2-amine (composé 1B7, Tableau 4)
4-(5-(4-(méthylthio)phényl)-1H-pyrrolo[2,3-b]pyridin-3-yl)-N-phénéthylpyrimidin-2-amine (composé 1B8, Tableau 4)
4-(5-(2-(méthylthio)phényl)-1H-pyrrolo[2,3-b]pyridin-3-yl)-N-phénéthylpyrimidin-2-amine (composé 1B9, Tableau 4)
4-(5-(2-éthylphényl)-1H-pyrrolo[2,3-b]pyridin-3-yl-N-phénéthylpyrimidin-2-amine (composé 1B10, Tableau 4)
4-(5-(4-méthoxyphényl)-1H-pyrrolo[2,3-b]pyridin-3-yl)-N-phénéthylpyrimidin-2-amine (composé 1B11, Tableau 4)
4-(5-(4-chlrophényl)-1H-pyrrolo[2,3-b]pyridin-3-yl)-N-phénéthylpyrimidin-2-amine (composé 1B12, Tableau 4)
4-(3-(2-(phénéthylamino)pyrimidin-4-yl)-1H-pyrrolo[2,3-b]pyridin-5-yl)benzaldéhyde (composé 1B13, Tableau 4)
4-(5-(naphtalén-1-yl)-1H-pyrrolo[2,3-b]pyridin-3-yl)-N-phénéthylpyrimidin-2-amine (composé 1B14, Tableau 4)
N-phénéthyl-4-(5-(pyridin-3-yl)-1H-pyrrolo[2,3-b]pyridin-3-yl)pyrimidin-2-amine (composé 1B15, Tableau 4)
4-(5-(furan-3-yl)-1H-pyrrolo[2,3-b]pyridin-3-yl)-N-phénéthylpyrimidin-2-amine (composé 1B16, Tableau 4)
4-(5-(5-méthoxy-1H-indol-2-yl)-1H-pyrrolo[2,3-b]pyridin-3-yl)-N-phénéthylpyrimidin-2-amine (composé 1B17, Tableau 4)
4-(5-(benzo[b]thiophén-2-yl)-1H-pyrrolo[2,3-b]pyridin-3-yl)-N-phénéthylpyrimidin-2-amine (composé 1B18, Tableau 4)
4-(5-(1H-indol-2-yl)-1H-pyrrolo[2,3-b]pyridin-3-yl)-N-phénéthylpyrimidin-2-amine (composé 1B19, Tableau 4)
4-(5-(isoquinoléin-1-yl)-1H-pyrrolo[2,3-b]pyridin-3-yl)-N-phénéthylpyrimidin-2-amine (composé 1B20, Tableau 4).

3. Composé de Formule générale 1 selon la revendication 1, pour une utilisation dans le traitement d'un cancer.

4. Composé pour une utilisation selon la revendication 3, ledit composé inhibant la CDK2 et la CDK9.

5. Composé de Formule générale 1 selon la revendication 1, ledit composé inhibant l'activité d'une lignée cellulaire cancéreuse *in vitro.*

6. Procédé pour la préparation du composé de Formule générale 1 selon la revendication 1, le procédé comprenant ;
la mise en réaction d'un composé intermédiaire de formule 13 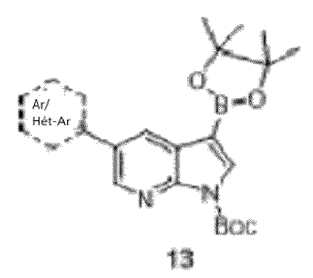
[Ar] étant indépendamment choisi parmi l'un quelconque parmi phényle substitué ou non substitué et aryle substitué ou non substitué, fixé par une quelconque position de cycle disponible, une substitution sur le phényle ou l'aryle, lorsqu'elle est présente, étant une monosubstitution par un substituant choisi parmi l'un quelconque parmi F, Cl, Br, I, CN, NR₁R₂, CF₃, CHCF₂, CH₂F, OCF₃, OCH₂CF₃, OR₃, NO₂, NO, CHR₄R₅, une chaîne alkyle de C1 à C14, COOR₆, CHO, COR₇, COCF₃, COCH₂CF₃, SR₈, SOR₉, SO₂R₁₀, SONR₁₁R₁₂, et SO₂NR₁₃R₁₄ ;
[Hétéro-Ar] étant indépendamment choisi parmi des hétérocycles substitués ou non substitués choisis parmi pyridinyle, triazolyle, triazinyle, pyrimidinyle, pyridazinyle, oxazolyle, furannyle, benzothiophényle, benzofurannyle, thiophényle, pyrrolyle, imidazoyle, thiazoyle, quinoléinyle, isoquinoléinyle, benzooxazolyle, benzothiazolyle, indolyle, benzotriazolyle, et benzoimidazolyle fixé par une quelconque position de cycle disponible, une substitution sur un hétérocycle, lorsqu'elle est présente, étant une monosubstitution par un substituant choisi parmi l'un quelconque parmi F, Cl, Br, I, CN, NR₄R₅, CF₃, CHCF₂, CH₂F, OCF₃, OCH₂CF₃, OR₆, NO₂, NO, CHR₄R₅, une chaîne alkyle de C1 à C14, COOR₆, CHO, COR₇, COCF₃, COCH₂CF₃, SR₈, SOR₉, SO₂R₁₀, SONR₁₁R₁₂, et SO₂NR₁₃R₁₄ ;
R₁, R₂, R₃, R₄, R₅, R₆, R₇, R₈, R₉, R₁₀, R₁₁, R₁₂, R₁₃, et R₁₄, étant indépendamment des monosubstituants choisis parmi l'un quelconque parmi H, C1-C10 alkyle linéaire, C3-C10 alkyle ramifié, et phényle non substitué,
avec un composé intermédiaire de type chloropyrimidine substitué en la présence d'un catalyseur dans un solvant suivie par l'ajout de la base à une température dans la plage de 80 °C à 100 °C pendant une durée dans la plage de 24 à 49 h pour obtenir un composé de Formule générale 1.

7. Procédé selon la revendication 6, le composé intermédiaire de type chloropyrimidine substituée étant choisi dans le groupe constitué par 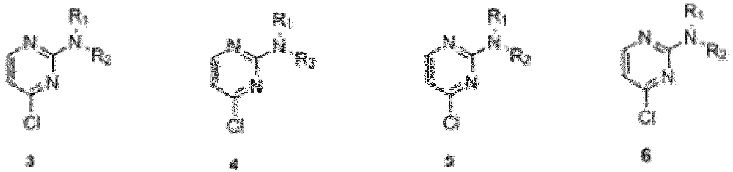 R1 = H ; R2 = cyclopropyle, cyclopentyle, cycloheptyle ou 2-phényléthyle.

8. Procédé selon la revendication 6, le catalyseur utilisé étant le tétrakis(triphénylphosphino)palladium(0) .

9. Procédé selon la revendication 6, le solvant étant choisi dans le groupe constitué par le dioxanne et le méthanol secs.

10. Procédé selon la revendication 6, la base étant choisie dans le groupe constitué par le carbonate de césium et la triéthylamine.
